(19) Europäisches Patentamt / European Patent Office / Office européen des brevets

(11) EP 2 591 673 A1

(12) EUROPEAN PATENT APPLICATION
published in accordance with Art. 153(4) EPC

(43) Date of publication:
**15.05.2013 Bulletin 2013/20**

(21) Application number: **11803550.0**

(22) Date of filing: **04.07.2011**

(51) Int Cl.:
*A01N 43/78* (2006.01) *A01N 47/18* (2006.01)
*A01N 47/36* (2006.01) *A01P 5/00* (2006.01)
*A01P 7/02* (2006.01) *A01P 7/04* (2006.01)
*C07D 277/20* (2006.01) *C07D 277/32* (2006.01)
*C07D 401/04* (2006.01) *C07D 401/14* (2006.01)
*C07D 417/06* (2006.01) *C07D 417/14* (2006.01)

(86) International application number:
**PCT/JP2011/065285**

(87) International publication number:
**WO 2012/005216 (12.01.2012 Gazette 2012/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.03.2011 JP 2011077912**
**07.07.2010 JP 2010154796**

(71) Applicants:
- **Meiji Seika Pharma Co., Ltd.**
  **Tokyo 104-8002 (JP)**
- **Gifu University**
  **Gifu-shi**
  **Gifu 501-1193 (JP)**

(72) Inventors:
- **KAGABU, Shinzo**
  **Gifu-shi**
  **Gifu 501-1193 (JP)**
- **MITOMI, Masaaki**
  **Yokohama-shi**
  **Kanagawa 222-8567 (JP)**
- **KITSUDA, Shigeki**
  **Yokohama-shi**
  **Kanagawa 222-8567 (JP)**
- **NOMURA, Masahiro**
  **Yokohama-shi**
  **Kanagawa 222-8567 (JP)**
- **NAKAMURA, Satoshi**
  **Yokohama-shi**
  **Kanagawa 222-8567 (JP)**
- **YAMAMOTO, Kazumi**
  **Yokohama-shi**
  **Kanagawa 222-8567 (JP)**

(74) Representative: **Cohausz & Florack**
**Bleichstraße 14**
**40211 Düsseldorf (DE)**

(54) **PEST CONTROL AGENT**

(57) Provided is a novel pest control agent using at least one imino derivative represented by the following chemical formula (I):

[Chem. 1]

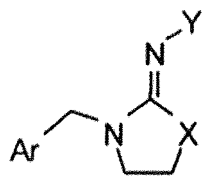

Formula (I)

EP 2 591 673 A1

## Description

[Technical Field]

The present invention relates to a novel pest control agent using an imino derivative.

[Background Art]

Although many pest control agents have been developed so far, novel drugs are still sought because of problems associated with decreased drug susceptibility, persistence of the effects of drugs, safety of drugs during the use, and the like.

As for the use of imino derivatives for controlling pests, the uses of imino derivatives as pesticides in the agricultural field have been reported (Patent Documents 1 to 3 and Non-Patent Document 1).

However, these Documents do not specifically describe the use of imino derivatives for controlling animal-parasitic pests or public health pests.

[Citation List]

[Patent Literature]

[PTL 1] Japanese Unexamined Patent Application Publication No. Sho 63-150275

[PTL 2] International Application Japanese-Phase Publication No. 2007-506674

[PTL 3] International Publication No. W02010/001922 [Non Patent Literature]

[NPL 1] Chemical Research in Toxicology, 2009, 22 (3), 476-482


[Summary of Invention]

[Technical Problem]

An object of the present invention is to provide a novel pest control agent.

[Solution to Problem]

To achieve the above-described object, the present inventors provide a pest control agent using at least one imino derivative represented by the following chemical formula (I):

[Chem. 1]

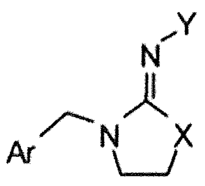

Formula (I)

Specifically, the present invention provides a pest control agent using at least one imino derivative represented by the chemical formula (I) (where Ar represents a heterocyclic group which may have a substituent on a ring thereof, X represents a sulfur atom, $CH_2$, or NR, Y represents $COR_1$, $CONR_3R_4$, $CONHCOR_5$, or $CO_2R_9$, and R represents a hydrogen atom or an alkyl group,
when Y is $COR_1$, $R_1$ represents a hydrogen atom, a C1-C18 alkyl group, a C1-C18 halogenated alkyl group, a C2-C18 alkenyl group, a C2-C18 halogenated alkenyl group, a C2-C18 alkynyl group, a C2-C18 halogenated alkynyl group, a substituted or unsubstituted C6-C10 aryl group, a substituted or unsubstituted (C6-C10)aryl(C1-C8)alkyl group, a substituted or unsubstituted (C6-C10)aryl(C2-C8)alkenyl group, a substituted or unsubstituted (C6-C10)aryl(C2-C8)alkynyl group, a substituted or unsubstituted (C1-C4)alkoxy(C1-C5)alkyl group, a substituted or unsubstituted (C1-C4)alkoxy (C2-C5)alkenyl group, a substituted or unsubstituted (C1-C4)alkoxy(C2-C5)alkynyl group, a C1-C3 alkoxycarbonyl group,

a (C1-C3)alkylsulfonyl(C1-C3)alkyl group, a (C1-C4)alkylthio(C1-C5)alkyl group, a C3-C12 substituted or unsubstituted cycloalkyl group, a 3-membered to 7-membered substituted or unsubstituted heterocycloalkyl group, a 3-membered to 7-membered substituted or unsubstituted heterocycloalkyl(C1-C8)alkyl group, a 3-membered to 7-membered substituted or unsubstituted heterocycloalkyl(C2-C8)alkenyl group, a 3-membered to 7-membered substituted or unsubstituted heterocycloalkyl(C2-C8)alkynyl group, a cyano(C1-C3)alkyl group, a substituted or unsubstituted phenoxy(C1-C8)alkyl group, a substituted or unsubstituted phenoxy(C2-C8)alkenyl group, a substituted or unsubstituted phenoxy(C2-C8) alkynyl group, a substituted or unsubstituted heterocyclyl(C1-C8)alkyl group, a substituted or unsubstituted heterocyclyl (C2-C8)alkenyl group, a substituted or unsubstituted heterocyclyl(C2-C8)alkynyl group, a substituted or unsubstituted heterocyclyloxy(C1-C8)alkyl group, a substituted or unsubstituted heterocyclyloxy(C2-C8)alkenyl group, a substituted or unsubstituted heterocyclyloxy(C2-C8)alkynyl group, a furanyl group, a morpholinyl group, a norbornenyl group, an adamantyl group, an isothiocyanatomethyl group, a rhodanine group, a substituted or unsubstituted heterocyclic or aromatic ring, a (C1-C5)alkylcarbonylamino(C1-C3)alkyl group, a (C1-C5)alkyloxycarbonylaminooxymethyl group, a (C1-C5)alkyloxycarbonylaminomethyl group, or a (C1-C5)alkylcarbonyloxymethyl group,

when Y is $CONR_3R_4$, $R_3$ and $R_4$ each represent a hydrogen atom, a C1-C5 alkyl group, a C1-C5 halogenated alkyl group, a C2-C5 alkenyl group, a C2-C5 halogenated alkenyl group, a C2-C5 alkynyl group, a C2-C5 halogenated alkynyl group, a C1-C3 alkoxy group, an alkenyloxy group, a substituted or unsubstituted C6-C10 aryl group, a substituted or unsubstituted (C6-C10)aryl(C1-C8)alkyl group, a substituted or unsubstituted (C6-C10)aryl(C2-C8)alkenyl group, a substituted or unsubstituted (C6-C10)aryl(C2-C8)alkynyl group, a (C1-C4)alkoxy(C1-C5)alkylgroup, a (C1-C4)alkoxy(C2-C5)alkenyl group, a (C1-C4)alkoxy(C2-C5)alkynyl group, a C1-C3 alkoxycarbonylmethyl group, a (C1-C4)alkylthio(C1-C5)alkyl group, a C3-C12 substituted or unsubstituted cycloalkyl group, a 3-membered to 7-membered substituted or unsubstituted heterocycloalkyl group, a substituted or unsubstituted pyridylmethyl group, a substituted or unsubstituted benzenesulfonyl group, a (C1-C5)alkylamino group, a substituted or unsubstituted phenylamino group, a (C1-C5)alkylcarbonylamino group, or a substituted or unsubstituted benzoylamino group, and $NR_3R_4$ may form a ring,

when Y is $CONHCOR_5$, $R_5$ represents a hydrogen atom, a C1-C5 alkyl group, a C1-C5 halogenated alkyl group, a C2-C5 alkenyl group, a C2-C5 halogenated alkenyl group, a substituted or unsubstituted C6-C10 aryl group, or a substituted or unsubstituted (C6-C10)aryl(C1-C5)alkyl group, and

when Y is $CO_2R_9$, $R_9$ represents a hydrogen atom, a C1-C18 alkyl group, a C1-C18 halogenated alkyl group, a C2-C18 alkenyl group, a C2-C18 halogenated alkenyl group, a C2-C18 alkynyl group, a C2-C18 halogenated alkynyl group, a substituted or unsubstituted C6-C10 aryl group, a substituted or unsubstituted (C6-C10)aryl(C1-C8)alkyl group, a substituted or unsubstituted (C6-C10)aryl(C2-C8)alkenyl group, a substituted or unsubstituted (C6-C10)aryl(C2-C8)alkynyl group, a substituted or unsubstituted (C1-C4)alkoxy(C1-C5)alkyl group, a substituted or unsubstituted (C1-C4)alkoxy (C2-C5)alkenyl group, a substituted or unsubstituted (C1-C4)alkoxy(C2-C5)alkynyl group, a (C1-C4)alkylthio(C1-C5) alkyl group, a tri(C1-C3 alkyl)silyl(C1-C3)alkyl group, a C3-C12 substituted or unsubstituted cycloalkyl group, a 3-membered to 7-membered substituted or unsubstituted heterocycloalkyl group, a 3-membered to 7-membered substituted or unsubstituted heterocycloalkyl(C1-C8)alkyl group, a 3-membered to 7-membered substituted or unsubstituted heterocycloalkyl(C2-C8)alkenyl group, a 3-membered to 7-membered substituted or unsubstituted heterocycloalkyl(C2-C8) alkynyl group, a (C1-C4)alkylthio(C1-C5)alkyl group, a (C1-C3)alkylsulfonyl(C1-C3)alkyl group, a substituted or unsubstituted phenoxy(C1-C8)alkylgroup, a substituted or unsubstituted phenoxy(C2-C8)alkenyl group, a substituted or unsubstituted phenoxy(C2-C8)alkynyl group, a substituted or unsubstituted heterocyclyl(C1-C8)alkyl group, a substituted or unsubstituted heterocyclyl(C2-C8)alkenyl group, a substituted or unsubstituted heterocyclyl(C2-C8)alkynyl group, a substituted or unsubstituted heterocyclyloxy(C1-C8)alkyl group, a substituted or unsubstituted heterocyclyloxy(C2-C8) alkenyl group, a substituted or unsubstituted heterocyclyloxy(C2-C8)alkynyl group, a succinimide group, or a 18-crown-6-methyl group, and

carbon chains thereof may be substituted with halogens).

In a preferred mode, provided is the pest control agent comprising at least one compound represented by the chemical formula (I) (where Ar is represented by the following chemical formula (II) or (III), and X and Y have the same meanings as described above:

[Chem. 2]

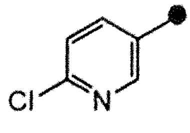

Formula (II)

,

or

[Chem. 3]

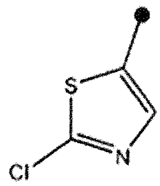
Formula (III)

In another preferred mode, provided is the pest control agent, wherein the imino derivative represented by the chemical formula (I) is any one of the compounds shown in the following Table 1.

[Table 1]

| Compound No. | Y | X | Ar |
|---|---|---|---|
| 6 | CO-phenyl | S | (II) |
| 42 | COOMe | S | (II) |
| 43 | COOC3H7-n | S | (II) |
| 44 | COOC3H7-i | S | (II) |
| 45 | COOCH2CH2Cl | S | (II) |
| 57 | COP h | S | (II) |
| 61 | CHO | S | (III) |
| 72 | COMe | S | (II) |
| 76 | COCHF2 | S | (II) |
| 81 | COCH2CH2OMe | S | (II) |
| 83 | COCH=CH2 | S | (II) |
| 06 | CONHOMe | S | (II) |
| 107 | CONHOEt | S | (II) |
| 113 | COOEt | S | (II) |
| 114 | COOCH2CH2F | S | (II) |
| 115 | COOCH2CHF2 | S | (II) |
| 122 | COOCH2CH2CH2F | S | (II) |
| 123 | COOCH(CH2F)2 | S | (II) |
| 124 | COOCH(Me)CF3 | S | (II) |
| 125 | COOCH(CF3)2 | S | (II) |
| 128 | COO-n-butyl | S | (II) |
| 132 | COOCH2CH2CH2CH2F | S | (II) |
| 134 | COOCH2CF2CF2CF3 | S | (II) |
| 135 | COOCH2CH=CMe2 | S | (II) |
| 139 | COO-CH2-cyclopropyl | S | (II) |
| 140 | COOCH2-2-oxiranyl | S | (II) |
| 141 | COO-cyclobutyl | S | (II) |
| 44 | COOCH2-3-tetrahydrofuranyl | S | (II) |
| 145 | COOCH2-(2,2-dimethyl-1,3-dioxolan-4-yl) | S | (II) |

(continued)

| Compound No. | Y | X | Ar |
|---|---|---|---|
| 155 | COCH2-3-thienyl | S | ( I I ) |
| 167 | COO-iso-propyl | S | ( I I I ) |
| 171 | COOCH2C≡CH | S | ( I I ) |
| 198 | CO-4-t-butylphenyl | S | ( I I I ) |
| 211 | COO-n-pentyl | S | ( I I ) |
| 213 | CO-2,2-difluorocyclopropyl | S | ( I I ) |
| 216 | COCH2CH2CF3 | S | ( I I ) |
| 226 | COO-n-hexyl | S | ( I I ) |
| 227 | COOCH2t-Bu | S | ( I I ) |
| 228 | COO-CH2-Crownether (18-C-6) | S | ( I I ) |
| 230 | COCH2OCOMe | S | ( I I ) |
| 233 | COCH2ONHCOOEt | S | ( I I ) |
| 240 | COCF3 | S | ( I I ) |
| 241 | COCF3 | S | ( I I I ) |
| 244 | COO-3-methoxyphenyl | S | ( I I ) |
| 251 | COOCH2CH2CH2NO2 | S | ( I I ) |
| 255 | COOCH2CH2morpholinyl | S | ( I I ) |
| 267 | CO-3-methylphenyl | S | ( I I ) |
| 268 | CO-2-fluorophenyl | S | ( I I ) |
| 292 | CO-2-fluorophenyl | S | ( I I I ) |
| 299 | CO-phenyl | S | 4,5-dichloro-3-pyridyl |
| 302 | CO-phenyl | S | 4-bromo-3-pyridyl |
| 303 | COOCH2CH2C≡CH | S | ( I I ) |
| 308 | COCH2CH2C≡CH | S | ( I I ) |
| 309 | COCH2CH2CH2C≡CH | S | ( I I ) |
| 313 | COCH2CH2CH2CH2C≡CH | S | ( I I I ) |
| 326 | COCH2-(2-tetrahydrofuranyl) | S | ( I I ) |
| 328 | COCH2CH2-(2-thienyl) | S | ( I I ) |
| 329 | COOCH2CH2-(2-tetrahydrofuranyl) | S | ( I I ) |
| 336 | COCH2CH2-(2-tetrahydrofuranyl) | S | ( I I ) |
| 337 | COOCH2CH2CH2-(2-furanyl) | S | ( I I ) |
| 338 | COOCH2CH2-(3-tetrahydrofuranyl) | S | ( I I ) |
| 341 | CO-CH2CH2-(3-furanyl) | S | ( I I ) |
| 343 | COCH2CH2-(4-methoxyphenyl) | S | ( I I ) |
| 345 | COCH2CH2-(3,5-dimethoxyphenyl) | S | ( I I ) |
| 349 | COOCH2CH2CH2-(3-furanyl) | S | ( I I ) |
| 351 | COO-CH2CH2CH2-(3-tetrahydrofuranyl) | S | ( I I ) |
| 352 | COOCH2CH2-(2-thienyl) | S | ( I I ) |

(continued)

| Compound No. | Y | X | Ar |
|---|---|---|---|
| 354 | COOCH2CH2-(3-thienyl) | S | ( I I ) |
| 355 | COCH2CH2-(benzo[d][1,3]dioxol-5-yl) | S | ( I I ) |
| 358 | COCH2CH2-(3-methoxyphenyl) | S | ( I I ) |
| 359 | COOCH2CH2-(2-furanyl) | S | ( I I ) |
| 366 | COOCH2-(2-thienyl) | S | ( I I ) |
| 374 | COCH2OCH2C≡CH | S | ( I I ) |
| 430 | COCH2OCH2-(2-furanyl) | S | ( I I ) |
| 431 | COCH2OCH2-(3-furanyl) | S | ( I I ) |

In another preferred mode, provided is the pest control agent, wherein the pest is an animal-parasitic pest.

In a particularly preferable mode, provided is the pest control agent, wherein the pest is an animal-parasitic tick or mite.

As a second aspect of the present invention, provided is a compound represented by the chemical formula (Ia):

[Chem. 4]

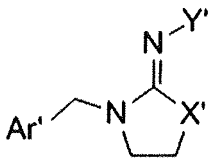

Formula (Ia)

(where Ar' represents a heterocyclic group which may have a substituent on a ring thereof, X' represents a sulfur atom, and Y' represents $COR_1$' or $CO_2R_9$' ,
when Y is $COR_1$', $R_1$' represents a substituted phenyl(C2-C4)alkyl group, a 3-membered to 7-membered substituted or unsubstituted heterocycloalkyl(C2-C3)alkyl group, a substituted or unsubstituted heterocyclyl(C2-C3)alkyl group, a substituted or unsubstituted (C1-C4)alkoxymethyl group, a 3-membered to 7-membered heterocycloalkyl group or a substituted or unsubstituted heterocycle, and
when Y' is $CO_2R_9$', $R_9$' represents a substituted or unsubstituted phenyl(C2-C4)alkyl group, a 3-membered to 7-membered substituted or unsubstituted heterocycloalkyl(C2-C3)alkyl group, or a substituted or unsubstituted heterocyclyl (C2-C3)alkyl group).

In a preferred mode, provided is the compound represented by the chemical formula (Ia) (where Ar' is represented by the following chemical formula (II) or (III), and X' and Y' have the same meaning as described above:

[Chem. 5]

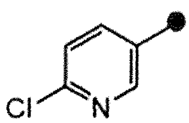

Formula (II)

,

or

[Chem. 6]

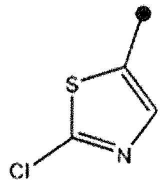

Formula (III)

In a third aspect of the present invention, provided is a method for controlling a pest by use of the above-described pest control agent.

[Advantageous Effects of Invention]

The present invention makes it possible to effectively control pests such as animal-parasitic pests.

[Description of Embodiments]

A pest control agent provided by the present invention means a pest control agent used in fields other than the agricultural field. For example, the pest control agent provided by the present invention is a control agent against an animal-parasitic pest, a public health pest, a nuisance pest, a stored-grain pest, a stored-product pest, a house and household pest, or the like, preferably a control agent against an animal-parasitic pest, and more preferably an acaricide against an animal-parasitic tick or mite.

Ar in the chemical formula (I) represents a heterocycle which may have a substituent on a ring thereof. Specific examples of 5-membered or 6-membered heterocycles include pyridine, thiazole, tetrahydrofuran, furan, and the like. A 3-pyridyl group, a 5-thiazolyl group, and a 3-tetrahydrofuranyl group are particularly desirable.

Moreover, the substituent on the heterocycle is not particularly limited, and examples thereof include halogen atoms (any of fluorine, chlorine, bromine, and iodine), C1-C4 alkyl groups, C1-C4 halogenated alkyl groups, C1-C4 alkoxy groups, di(C1-C4 alkyl) amino groups, a nitro group, and the like.

X in the chemical formula (I) represents a sulfur atom, $CH_2$, or NR, where R represents a hydrogen atom or an alkyl group. The alkyl group is a C1-C4 alkyl group, and may be any one of primary, secondary, and tertiary groups. Examples thereof include a methyl group, an ethyl group, a n-propyl group, an isopropyl group, a n-butyl group, an isobutyl group, a sec-butyl group, a tert-butyl group, and the like. X is preferably a sulfur atom.

The alkyl group of each of the C1-C18 alkyl group represented by $R_1$ or $R_9$ and the C1-C18 halogenated alkyl group represented by $R_1$ or $R_9$ may be any one of primary, secondary and tertiary groups, and examples thereof include a methyl group, an ethyl group, a n-propyl group, an isopropyl group, a n-butyl group, an isobutyl group, a sec-butyl group, a tert-butyl group, a n-pentyl group, a n-hexyl group, a n-decanyl group, a n-heptadecanyl group, and the like. $R_1$ is a preferably C1-C4, more preferably C1-C3 halogenated alkyl group. $R_9$ is a preferably C1-C6, more preferably C3-C4 halogenated alkyl group.

The alkyl group of each of the C1-C5 alkyl group represented by $R_3$, $R_4$, or $R_5$ and the C1-C5 halogenated alkyl group represented by $R_3$, $R_4$, or $R_5$ may be any one of primary, secondary and tertiary groups, and examples thereof include a methyl group, an ethyl group, a n-propyl group, an isopropyl group, a n-butyl group, an isobutyl group, a sec-butyl group, a tert-butyl group, and the like. C1-C3 alkyl groups are preferable.

The alkenyl group of each of the C2-C18 alkenyl group represented by the $R_1$ or $R_9$ and the C2-C18 halogenated alkenyl group represented by the $R_1$ or $R_9$ may be any one of primary, secondary and tertiary groups, and examples thereof include a vinyl group, a 2-propenyl group, a 3-butenyl group, a 4-pentenyl group, a 5-hexenyl group, 9-decenyl group, 16-heptadecenyl group, and the like. $R_1$ is preferably a C2-C6 alkenyl group, and more preferably a C4-C6 alkenyl group. $R_9$ is preferably a C3-C6 alkenyl group, and more preferably a C5-C6 alkenyl group.

The alkenyl group of each of the C2-C5 alkenyl group represented by $R_3$, $R_4$, or $R_5$ and the C2-C5 halogenated alkenyl group represented by $R_3$, $R_4$, or $R_5$ may be any one of primary, secondary and tertiary groups, and examples thereof include a vinyl group, a 2-propenyl group, and a 3-butenyl group.

The alkynyl group of each of the C2-C18 alkynyl group represented by the $R_1$ or $R_9$ and the C2-C18 halogenated alkynyl group represented by the $R_1$ or $R_9$ may be any one of primary, secondary and tertiary groups, and examples thereof include a 1-propynyl group, a 2-propynyl group, a 3-butynyl group, a 4-pentynyl group, a 5-hexynyl group, a 9-decynyl group, a 16-hepta decynyl group, and the like. $R_1$ is preferably a C3-C6 alkynyl group, and more preferably a C4-C6 alkynyl group. $R_9$ is preferably a C3-C6 alkynyl group, and more preferably a C3 or C4 alkynyl group.

The alkynyl group of each of the C2-C5 alkynyl group represented by $R_3$ or $R_4$ and the C2-C5 halogenated

alkynyl group represented by $R_3$ or $R_4$ may be any one of primary, secondary and tertiary groups, and examples thereof include a 1- propynyl group, a 2-propynyl group, a 3-butynyl group, a 4-pentynyl group, and the like.

The C1-C3 alkoxy group represented by $R_3$ or $R_4$ may be any one of primary and secondary groups, and examples thereof include a methoxy group, an ethoxy group, and the like. C1-C2 alkoxy groups are preferable.

The substituted or unsubstituted C6-C10 aryl group represented by $R_1$, $R_3$, $R_4$, $R_5$, or $R_9$ specifically represents a phenyl group, or a naphthyl group. Examples of substituents which may be introduced include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like. The substituents which are introduced in adjacent carbons together may form -O-(CH2)n-O-, wherein n is 1 or 2.

The (C6-C10)aryl of the substituted or unsubstituted (C6-C10) aryl (C1-C8) alkyl group represented by $R_1$, $R_3$, $R_4$, $R_5$, or $R_9$ specifically represents a phenyl group or a naphthyl group, and the (C1-C8)alkyl group of the substituted or unsubstituted (C6-C10)aryl(C1-C8)alkyl group may be linear, branched, or cyclic. Examples of substituents which may be introduced include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like. The substituents which are introduced in adjacent carbons together may form -O-(CH2)n-O-, wherein n is 1 or 2. Examples of the substituted or unsubstituted (C6-C10)aryl(C1-C8)alkyl group include a benzyl group, a 1-phenylethyl group, a 2-phenylethyl group, a 1-phenyl-2-methylpropyl group, a phenylcyclopropyl group, a 3-phenylcyclobutyl group, a 1-phenyl-1-methylethyl group, a 2-(3-methoxyphenyl)ethyl group, and the like. $R_1$ is preferably a substituted or unsubstituted phenyl(C1-C4)alkyl group, and more preferably a substituted phenyl(C2-C4) alkyl group. Specific examples of $R_1$ include a benzyl group, a 1-phenylethyl group, a 2-phenylethyl group, a 1-phenyl-1-methylethyl group, a 1-phenylcyclopropyl group, a 2-(2-methoxyphenyl)ethyl group, a 2-(3-methoxyphenyl)ethyl group, a 2-(4-methoxyphenyl)ethyl group, a 2-(3,5-dimethoxyphenyl)ethyl group, a 2-(2,3-dihydrobenzo[b][1,4]dioxin-6-yl)ethyl group, a 2-(benzo[d][1,3]dioxole-5-yl)ethyl group, a 2-(2-methoxyphenyl)propyl group, and the like. $R_1$ is more preferably a 2-(2-methoxyphenyl)ethyl group, a 2-(3-methoxyphenyl)ethyl group, a 2-(4-methoxyphenyl)ethyl group, a 2-(3,5-dimethoxyphenyl)ethyl group, a 2-(2,3-dihydrobenzo[b][1,4]dioxin-6-yl)ethyl group, a 2-(benzo[d] [1,3]dioxole-5-yl)ethyl group or the like. $R_9$ is preferably a substituted or unsubstituted phenyl(C1-C4)alkyl group, and examples thereof include a 3-methoxybenzyl group and a 2-(3-methoxyphenyl)ethyl group or the like.

The (C6-C10)aryl of the substituted or unsubstituted (C6-C10)aryl(C2-C8)alkenyl group represented by $R_1$, $R_3$, $R_4$, or $R_9$ specifically represents a phenyl group or a naphthyl group, and the (C2-C8)alkenyl group of the substituted or unsubstituted (C6-C10)aryl(C2-C8)alkenyl group may be linear, branched, or cyclic. Examples of substituents which may be introduced include halogen atoms, C1-C4 alkyl groups which may be substituted with halogens, C1-C4 alkyloxy groups which may be substituted with halogens, C3-C6 cycloalkyl groups, a methyl sulfonyl group, a methoxy group, a nitro group, a cyano group, and the like. The substituents which are introduced in adjacent carbons together may form -O-(CH2)n-O-, wherein n is 1 or 2. Examples of the substituted or unsubstituted (C6-C10)aryl(C1-C5)alkenyl group include a 2-phenylethenyl group, a 3-phenyl-2-propenyl group, a phenyl cyclopropenyl group, a 4-phenyl-3-butenyl group, and the like. $R_1$ is preferably a 2-phenylethenyl group. $R_9$ is preferably a 3-phenyl-2-propenyl group.

The (C6-C10) aryl of the substituted or unsubstituted (C6-C10)aryl(C2-C8)alkynyl group represented by $R_1$, $R_3$, $R_4$, or $R_9$specifically represents a phenyl group or a naphthyl group, and the (C2-C8)alkynyl group of the substituted or unsubstituted (C6-C10)aryl(C2-C8)alkynyl group may be linear, branched, or cyclic. Examples of substituents which may be introduced include halogen atoms, C1-C4 alkyl groups which may be substituted with halogens, C1-C4 alkyloxy groups which may be substituted with halogens, C3-C6 cycloalkyl groups, a methyl sulfonyl group, a methoxy group, a nitro group, a cyano group, and the like. The substituents which are introduced in adjacent carbons together may form -O-(CH2)n-O-, wherein n is 1 or 2. Examples of the substituted or unsubstituted (C6-C10)aryl(C1-C5)alkynyl group include a 2-phenylethynyl group and the like.

The (C1-C4)alkoxy of the substituted or unsubstituted (C1-C4)alkoxy(C1-C5)alkyl group represented by $R_1$, $R_3$, $R_4$, or $R_9$ represents a (C1-C4)alkyloxy, alkenyloxy, or alkynyloxy which may be linear, branched, or cyclic. Examples of substituents which may be introduced include halogen atoms, C3-C6 cycloalkyl groups, a phenyl group, a 3-membered to 7-membered substituted or unsubstituted heterocycloalkyl group, a substituted or unsubstitutedheterocycle, and the like. The phenyl group may be substituted with halogens, C1-C4 alkyl groups which may be substituted with halogens, C1-C4 alkyloxy groups which may be substituted with halogens, C3-C6 cycloalkyl groups, a methylsulfonyl group, a methoxy group, a nitro group, or a cyano group. The substituents which are introduced in adjacent carbons together may form -O-(CH2)n-O-, wherein n is 1 or 2. The heterocycloalkyl group may be substituted with halogens. The heterocycle may be substituted with halogens. The (C1-C5)alkyl group of the substituted or unsubstituted (C1-C4)alkoxy(C1-C5) alkyl group may be linear, branched, or cyclic. Examples of the (C1-C4)alkoxy(C1-C5)alkyl group include a methyloxymethyl group, an ethyloxymethyl group, a methyloxyethyl group, an ethyloxyethyl group, an isopropyloxymethyl group, a t-butyloxymethyl group, and the like. The substituted or unsubstituted (C1-C4)alkoxy(C1-C5)alkylgroup represented by $R_1$ is preferably a substituted or unsubstituted (C1-C4)alkoxy(C1-C3)alkyl group, more preferably a substituted or unsubstituted (C1-C4)alkoxymethyl. Examples of the substituted or unsubstituted (C1-C4)alkoxy(C1-C5)alkylgroup

include a methoxymethyl group, a ethoxymethyl group, a ethynyloxymethyl group, a 2-propynyloxymethyl group, a (3,3,3,-trifluoropropyloxy)methyl group, a (4,4,4-trifluorobutyloxy)methyl group, a (2-furanylmethyloxy)methyl group, a (3-furanylmethyloxy)methyl group, a (2-tetrahydrofuranylmethyloxy)methyl group, a (3-tetrahydrofuranylmethyloxy)methyl group, a 2-(2-methoxyphenyl)ethyloxymethyl group, and a 2-(2-fluorophenyl)ethyloxymethyl group, and more preferably include a methoxymethyl group, a ethoxymethyl group, a propynyloxymethyl group, a (3,3,3,-trifluoropropyloxy) methyl group, a (4,4,4-trifluorobutyloxy)methyl group, a (2-furanylmethyloxy)methyl group, and a (3-furanylmethyloxy) methyl group. $R_9$ is preferably a methoxy(C3-C4)alkyl group.

The (C1-C4)alkoxy of the substituted or unsubstituted (C1-C4)alkoxy(C2-C5)alkenyl group represented by $R_1$, $R_3$, $R_4$, or $R_9$ represents a (C1-C4)alkyloxy, alkenyloxy, or alkynyloxy group which may be linear, branched, or cyclic. Examples of substituents which may be introduced include halogen atoms, C3-C6 cycloalkyl groups, a phenyl group, a 3-membered to 7-membered substituted or unsubstituted heterocycloalkyl group, a substituted or unsubstituted heterocycle, and the like. The phenyl group may be substituted with halogens, C1-C4 alkyl groups which may be substituted with halogens, C1-C4 alkyloxy groups which may be substituted with halogens, C3-C6 cycloalkyl groups, a methylsulfonyl group, a methoxy group, a nitro group, or a cyano group. The substituents which are introduced in adjacent carbons together may form -O-(CH2)n-O-, wherein n is 1 or 2. The heterocycloalkyl group may be substituted with halogens. The heterocycle may be substituted with halogens. The (C2-C5) alkenyl group of the substituted or unsubstituted (C1-C4)alkoxy(C2-C5)alkenyl group may be linear, branched, or cyclic. Examples of the substituted or unsubstituted (C1-C4)alkoxy(C2-C5)alkenyl group include a 2-methoxyethenyl group, a 2-ethoxyethenyl group, a 3-methoxy-2-propenylgroup, a 3-ethoxy-2-propenylgroup, a (2-furanylmethyloxy) ethenyl group, a (3-furanylmethyloxy) ethenyl group, a (2-tetrahydrofuranylmethyloxy) ethenyl group, a (3-tetrahydrofuranylmethyloxy) ethenyl group and the like.

The (C1-C4)alkoxy of the substituted or unsubstituted (C1-C4)alkoxy(C2-C5)alkynyl group represented by $R_1$, $R_3$, $R_4$, or $R_9$ represents a (C1-C4)alkyloxy, alkenyloxy, or alkynyloxy group which may be linear, branched, or cyclic. Examples of substituents which may be introduced include halogen atoms, C3-C6 cycloalkyl groups, a phenyl group, a 3-membered to 7-membered substituted or unsubstituted heterocycloalkyl group, a substituted or unsubstituted heterocycle, and the like. The phenyl group may be substituted with halogens, C1-C4 alkyl groups which may be substituted with halogens, C1-C4 alkyloxy groups which may be substituted with halogens, C3-C6cycloalkyl groups, a methylsulfonyl group, a methoxy group, a nitro group, or a cyano group. The substituents which are introduced in adjacent carbons together may form -O-(CH2)n-O-, wherein n is 1 or 2. The heterocycloalkyl group may be substituted with halogens. The heterocycle may be substituted with halogens. The (C2-C5) alkynyl group of the substituted or unsubstituted (C1-C4)alkoxy(C2-C5)alkynyl group may be linear, branched, or cyclic. Examples of the substituted or unsubstituted (C1-C4)alkoxy(C2-C5)alkynyl group include a 2-methoxyethynyl group, a 2-ethoxyethynyl group, a 3-methoxy-2-propynyl group, a 3-ethoxy-2-propynyl group, a (2-furanylmethyloxy) ethynyl group, a (3-furanylmethyloxy) ethynyl group, a (2-tetrahydrofuranylmethyloxy) ethynyl group, a (3-tetrahydrofuranylmethyloxy) ethynyl group and the like.

In the C1-C3 alkoxycarbonyl group represented by $R_1$, the alkyl group of the alkoxy represents a C1-C3 alkyl group which may be linear, branched, or cyclic. Examples thereof include an ethyloxycarbonyl group and the like.

In the C1-C3 alkoxycarbonylmethyl group represented by $R_3$ or $R_4$, the alkyl group of the alkoxy represents a C1-C3 alkyl group which may be linear, branched, or cyclic. Examples thereof include a methoxycarbonylmethyl group, an ethoxycarbonylmethyl group, a n-propyloxycarbonylmethyl group, an isopropyloxycarbonylmethyl group, a cyclopropyloxycarbonylmethyl group, and the like.

The (C1-C3)alkylsulfonyl of the (C1-C3)alkylsulfonyl(C1-C3)alkyl group represented by $R_1$ represents a (C1-C3) alkylsulfonyl, alkenylsulfonyl or alkynylsulfonyl which may be linear, branched, or cyclic, and the (C1-C3)alkyl group of the (C1-C3)alkylsulfonyl(C1-C3)alkyl group represents a (C1-C3)alkyl group which may be linear, branched, or cyclic. Examples thereof include a methylsulfonylethyl group, and the like.

The (C1-C4)alkylthio of the (C1-C4) alkylthio (C1-C5)alkyl group represented by $R_1$, $R_3$, $R_4$, or $R_9$ represents (C1-C4)alkylthio, alkenylthio, or alkynylthio which may be linear, branched, or cyclic, and the (C1-C5)alkyl group of the (C1-C4)alkylthio(C1-C5)alkyl group represents a (C1-C5)alkyl group which may be linear, branched, or cyclic. Examples thereof include a methylthiomethyl group, a methylthioethyl group, an ethylthiomethyl group, an isopropylthiomethyl group, a trifluoromethylthiomethyl group, and the like. A methylthiomethyl group and a methylthioethyl group are preferable.

The C3-C12 substituted or unsubstituted cycloalkyl group represented by $R_1$, $R_3$, $R_4$, or $R_9$ represents a C3-C12 alkyl group having one or more cycloalkyl groups, which may have alkenyl or alkynyl on the ring or in an alkyl moiety on the chain. Examples thereof include a cyclopropyl group, a cyclobutyl group, a cyclopentyl group, a cyclohexyl group, a cyclopropylmethyl group, a cyclohexylmethyl group, a cyclopentenyl group, and the like. The substituent is not particularly limited, and examples thereof include halogen atoms (any of chlorine, bromine, fluorine, and iodine) and C1-C3 alkyl groups. $R_1$ is preferably a cyclopropyl group, a cyclopentyl group, or a cyclohexyl group, and more preferably a cyclohexyl group. $R_9$ is preferably a cyclobutyl group, a cyclopentyl group, a cyclohexyl group, or the like, and more preferably a cyclo butyl group.

The 3-membered to 7-membered substituted or unsubstituted heterocycloalkyl group represented by $R_1$, $R_3$,

$R_4$, or $R_9$ represents a heterocycloalkyl group containing one or two hetero atoms such as oxygen atoms, sulfur atoms, and nitrogen atoms. Examples thereof include an oxiranyl group, an azetidinyl group, a pyrrolidinyl group, a piperidinyl group, a piperazinyl group, a morpholinyl group, an oxetanyl group, a thietanyl group, a tetrahydrofuranyl group, a tetrahydropyranyl group, and the like. $R_1$ is preferably a 2-oxiranyl group, a 3-azetidinyl group, a 1-morpholinyl group, or a tetrahydrofuranyl group, and more preferably a tetrahydrofuranyl group. $R_9$ is preferably a 3-oxetanyl group, a 3-thietanyl group, or the like, and more preferably a 3-oxetanyl group or the like. Examples of substituents which may be introduced include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like.

The 3-membered to 7-membered substituted or unsubstituted heterocycloalkyl of each of the 3-membered to 7-membered substituted or unsubstituted heterocycloalkyl(C1-C8)alkyl group, the 3-membered to 7-membered substituted or unsubstituted heterocycloalkyl(C2-C8)alkenyl group, and the 3-membered to 7-membered substituted and unsubstituted heterocycloalkyl(C2-C8)alkynyl group represented by the $R_1$ or $R_9$ represents a heterocycloalkyl group containing one or two hetero atoms such as oxygen atoms, sulfur atoms, and nitrogen atoms. Examples thereof include an oxiranyl group, an azetidinyl group, a pyrrolidinyl group, a piperidinyl group, a piperazinyl group, a morpholinyl group, an oxetanyl group, a thietanyl group, a tetrahydrofuranyl group, a tetrahydropyranyl group, and the like. $R_1$ is preferably a 3-membered to 7-membered substituted or unsubstituted heterocycloalkyl(C1-C3)alkyl group, or a 3-membered to 7-membered substituted or unsubstituted heterocycloalkyl(C2-C3)alkenyl group, and more preferably a 3-membered to 7-membered substituted or unsubstituted heterocycloalkyl(C2-C3)alkyl group or a 3-membered to 7-membered substituted or unsubstituted heterocycloalkyl(C2-C3)alkenyl group. Specific examples thereof include a morpholinylmethyl group, a 2-(2-tetrahydrofuranyl)ethyl group, and a 3-tetrahydrofuranylmethyl group. $R_9$is preferably a 3-membered to 7-membered substituted or unsubstituted heterocycloalkyl(C1-C3)alkyl group, and more preferably a 3-membered to 7-membered substituted or unsubstituted heterocycloalkyl(C2-C3)alkyl group. Specific examples thereof include a 2-oxiranylmethyl group, a 2-tetrahydrofuranylmethyl group, a 2-tetrahydropyranylmethyl group, a 3-(2-tetrahydrofuranyl)propyl group, a 2-(3-tetrahydrofuranyl)ethyl group, and the like. Examples of substituents which may be introduced include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like.

The (C1-C3)alkyl group of the cyano(C1-C3)alkyl group represented by $R_1$ represents a (C1-C3)alkyl group which may be linear, branched, or cyclic. Examples of the cyano(C1-C3)alkyl group include a cyanomethyl group, and the like.

Examples of substituents which may be introduced in the phenoxy group of each of the substituted or unsubstituted phenoxy(C1-C8)alkyl group, the substituted or unsubstituted phenoxy(C2-C8)alkenyl group, and the substituted or unsubstituted phenoxy(C2-C8)alkynyl group represented by the $R_1$ or $R_9$ include halogen atoms, C1-C4 alkyl groups which may be substituted with halogens, C1-C4 alkyloxy groups which may be substituted with halogens, C3-C6 cycloalkyl groups, a methyl sulfonyl group, a methoxy group, a nitro group, a cyano group, and the like. The (C1-C8)alkyl group of the substituted or unsubstituted phenoxy(C1-C8)alkyl group, the (C2-C8)alkenyl group of the substituted or unsubstituted phenoxy(C2-C8)alkenyl group, and the (C2-C8)alkynyl group of the substituted or unsubstituted phenoxy(C2-C8)alkynyl group may be linear, branched, or cyclic. Examples thereof include a phenoxymethyl group, a 1-phenoxyethyl group, a 2-phenoxyethyl group, a 2-phenoxyethenyl group, a 2-phenoxyethynyl group, a 1-phenoxy-1-methylethyl group, a 1-phenoxycyclopropyl group, a 2-(3-methoxyphenoxy)ethyl group, a 3-phenoxy-2-propenyl group, and the like. $R_1$ is preferably a phenoxymethyl group.

The heterocyclyl of each of the substituted or unsubstituted heterocyclyl(C1-C8)alkyl group, the substituted or unsubstituted heterocyclyl(C2-C8)alkenyl group, and the substituted or unsubstituted heterocyclyl (C2-C8) alkynyl group represented by the $R_1$or $R_9$ represents a 3- to 10-membered aromatic heterocyclyl group containing one or more hetero atoms such as oxygen atoms, sulfur atoms, and nitrogen atoms. Examples of the heterocyclyl include furanyl, thienyl, pyrazolyl, thiazolyl, imidazolyl, triazolyl, pyridyl, pyrimidinyl, quinolinyl, and the like. Examples of substituents which may be introduced in the heterocyclyl include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like. The (C1-C8)alkyl group of the substituted or unsubstituted heterocyclyl(C1-C8)alkyl group, the (C2-C8)alkenyl group of the substituted or unsubstituted heterocyclyl(C2-C8)alkenyl group, and the (C2-C8)alkynyl group of the substituted or unsubstituted heterocyclyl(C2-C8)alkynylgroup may be linear, branched, or cyclic. Examples of the substituted or unsubstituted heterocyclyl(C1-C8)alkyl group, the substituted or unsubstituted heterocyclyl(C2-C8)alkenyl group, and the substituted or unsubstituted heterocyclyl(C2-C8)alkynyl group include a 2-furanylmethyl group, a 2-(2-furanyl)ethyl group, a 2-(3-furanyl)ethyl group, a 1-pyrazolylmethyl group, a 2-(1-pyrazolyl)ethyl group, a 1-imidazolylmethyl group, a 2-imidazolylethyl group, a 2-thienylmethyl group, a 1-triazolylmethyl group, and a 2-(4-thiazolyl)ethyl group. $R_1$ is preferably a substituted or unsubstituted heterocyclyl(C1-C3)alkyl group or a substituted or unsubstituted heterocyclyl(C2-C3)alkenyl group, and more preferably a substituted or unsubstituted

heterocyclyl(C2-C3)alkyl group. Specific examples thereof include a 2-furanylmethyl group, a 1-pyrazolylmethyl group, a 1-imidazolylmethyl group, a 2-thienylmethyl group, and a 1-triazolylmethyl group. $R_1$ is more preferably a 1-pyrazolylmethyl group, a 2-furanylmethyl group, a 2-thienylmethyl group, a 2-(3-furanyl)ethyl group, or a 2-(2-furanyl)ethenyl group. $R_9$ is preferably a substituted or unsubstituted heterocyclyl(C2-C3)alkyl group, and specifically a 2-furanyl group, a 3-pyridylmethyl group, a 4-pyridylmethyl group, a 3-(2-furanyl)propyl group, or a 3-(2-thienyl)propyl group.

The heterocyclyl of each of the substituted or unsubstituted heterocyclyloxy(C1-C8)alkyl group, the substituted or unsubstituted heterocyclyloxy(C2-C8)alkenyl group, and the substituted or unsubstituted heterocyclyloxy(C2-C8) alkynyl group represented by $R_1$ or $R_9$ represents a 3-10-membered aromatic heterocyclyl containing one or more hetero atoms such as oxygen atoms, sulfur atoms, and nitrogen atoms. Examples of the heterocyclyl include furanyl, thienyl, pyrazolyl, thiazolyl, imidazolyl, triazolyl, pyridyl, pyrimidinyl, quinolinyl, and the like. Examples of substituents which may be introduced in the heterocyclyl include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like. The (C1-C8)alkyl group of the substituted or unsubstituted heterocyclyloxy(C1-C8)alkyl group, the (C2-C8) alkenyl group of the substituted or unsubstituted heterocyclyloxy(C2-C8)alkenyl group, and the (C2-C8) alkynyl group of the substituted or unsubstituted heterocyclyloxy(C2-C8)alkynyl group may be linear, branched, or cyclic. Examples of the substituted or unsubstituted heterocyclyloxy(C1-C8)alkyl group, the substituted or unsubstituted heterocyclyloxy(C2-C8)alkenyl group, and the substituted or unsubstituted heterocyclyloxy (C2-C8)alkynyl group include a 2-furanyloxymethyl group, a 2-(2-furanyl)oxyethyl group, a 2-(3-furanyl)oxyethyl group, a 1-pyrazolyloxymethyl group, a 2-(1-pyrazolyl)oxyethyl group, a 1-imidazolyloxymethyl group, a 2-imidazolyloxyethyl group, a 2-thienyloxymethyl group, a 1-triazolyloxymethyl group, and a 2-(4-thiazolyl)oxyethyl group.

Examples of substituents which may be introduced in the pyridine ring of the substituted pyridylmethyl group represented by $R_3$ or $R_4$ include halogen atoms, C1-C4 alkyl groups which may be substituted with halogens, C1-C4 alkyloxy groups which may be substituted with halogens, C3-C6cycloalkyl groups, amethylsulfonylgroup, a methoxy group, a nitro group, a cyano group, and the like.

The substituted or unsubstituted heterocyclic ring represented by $R_1$ represents a 5- to 10-membered heteroaromatic ring, and specific examples thereof include a quinoline ring, a benzofuran ring, an indole ring, an imidazoline ring, a pyridine ring, a pyrazine ring, a pyridazine ring, a pyrimidine ring, a thiophene ring, a thiazole ring, a tetrahydrofuran ring, a furan ring, and the like. The substituted or unsubstituted heterocyclic ring is preferably a 2,6-dichloro-3-pyridyl group, a 2,6-dichloro-4-pyridyl group, a 2-benzofuranyl group, a 2-imidazolyl group, a 2-pyridyl group, a 3-pyridyl group, a 4-pyridyl group, a 3-quinolinyl group, a 3-thienyl group, a 4-pyridazinyl group, a 5-pyrimidinyl group, or a 3-tetrahydrofuranyl group, and particularly preferably a 2-thienyl group or a 3-thienyl group.

The substituted or unsubstituted aromatic ring represented by $R_1$has the same meaning as C6-C10 aryl.

The (C1-C5)alkylcarbonylamino(C1-C3)alkyl group represented by $R_1$ is a carbonylamino(C1-C3)alkyl group having a linear or branched (C1-C5)alkyl, and specific examples thereof include a methylcarbonylaminoethyl group and the like.

The (C1-C5)alkyloxycarbonylaminooxymethyl group represented by $R_1$ is a carbonylaminooxymethyl group having a linear or branched (C1-C5)alkyloxy group, and specific examples thereof include an ethoxycarbonylaminooxymethyl group, and the like.

The (C1-C5)alkyloxycarbonylaminomethyl group represented by $R_1$ is a carbonylaminomethyl group having a linear or branched (C1-C5)alkyloxy group, and specific examples thereof include a t-butyloxycarbonylaminomethyl group and the like.

The (C1-C5)alkylcarbonyloxymethyl group represented by $R_1$ is a carbonyloxymethyl group having a linear or branched (C1-C5)alkyl group, and specific examples thereof include a methylcarbonyloxymethyl group and the like.

Examples of substituents which may be introduced on the benzene ring of the substituted or unsubstituted benzenesulfonyl group represented by $R_3$ or $R_4$ include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like.

The (C1-C5)alkylamino group represented by $R_3$ or $R_4$ is an amino group having a linear or branched (C1-C5) alkyl, and specific examples thereof include a methylamino group and the like.

Examples of substituents which may be introduced in the phenyl group of the substituted or unsubstituted phenylamino group represented by $R_3$ or $R_4$ include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group, and the like.

The (C1-C5)alkylcarbonylamino group represented by $R_3$ or $R_4$ is an amino group having a linear or branched (C1-C5)alkyl, and specific examples thereof include a methylcarbonylamino group and the like.

Examples of substituents which may be introduced on the benzene ring of the substituted or unsubstituted benzoylamino group represented by $R_3$ or $R_4$ include halogen atoms, C1-C4 alkyl groups which may be substituted with halogens, C1-C4 alkyloxy groups which may be substituted with halogens, C3-C6 cycloalkyl groups, a methylsulfonyl

group, a methoxy group, a nitro group, a cyano group, and the like.

Examples of the ring formed by $NR_3R_4$ include an azetidine ring, a pyrrolidine ring, a piperidine ring, a morpholine ring, and the like.

Unless otherwise noted, the above-described alkyl groups, alkenyl groups, and alkynyl groups may be substituted with halogen atoms.

A preferred mode of the compound represented by the formula (I) is as follows.

Ar represents a heterocyclic group which may have a substituent on a ring thereof, X represents a sulfur atom, and Y represents $COR_1$ or $CO_2R_9$.

When Y is $COR_1$, $R_1$ represents a C1-C4 halogenated alkyl group, a C2-C6 alkenyl group, a C3-C6 alkynyl group, a substituted or unsubstituted phenyl(C1-C4)alkyl group, a substituted or unsubstituted phenyl(C2-C3)alkenyl group, a substituted or unsubstituted (C1-C4)alkoxy(C1-C3)alkyl group, a 3-membered to 7-membered substituted or unsubstituted heterocycloalkyl(C1-C3)alkyl group, a 3-membered to 7-membered substituted or unsubstituted heterocycloalkyl(C1-C3)alkenyl group, a substituted or unsubstituted heterocyclyl(C1-C3)alkyl group, or a substituted or unsubstituted heterocyclyl(C2-C3)alkenyl group.

When Y is $CO_2R_9$, $R_9$represents a C1-C6 halogenated alkyl group, more preferably a C3-C4 halogenated alkyl group; a C3-C6 alkenyl group, more preferably a C5-C6 alkenyl group; a C3-C6 alkynyl group, more preferably a C3-C4 alkynyl group; a substituted or unsubstituted phenyl(C1-C4)alkyl group or a substituted or unsubstituted phenyl(C1-C4)alkenyl group, preferably a substituted or unsubstituted phenyl(C2-C4)alkyl group or a substituted or unsubstituted phenyl(C2-C4)alkenyl group; a methoxy(C3-C4)alkyl group; a 3-membered to 7-membered substituted or unsubstituted heterocycloalkyl(C1-C3)alkyl group, more preferably a 3-membered to 7-membered substituted or unsubstituted heterocycloalkyl(C2-C3)alkyl group; or a substituted or unsubstituted heterocyclyl(C2-C3)alkyl group.

A more preferred mode of the compound represented by formula (I) is as follows.

Ar represents a heterocyclic group represented by the chemical formula (II) or (III), X represents a sulfur atom, and Y represents $COR_1$ or $CO_2R_9$.

When Y is $COR_1$, $R_1$ represents a C1-C3 halogenated alkyl group, a C4-C6 alkenyl group, a C4-C6 alkynyl group, a substituted phenyl(C2-C4)alkyl group, a substituted or unsubstituted phenyl(C2-C3)alkenyl group, a substituted or unsubstituted (C1-C4)alkoxymethyl group, a 3-membered to 7-membered substituted or unsubstituted heterocycloalkyl(C2-C3)alkyl group, a 3-membered to 7-membered substituted or unsubstituted heterocycloalkyl(C2-C3)alkenyl group, a substituted or unsubstituted heterocyclyl(C2-C3)alkyl group, or a substituted or unsubstituted heterocyclyl(C2-C3)alkenyl group.

When Y of the compound represented by formula (I) is $CO_2R_9$, $R_9$ represents a C3-C4 halogenated alkyl group, a C5-C6 alkenyl group, a C3-C4 alkynyl group, a substituted or unsubstituted phenyl(C2-C4)alkyl group, a substituted or unsubstituted phenyl(C2-C4)alkenyl group, a methoxy(C3-C4)alkyl group, a 3-membered to 7-membered substituted or unsubstituted heterocycloalkyl(C2-C3)alkyl group, or a substituted or unsubstituted heterocyclyl(C2-C3)alkyl group.

Tables 2 to 8 show specific examples of the compound of the present invention.

[Table 2]

| Compound No. | Y | X | Ar |
|---|---|---|---|
| 2 | COCH2CF3 | S | ( I I ) |
| 3 | COCMe3 | S | ( I I ) |
| 4 | COCMe(CF3)2 | S | ( I I ) |
| 5 | COCCl3 | S | ( I I ) |
| 6 | CO-phenyl | S | ( I I ) |
| 7 | CO-4-chlorophenyl | S | ( I I ) |
| 8 | CO-4-methylphenyl | S | ( I I ) |
| 9 | CO-cyclohexyl | S | ( I I ) |
| 10 | CO-benzyl | S | ( I I ) |
| 11 | CO-3-quinolinyl | S | ( I I ) |
| 12 | CO-3-indolyl | S | ( I I ) |
| 13 | CO-3-pyridyl | S | ( I I ) |
| 14 | CO-2-pyrazinyl | S | ( I I ) |

(continued)

| Compound No. | Y | X | Ar |
|---|---|---|---|
| 15 | CO-(6-chloro-3-pyridyl) | S | ( I I ) |
| 15 | CO-6-C \| -3-Py | S | ( I I ) |
| 16 | CO-(5-chloro-2-pyrazinyl) | S | ( I I ) |
| 17 | CO-(2,6-dichloro-4-pyridyl} | S | ( I I ) |
| 18 | CO-2-pyridazinyl | S | ( I I ) |
| 20 | CO-(5-bromo-3-pyridyl) | S | ( I I ) |
| 21 | CO-(4-trifluoromethyl-3-pyridyl) | S | ( I I ) |
| 22 | CO-(5,6-dichloro-3-pyridyl) | S | ( I I ) |
| 23 | CO-(2,6-dichloro-3-pyridyl) | S | ( I I ) |
| 24 | CO-(2,3,4,5,6-pentafluorophenyl) | S | ( I I ) |
| 28 | CONH2 | S | ( I I ) |
| 29 | CONHMe | S | ( I I ) |
| 32 | CONHCOPh | S | ( I I ) |
| 34 | CONHCHMe2 | S | ( I I ) |
| 35 | CO-4-morpholinyl | S | ( I I ) |
| 36 | CONH-phenyl | S | ( I I ) |
| 37 | CONH-cyclohexyl | S | ( I I ) |
| 42 | COOMe | S | ( I I ) |
| 43 | COOC3H7-n | S | ( I I ) |
| 44 | COOC3H7-i | S | ( I I ) |
| 45 | COOCH2CH2Cl | S | ( I I ) |
| 46 | COOCH2CH2OMe | S | ( I I ) |
| 47 | COOCH2-phenyl | S | ( I I ) |
| 48 | COO-4-chlorophenyl | S | ( I I ) |
| 49 | COO-2-chlorophenyl | S | ( I I ) |
| 51 | COO-phenyl | S | ( I I ) |
| 51 | COO-phenyl | S | ( I I ) |
| 52 | COO-cyclohexyl | S | ( I I ) |
| 53 | COO-3-naphtalene | S | ( I I ) |
| 57 | COPh | S | ( I I I ) |
| 58 | CHO | S | ( I I ) |
| 59 | CHO | CH2 | ( I I ) |
| 61 | CHO | S | ( I I I ) |
| 62 | CHO | CH2 | ( I I I ) |
| 63 | CO-2-pyrazinyl | CH2 | ( I I ) |
| 64 | CO-(4-trifluoromethyl-3-pyridyl) | CH2 | ( I I ) |
| 65 | CO-2-pyrazinyl | NH | ( I I ) |
| 66 | CO-4-trifluoromethyl-3-pyridyl | NH | ( I I ) |

(continued)

| Compound No. | Y | X | Ar |
|---|---|---|---|
| 67 | COCH(CF3)2 | S | ( I I ) |
| 70 | CO-6-norborna-2-enyl | S | ( I I ) |
| 71 | CO-(1-adamantyl) | S | ( I I ) |
| 72 | COMe | S | ( I I ) |
| 73 | COCH2Cl | S | ( I I ) |
| 74 | COCHCl2 | S | ( I I ) |
| 75 | COCH2Br | S | ( I I ) |
| 76 | COCHF2 | S | ( I I ) |
| 77 | COCClF2 | S | ( I I ) |
| 78 | COCH2OMe | S | ( I I ) |
| 79 | COCH2CN | S | ( I I ) |
| 80 | COCH2O-phenyl | S | ( I I ) |
| 81 | COCH2CH2OMe | S | ( I I ) |
| 82 | COCH2NCS | S | ( I I ) |
| 83 | COCH=CH2 | S | ( I I ) |

[Table 3]

| Compound No. | Y | X | Ar |
|---|---|---|---|
| 84 | COCCl=CCl2 | S | ( I I ) |
| 85 | COCH2CH=CHMe | S | ( I I ) |
| 88 | COCO2CH2CH3 | S | ( I I ) |
| 89 | COCH2SMe | S | ( I I ) |
| 90 | COCH2CH2SMe | S | ( I I ) |
| 93 | CO-2-methylcyclopropyl | S | ( I I ) |
| 94 | CO-1-methylcyclopropyl | S | ( I I ) |
| 96 | CO-2-furanyl | S | ( I I ) |
| 100 | COCH2-3-pyridyl | S | ( I I ) |
| 104 | COCH2-1-imidazolyl | S | ( I I ) |
| 106 | CONHOMe | S | ( I I ) |
| 107 | CONHOEt | S | ( I I ) |
| 110 | CONHCH2-2-pyridyl | S | ( I I ) |
| 111 | COO-4-methoxyphenyl | S | ( I I ) |
| 111 | CONHCH2CH2OMe | S | ( I I ) |
| 113 | COOC2H5 | S | ( I I ) |
| 113 | COOEt | S | ( I I ) |
| 114 | COOCH2CH2F | S | ( I I ) |
| 115 | COOCH2CHF2 | S | ( I I ) |
| 116 | COOCH2CF3 | S | ( I I ) |

(continued)

| Compound No. | Y | X | Ar |
|---|---|---|---|
| 117 | COOCH2CC13 | S | ( I I ) |
| 119 | COOCH2CH2SMe | S | ( I I ) |
| 120 | COOCH2CH2SiMe3 | S | ( I I ) |
| 121 | COOCH2CH2OCH2CF3 | S | ( I I ) |
| 122 | COOCH2CH2CH2F | S | ( I I ) |
| 123 | COOCH(CH2F)2 | S | ( I I ) |
| 124 | COOCH(Me)CF3 | S | ( I I ) |
| 125 | COOCH(CF3)2 | S | ( I I ) |
| 126 | COOCH2CH=CH | S | ( I I ) |
| 127 | COOCH2CCl=CCl2 | S | ( I I ) |
| 128 | COO-n-butyl | S | ( I I ) |
| 129 | COOCH2CHMe2 | S | ( I I ) |
| 130 | COOCH(Me)CH2CH3 | S | ( I I ) |
| 131 | COO-t-butyl | S | ( I I ) |
| 132 | COOCH2CH2CH2CH2F | S | ( I I ) |
| 133 | COOCH2CH2CH2CF3 | S | ( I I ) |
| 134 | COOCH2CF2CF2CF3 | S | ( I I ) |
| 135 | COOCH2CH=CMe2 | S | ( I I ) |
| 139 | COO-CH2-cyclopropyl | S | ( I I ) |
| 140 | COOCH2-2-oxiranyl | S | ( I I ) |
| 141 | COO-cyclobutyl | S | ( I I ) |
| 142 | COO-3-oxetanyl | S | ( I I ) |
| 143 | COO-cycopentyl | S | ( I I ) |
| 144 | COOCH2-3-tetrahydrofuranyl | S | ( I I ) |
| 145 | COOCH2-(2,2-dimethyl-1,3-dioxolan-4-yl) | S | ( I I ) |
| 147 | COOCH2-2-tetrahydropyranyl | S | ( I I ) |
| 148 | COO-4-nitrophenyl | S | ( I I ) |
| 149 | COO-2-methoxyphenyl | S | ( I I ) |
| 150 | COOCH2-3-pyridyl | S | ( I I ) |
| 151 | COOCH2-4-pyridyl | S | ( I I ) |
| 152 | COOCH2-2-furanyl | S | ( I I ) |
| 153 | COCH2-3-furanyl | S | ( I I ) |
| 154 | COCH2-2-thienyl | S | ( I I ) |
| 155 | COCH2-3-thienyl | S | ( I I ) |
| 156 | COO-3-pyridyl | S | ( I I ) |
| 163 | COO-phenyl | S | ( I I I ) |
| 160 | COO-iso-propyl | S | ( I I I ) |
| 167 | COOCHMe2 | CH2 | ( I I ) |

(continued)

| Compound No. | Y | X | Ar |
|---|---|---|---|
| 169 | COOMe | NH | ( I I ) |
| 170 | COOMe | N-Me | ( I I ) |
| 171 | COOCH2C≡CH | S | ( I I ) |
| 173 | COOCH2CH2CH=CH2 | S | ( I I ) |
| 174 | CO-(2,4,6-trimethylphenyl) | S | ( I I ) |
| 175 | CO-4-biphenyl | S | ( I I ) |
| 166 | COCH2tBu | S | ( I I ) |
| 167 | CO-(4-t-buthylphenyl) | S | ( I I ) |

[Table 4]

| Compound No. | Y | X | Ar |
|---|---|---|---|
| 178 | CO-2-biphenyl | S | ( I I ) |
| 179 | CO-2-naphthyl | S | ( I I ) |
| 180 | CO-3-naphthyl | S | ( I I ) |
| 181 | CO-(2-nitro-3-chlorophenyl) | S | ( I I ) |
| 182 | CO-(2-iodophenyl) | S | ( I I ) |
| 183 | CO-3-quinolinyl | S | ( I I ) |
| 184 | COCH2OEt | S | ( I I ) |
| 185 | CO-4-fluorophenyl | S | ( I I ) |
| 186 | CO-cyclopentyl | S | ( I I ) |
| 187 | CO-1-cyclopentenyl | S | ( I I ) |
| 188 | CO-3-cyclopentenyl | S | ( I I ) |
| 189 | CO(CH2)16CH3 | S | ( I I ) |
| 190 | CO-CH2-1-pyrazolyl | S | ( I I ) |
| 191 | CO-CH2-(4-oxo-2-thioxothiazolidin-3-yl) | S | ( I I ) |
| 192 | CO-CH2-rodanine | S | ( I I ) |
| 193 | CO-CH2-(3-methyl-5-oxo-1,2,4-oxadiazol-5(4H)-yl) | S | ( I I ) |
| 194 | COO-CH2-1-cyclopentyl | S | ( I I ) |
| 195 | COO-CH2-3-cyclopentyl | S | ( I I ) |
| 196 | COCH(Br)-t-Bu | S | ( I I ) |
| 197 | COCH2tBu | S | ( I I I ) |
| 198 | CO-4-t-butylphenyl | S | ( I I I ) |
| 199 | CO-3-iodophenyl | S | ( I I ) |
| 200 | COO-4-trifluoromethylpheny | S | ( I I ) |
| 201 | COCH2-cyclohexyl | S | ( I I ) |
| 202 | CO-3,5-dimethylphenyl | S | ( I I ) |
| 203 | CO-2,3-dimethylphenyl | S | ( I I ) |
| 204 | COCH(Me)-phenyl | S | ( I I ) |

(continued)

| Compound No. | Y | X | Ar |
|---|---|---|---|
| 205 | COCH(Me)-phenyl | S | ( I I I ) |
| 206 | COOCH2-2-tetrahydrofurany I | S | ( I I ) |
| 207 | COCH2CH2OEt | S | ( I I ) |
| 208 | COOCH2-3-tetrahydrofuranyl | S | ( I I I ) |
| 209 | COO-3-thietanyl | S | ( I I ) |
| 210 | COO-3-thietanyl | S | ( I I I ) |
| 211 | COO-n-pentyl | S | ( I I ) |
| 212 | COOCH2CH2SOOMe | S | ( I I ) |
| 213 | CO-2, 2-difluorocyclopropyl | S | ( I I ) |
| 214 | CONMeOMe | S | ( I I ) |
| 215 | CONHOCH2CH=CH2 | S | ( I I ) |
| 216 | COCH2CH2CF3 | S | ( I I ) |
| 217 | COCH2CH2NHCOMe | S | ( I I ) |
| 218 | COOCH2CH2OMe | S | ( I I I ) |
| 219 | COO-3-oxetanyl | S | ( I I I ) |
| 220 | COOCH2C≡CMe | S | ( I I ) |
| 221 | COC≡CMe | S | ( I I ) |
| 222 | COCH=CHCH3 | S | ( I I ) |
| 223 | COCH2CH=CH2 | S | ( I I ) |
| 224 | COO- (2, 5-dioxopyrrolidin-1-yl) | S | ( I I ) |
| 225 | CONHCH2COOEt | S | ( I I ) |
| 226 | COO-n-hexyl | S | ( I I ) |
| 227 | COOCH2t-Bu | S | ( I I ) |
| 228 | COO-CH2-Crownether(18-C-6) | S | ( I I ) |
| 229 | COCH2-2-furanyl | S | ( I I ) |
| 230 | COCH2OCOMe | S | ( I I ) |
| 231 | COCH2-4-morpholinyl | S | ( I I ) |
| 232 | COCH2-1- (1, 2, 4-triazolyl) | S | ( I I ) |
| 233 | COCH2ONHCOOEt | S | ( I I ) |
| 234 | CO- (2-oxo-2H-pyran-5-yl) | S | ( I I ) |
| 235 | COCH2-4-imidazolyl | S | ( I I ) |
| 236 | COCH2- (2, 4-dioxothiazolidin-3-yl) | S | ( I I ) |
| 237 | COCH2- (2, 5-dioxoimidazolidin-1-yl) | S | ( I I ) |
| 238 | COOMe | CH2 | ( I I ) |
| 239 | COCF3 | NH | ( I I I ) |
| 240 | COCF3 | S | ( I I ) |
| 241 | COCF3 | S | ( I I I ) |
| 242 | CONHCH2-3-pyridyl | S | ( I I ) |

(continued)

| Compound No. | Y | X | Ar |
|---|---|---|---|
| 243 | CONHCH2CH2SMe | S | ( I I ) |

[Table 5]

| Compound No. | Y | X | Ar |
|---|---|---|---|
| 244 | COO-3-methoxyphenyl | S | ( I I ) |
| 245 | COCH=CH-phenyl | S | ( I I ) |
| 246 | COOCH2CH=CH-phenyl | S | ( I I ) |
| 247 | CO- (2-acetoaminophenyl) | S | ( I I ) |
| 248 | CO- (2-acetoamino-3-methylphenyl) | S | ( I I ) |
| 249 | CO-3-tetrahydrofuranyl | S | ( I I ) |
| 250 | COCF3 | NH | ( I I ) |
| 251 | COOCH2CH2CH2NO2 | S | ( I I ) |
| 252 | CO-4-idophenyl | S | ( I I ) |
| 253 | CO-C (Me) 2-O- (4-chlorophenyl) | S | ( I I ) |
| 254 | COOCH2CH25iMe3 | S | ( I I ) |
| 255 | COOCH2CH2morpholinyl | S | ( I I ) |
| 256 | CO-2-methoxyphenyl | S | ( I I ) |
| 257 | CO-4-methoxyphenyl | S | ( I I ) |
| 258 | CO-3-thienyl | S | ( I I ) |
| 259 | CO-2-chlorophenyl | S | ( I I ) |
| 260 | CO-2-thienyl | S | ( I I ) |
| 261 | CO-4cyclohexylphenyl | S | ( I I ) |
| 262 | CO-2-benzofuranyl | S | ( I I ) |
| 263 | COC≡C-phenyl | S | ( I I ) |
| 264 | CO-3-methoxyphenyl | S | ( I I ) |
| 265 | CO-4-ethylphenyl | S | ( I I ) |
| 266 | CO-4-cyanophenyl | S | ( I I ) |
| 267 | CO-3-methylphenyl | S | ( I I ) |
| 268 | CO-2-fluorophenyl | S | ( I I ) |
| 269 | CO-3-cyanophenyl | S | ( I I ) |
| 270 | CO-2-methylphenyl | S | ( I I ) |
| 271 | CO- (2, 3-dihydro-1H-indenyl) | S | ( I I ) |
| 272 | CO-4-methylsulfonylphenyl | S | ( I I ) |
| 273 | CO-3-chlorophenyl | S | ( I I ) |
| 274 | COCH2CH (Me) 2 | S | ( I I ) |
| 275 | CO-3-nitrophenyl | S | ( I I ) |
| 276 | CO-4-nitrophenyl | S | ( I I ) |
| 277 | COCH2OCH (Me) 2 | S | ( I I ) |

(continued)

| Compound No. | Y | X | Ar |
|---|---|---|---|
| 278 | CO-{ 3- (1, 2, 3, 4-tetrahydronaphthalenyl)] | S | ( I I ) |
| 279 | CO-CH2O-t-Bu | S | ( I I ) |
| 280 | CO-3-fluorophenyl | S | ( I I ) |
| 281 | CO-1-phenylcyclopropyl | S | ( I I ) |
| 282 | CO-C (Me) 2-phenyl | S | ( I I ) |
| 283 | CO (CH2CH2) -phenyl | S | ( I I ) |
| 284 | CO-3-pyrimidinyl | S | ( I I ) |
| 285 | COCH2NHCOO-t-Bu | S | ( I I ) |
| 286 | CO-2-oxirane | S | ( I I ) |
| 287 | CO-3-bromophenyl | S | ( I I ) |
| 288 | CO-3-azetydine | S | ( I I ) |
| 289 | CO-2-pyridyl | S | ( I I ) |
| 290 | CO-4-pyridyl | S | ( I I ) |
| 291 | CO-5-chloro-2-thienyl | S | ( I I ) |
| 292 | CO-2-fluorophenyl | S | ( I I I ) |
| 293 | CO-phenyl | NH | ( I I ) |
| 294 | CO-2-fluorophenyl | NH | ( I I ) |
| 295 | CO-phenyl | S | 4CF3-3pyr |
| 296 | CO-2-fluorophenyl | S | 4CF3-3pyr |
| 297 | COO-phenyl | S | 4CF3-3pyr |
| 298 | CO-2-trifluoromethylphenyl | S | ( I I ) |
| 299 | CO-phenyl | S | 4, 5-dichloro-3pyr |
| 300 | CO-phenyl | S | 4F-3pyr |
| 301 | CO-phenyl | S | 4Cl5F3pyr |
| 302 | CO-phenyl | S | 4Br-3pyr |
| 303 | COOCH2CH2C≡CH | S | ( I I ) |
| 304 | COCH2CH2-2-methoxyphenyl | S | ( I I ) |
| 305 | COCH2CH2-2-methoxyphenyl | S | ( I I I ) |
| 306 | COOCH2CH2CH2C≡CH | S | ( I I ) |
| 307 | COOCH2CH2CH2CH2C≡CH | S | ( I I ) |
| 308 | COCH2CH2C≡CH | S | ( I I ) |
| 309 | COCH2CH2CH2C≡CH | S | ( I I ) |

[Table 6]

| Compound No. | Y | X | Ar |
|---|---|---|---|
| 310 | COOCH2CH2CH2CH2CH=CH2 | S | ( I I ) |
| 311 | COOCH2CH2CH2CH2CH=CH2 | S | ( I I I ) |
| 312 | COCH2CH2CH2CH2C≡CH | S | ( I I ) |

(continued)

| Compound No. | Y | X | Ar |
|---|---|---|---|
| 313 | COCH2CH2CH2CH2C≡CH | S | ( I I I ) |
| 314 | COOCH2CH2CH2CH=CH2 | S | ( I I ) |
| 315 | COOCH2CH2CH2CH=CH2 | S | ( I I I ) |
| 316 | COCH2CH2CH2CH2CH=CH2 | S | ( I I ) |
| 317 | COCH2CH2CH2CH2CH=CH2 | S | ( I I I ) |
| 318 | COCH2CH2CH2CH=CH2 | S | ( I I ) |
| 319 | COCH2CH2CH2CH=CH2 | S | ( I I I ) |
| 320 | COCH2CH2CH=CH2 | S | ( I I ) |
| 321 | COCH2CH2CH=CH2 | S | ( I I I ) |
| 322 | COOCHCH3CF3 | S | ( I I I ) |
| 323 | COCH=CH- (3-thienyl) | S | ( I I ) |
| 324 | COCH=CH- (4- (2-thienyl)-2-thienyl) | S | ( I I ) |
| 325 | COOCH2CH=CH- (3-thienyl) | S | ( I I ) |
| 326 | COCH2- (2-tetrahydrofuranyl) | S | ( I I ) |
| 327 | COCH=CH- (2-furanyl) | S | ( I I ) |
| 328 | COCH2CH2- (2-thienyl) | S | ( I I ) |
| 329 | COOCH2CH2- (2-tetrahydrofuranyl) | S | ( I I ) |
| 330 | COOCH2CH2CH2- (2-thienyl) | S | ( I I ) |
| 331 | COCH=CH- (2-thienyl) | S | ( I I ) |
| 332 | COCH2CH2- (2-furanyl) | S | ( I I ) |
| 333 | COCH2CH2CF3 | S | ( I I ) |
| 334 | COCH2CH2CH2OCH3 | S | ( I I ) |
| 335 | COCH2- (3-tetrahydrofuranyl) | S | ( I I ) |
| 336 | COCH2CH2- (2-tetrahydrofuranyl) | S | ( I I ) |
| 337 | COOCH2CH2CH2- (2-furanyl) | S | ( I I ) |
| 338 | COOCH2CH2- (3-tetrahydrofuranyl) | S | ( I I ) |
| 339 | COCH=CH- (3-furanyl) | S | ( I I ) |
| 340 | COO-CH2CH2CH2-(2-tetrahydrofuranyl) | S | ( I I ) |
| 341 | CO-CH2CH2- (3-furanyl) | S | ( I I ) |
| 342 | COOCH2CH2CH2CF3 | S | ( I I ) |
| 343 | COCH2CH2- (4-methoxyphenyl) | S | ( I I ) |
| 344 | COOCH2CH2CH2OCH3 | S | ( I I ) |
| 345 | COCH2CH2-(3,5-dimethoxyphenyl) | S | ( I I ) |
| 346 | COOCH2CH2CH2CH2OCH3 | S | ( I I ) |
| 347 | COOCH2-(3-methoxyphenyl) | S | ( I I ) |
| 348 | COOCH2CH2-(3-methoxyphenyl) | S | ( I I ) |
| 349 | COOCH2CH2CH2-(3-furanyl) | S | ( I I ) |
| 350 | COCH2CH2-(3-tetrahydrofuranyl) | S | ( I I ) |

(continued)

| Compound No. | Y | X | Ar |
|---|---|---|---|
| 351 | COO-CH2CH2CH2-(3-tetrahydrofuranyl) | S | ( I I ) |
| 352 | COOCH2CH2-(2-thienyl) | S | ( I I ) |
| 353 | COCH2CH2CH2-(2, 3-dihydrobenzo[ b][ 1,4]dioxin-6-yl) | S | ( I I ) |
| 354 | COOCH2CH2-(3-thienyl) | S | ( I I ) |
| 355 | COCH2CH2- (benzo[ d][ 1, 3]dioxol-5-yl) | S | ( I I ) |
| 356 | COOCH2CH2CF3 | S | ( I I ) |
| 357 | COCH2CH2-(2,3-dihydrobenzo[ b][ 1,4]dioxin-6-yl) | S | ( I I ) |
| 358 | COCH2CH2-(3-methoxyphenyl) | S | ( I I ) |
| 359 | COOCH2CH2-(2-furanyl) | S | ( I I ) |
| 360 | COOCH2CH2-(3-furanyl) | S | ( I I ) |
| 361 | COCH2Br | S | ( I I ) |
| 362 | COCH2I | S | ( I I ) |
| 363 | COCHF2 | S | ( I I ) |
| 364 | COCF2Cl | S | ( I I ) |
| 365 | COCC13 | S | ( I I ) |
| 366 | COOCH2-(2-thienyl) | S | ( I I ) |
| 367 | COOCH2-(3-thienyl) | S | ( I I ) |
| 368 | COCF3 | NMe | ( I I ) |
| 369 | COCF3 | S | 3-tetrahydrofuranyl |
| 370 | COCF3 | NMe | ( I I ) |
| 371 | CO-(6-Cl-3-pyridyl) | S | ( I I ) |
| 372 | COCH=CHCF3 | S | ( I I ) |
| 373 | COOCH2CH2CN | S | ( I I ) |
| 374 | COCH2OCH2C≡CH | S | ( I I ) |
| 375 | COCH2CH=CH-(2-furanyl) | S | ( I I ) |

[Table 7]

| Compound No. | Y | X | Ar |
|---|---|---|---|
| 376 | COCH2CH=CH-(2-thienyl) | S | ( I I ) |
| 377 | COCH2CH=CH-(3-furanyl) | S | ( I I ) |
| 378 | COCH2CH=CH-(3-thienyl) | S | ( I I ) |
| 379 | COCH2CH2-(3-thienyl) | S | ( I I ) |
| 380 | COCH2CH2CH=CH-(2-furanyl) | S | ( I I ) |
| 381 | COCH2CH2CH=CH-(2-thienyl) | S | ( I I ) |
| 382 | COCH2CH2CH=CH-(3-furanyl) | S | ( I I ) |
| 383 | COCH2CH2CH=CH-(3-thienyl) | S | ( I I ) |
| 384 | COCH2CH2CH2-(2-furanyl) | S | ( I I ) |
| 385 | COCH2CH2CH2-(2-methoxyphenyl) | S | ( I I ) |

(continued)

| Compound No. | Y | X | Ar |
|---|---|---|---|
| 386 | COCH2CH2CH2-(2-tetrahydrofuranyl) | S | ( I I ) |
| 387 | COCH2CH2CH2-(2-thienyl) | S | ( I I ) |
| 388 | COCH2CH2CH2-(3-furanyl) | S | ( I I ) |
| 389 | COCH2CH2CH2-(3-methoxyphenyl) | S | ( I I ) |
| 390 | COCH2CH2CH2-(3-tetrahydrofuranyl) | S | ( I I ) |
| 391 | COCH2CH2CH2-(3-thienyl) | S | ( I I ) |
| 392 | COCH2CH2CH2CF3 | S | ( I I ) |
| 393 | COCH2CH2CH2CH2-(2-furanyl) | S | ( I I ) |
| 394 | COCH2CH2CH2CH2-(2-tetrahydrofuranyl) | S | ( I I ) |
| 395 | COCH2CH2CH2CH2-(2-thienyl) | S | ( I I ) |
| 396 | COCH2CH2CH2CH2-(3-furanyl) | S | ( I I ) |
| 397 | COCH2CH2CH2CH2-(3-tetrahydrofuranyl) | S | ( I I ) |
| 398 | COCH2CH2CH2CH2- (3-thienyl) | S | ( I I ) |
| 399 | COCH2CH2CH2CH2CF3 | S | ( I I ) |
| 400 | COCH2CH2CH2CH2CH2OMe | S | ( I I ) |
| 401 | COCH2CH2CH2CH2OMe | S | ( I I ) |
| 402 | COOCH2CH2CH=CH-(2-furanyl) | S | ( I I ) |
| 403 | COOCH2CH2CH=CH-(2-thienyl) | S | ( I I ) |
| 404 | COOCH2CH2CH=CH-(3-furanyl) | S | ( I I ) |
| 405 | COOCH2CH2CH=CH-(3-thienyl) | S | ( I I ) |
| 406 | COOCH2CH2CH2-(3-thienyl) | S | ( I I ) |
| 407 | COOCH2CH2CH2CH=CH-(2-furanyl) | S | ( I I ) |
| 408 | COOCH2CH2CH2CH=CH-(2-thienyl) | S | ( I I ) |
| 409 | COOCH2CH2CH2CH=CH-(3-furanyl) | S | ( I I ) |
| 410 | COOCH2CH2CH2CH=CH-(3-thienyl) | S | ( I I ) |
| 411 | COOCH2CH2CH2CH2-(2-furanyl) | S | ( I I ) |
| 412 | COOCH2CH2CH2CH2-(2-tetrahydrofuranyl) | S | ( I I ) |
| 413 | COOCH2CH2CH2CH2-(2-thienyl) | S | ( I I ) |
| 414 | COOCH2CH2CH2CH2-(3-furanyl) | S | ( I I ) |
| 415 | COOCH2CH2CH2CH2-(3-tetrahydrofuranyl) | S | ( I I ) |
| 416 | COOCH2CH2CH2CH2-(3-thienyl) | S | ( I I ) |
| 417 | COOCH2CH2CH2CH2CF3 | S | ( I I ) |
| 418 | COOCH2CH2CH2CH2CH2-(2-furanyl) | S | ( I I ) |
| 419 | COOCH2CH2CH2CH2CH2-(2-tetrahydrofuranyl) | S | ( I I ) |
| 420 | COOCH2CH2CH2CH2CH2-(2-thienyl) | S | ( I I ) |
| 421 | COOCH2CH2CH2CH2CH2-(3-furanyl) | S | ( I I ) |
| 422 | COOCH2CH2CH2CH2CH2-(3-tetrahydrofuranyl) | S | ( I I ) |
| 423 | COOCH2CH2CH2CH2CH2-(3-thienyl) | S | ( I I ) |

(continued)

| Compound No. | Y | X | Ar |
|---|---|---|---|
| 424 | COOCH2CH2CH2CH2CH2CF3 | S | ( I I ) |
| 425 | COOCH2CH2CH2CH2CH2CH2OMe | S | ( I I ) |
| 426 | COOCH2CH2CH2CH2CH2OMe | S | ( I I ) |
| 427 | COCH2OCH2CH2 | S | ( I I ) |
| 428 | COCH2OCH2CH2C=CH | S | ( I I ) |
| 429 | COCH2OCH2CH2CH2CF3 | S | ( I I ) |
| 430 | COCH2OCH2-(2-furanyl) | S | ( I I ) |
| 431 | COCH2OCH2-(3-furanyl) | S | ( I I ) |
| 432 | CO-(4-ethynylphenyl) | S | ( I I ) |
| 433 | CO-CH2OCH2CH2CH=CH2 | S | ( I I ) |
| 434 | CO-CH2OCH2CH=CH2 | S | ( I I ) |
| 435 | COOCH2CH2CH2-(2-methoxyphenyl) | S | ( I I ) |
| 436 | CO-CH2OCH2-(2-tetrahydrofuranyl) | S | ( I I ) |
| 437 | CO-CH2OCH2-(3-tetrahydrofuranyl) | S | ( I I ) |
| 438 | CO-CH2OCH2-(2-thienyl) | S | ( I I ) |
| 439 | CO-CH2OCH2-(3-thienyl) | S | ( I I ) |
| 440 | CO-CH2OCH2-phenyl | S | ( I I ) |
| 441 | CO-CH2OCH2-(2-fluorophenyl) | S | ( I I ) |

[Table 8]

| Compound No. | Y | X | Ar |
|---|---|---|---|
| 442 | CO-CH2OCH2-(3-fluorophenyl) | S | ( I I ) |
| 443 | CO-CH2OCH2-(2-methoxyphenyl) | S | ( I I ) |
| 444 | CO-CH2OCH2-(3-methoxyphenyl) | S | ( I I ) |
| 445 | CO-CH2OCH2-(2-trifluoromethoxyphenyl) | S | ( I I ) |
| 446 | CO-CH2CH2-(2-fluorophenyl) | S | ( I I ) |
| 447 | CO-CH2CH2-(3-fluorophenyl) | S | ( I I ) |

Examples of substituents which may be introduced on the substituted phenyl(C2-C4)alkyl group represented by $R_1$' in the chemical formula (Ia) include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group and the like. The substituents which are introduced in adjacent carbons together may form -O-(CH2)n-O-, wherein n is 1 or 2. The substituents are preferably halogen atoms, C1-C4 alkyl groups which may be substituted with halogens, or C1-C4 alkyloxy groups which may be substituted with halogens. The substituents which are introduced in adjacent carbons together may form -O-(CH2)n-O-, wherein n is 1 or 2. The substituents are more preferably a methoxy group. The substituents which are introduced in adjacent carbons together may form -O-(CH2)n-O-, wherein n is 1 or 2.

Examples of substituents which may be introduced on the 3-membered to 7-membered substituted or unsubstituted heterocycloalkyl(C2-C3)alkyl group represented by $R_1$' include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group and the like.

Examples of substituents which may be introduced on the substituted or unsubstituted heterocyclyl (C2-C3)

alkyl group represented by $R_1$' include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group and the like.

Examples of substituents which may be introduced on the substituted or unsubstituted (C1-C4)alkoxymethyl group represented by $R_1$' include halogen atoms, C3-C6 cycloalkyl groups, a phenyl group, a 3-membered to 7-membered heterocycloalkyl group, a substituted or unsubstituted heterocycle, and the like, and preferably include halogen atoms, a 3-membered to 7-membered heterocycloalkyl group, a heterocycle, and the like. The phenyl group may be substituted with halogens, C1-C4 alkyl groups which may be substituted with halogens, C1-C4 alkyloxy groups which may be substituted with halogens, C3-C6 cycloalkyl groups, a methylsulfonyl group, a methoxy group, a nitro group, or a cyano group. The substituents which are introduced in adjacent carbons together may form -O-(CH2)n-O-, wherein n is 1 or 2. The heterocycloalkyl group and the heterocycle may be substituted with halogens.

Examples of substituents which may be introduced on the substituted or unsubstituted phenyl (C2-C4) alkyl group represented by $R_9$' include halogen atoms, C1-C4 alkyl groups which may be substituted with halogens, C1-C4 alkyloxy groups which may be substituted with halogens, C3-C6cycloalkyl groups, methylsulfonyl group, a methoxy group, a nitro group, a cyano group and the like, and preferably include halogen atoms, C1-C4 alkyl groups which may be substituted with halogens, C1-C4 alkyloxy groups which may be substituted with halogens and the like. The substituents which are introduced in adjacent carbons together may form -O-(CH2)n-O-, wherein n is 1 or 2.

Examples of substituents which may be introduced on the 3-membered to 7-membered substituted or unsubstituted heterocycloalkyl(C2-C3)alkyl group represented by $R_9$' include halogen atoms, C1-C4 alkyl groups which may be substituted with halogens, C1-C4alkyloxygroups which may be substituted with halogens, C3-C6 cycloalkyl groups, a methylsulfonyl group, a methoxy group, a nitro group, a cyano group and the like.

Examples of substituents which may be introduced on the substituted or unsubstituted heterocyclyl (C2-C3) alkyl group represented by $R_9$' include halogen atoms, C1-C4 alkyl groups which may be substituted with halogens, C1-C4 alkyloxy groups which may be substituted with halogens, C3-C6 cycloalkyl groups, a methylsulfonyl group, a methoxy group, a nitro group, a cyano group and the like.

A preferred mode of the compound represented by formula (Ia) is as follows.

Ar' represents a heterocyclic group represented by the chemical formula (II) or (III), X' represents a sulfur atom, and Y' represents $COR_1$' or $CO_2R_9$'.

When Y' is $COR_1$', $R_1$' represents a substituted phenyl(C2-C4)alkyl group, a 3-membered to 7-membered unsubstituted heterocycloalkyl(C2-C3)alkyl group, a unsubstituted heterocycloalkyl(C2-C3)alkyl group, or a substituted or unsubstituted(C1-C4)alkoxymethyl group.

When Y' is $CO_2R_9$', $R_9$' represents a substituted or unsubstituted phenyl(C2-C4)alkyl group, a 3-membered to 7-membered unsubstituted heterocycloalkyl(C2-C3)alkyl group, or a unsubstituted heterocyclyl (C2-C3) alkyl group.

A more preferred mode of the compound represented by formula (Ia) is as follows.

Ar' represents a heterocyclic group represented by the chemical formula (II) or (III), X' represents a sulfur atom, and Y' represents $COR_1$' or $CO_2R_9$'.

When Y' is $COR_1$', $R_1$' represents a phenylethyl group, a tetrahydrofuranyl(C2-C3)alkyl group, a furanyl(C2-C3)alkyl group, a thienyl(C2-C3)alkyl group, a furanylmethyloxymethyl group, or a halogenated (C1-C4)alkyloxymethyl group . The phenylethyl group is substituted with one or two methoxy group. The substituents which are introduced in adjacent carbons together may form -O-(CH2)n-O-, wherein n is 1 or 2.

When Y' is $CO_2R_9$', $R_9$' represents a substituted or unsubstituted phenyl(C2-C4)alkyl group, a 3-membered to 7-membered unsubstituted heterocycloalkyl(C2-C3)alkyl group, or a unsubstituted heterocyclyl(C2-C3)alkylgroup. Examples of substituents which may be introduced include halogen atoms, C1-C4 alkyl groups which may be substituted with halogens, C1-C4 alkyloxy groups which may be substituted with halogens and the like. The substituents which are introduced in adjacent carbons together may form -O-(CH2)n-O-, wherein n is 1 or 2.

The compound represented by the chemical formula (I) and contained in the pest control agent of the present invention can be obtained by the method described in WO2010/001922 (Patent Document 3).

Examples of animal-parasitic pests against which the pest control agent of the present invention is used include ticks (for example, *Amblyomma americanum, Amblyomma maculatum, Boophilus microplus, Dermacentor andersoni, Dermacentor occidentalis, Dermacentor variabilis, Haemaphysalis campanulata, Haemaphysalis flava, Haemaphysalis longicornis, Haemaphysalis megaspinosa, Ixodes nipponensis, Ixodes ovatus, Ixodes pacificus, Ixodes persulcatus, Ixodes ricinus, Ixodes scapularis, Ornithodoros moubata,* and *Rhipicephalus sanguineus);* mites of the family Cheyletidae (for example, *Cheyletiella blakei* and *Cheyletiella yasguri*); mites of the family Demodicidae (for example, *Demodex canis* and *Demodex cati);* mites of the family *Psoroptidae* (for example, *Psoroptes cummunis) ;* mites of the family Sarcoptidae (for example, *Chorioptes bovis* and *Otodectes cynotis) ;* mites of the family Macronyssidae (for example, *Ornithonyssus sylviarum*); *Dermanyssus gallinae,* mites of the Family Analgidae (for example, *Megninia cubitalis* and *Pterolichus obtusus);* mites of the family Trombiculidae (for example, *Helenicula miyagawai* and *Leptotrombidium akamushi*), fleas (for example, *Ctenocephalides felis* and *Pulex irritans*); lice of the order Mallophaga (for example, *Tri-*

*chodectes canis* and *Menopon gallinae*); lice of the order Anoplura (for example, *Haematopinus suis, Linognathus setosus, Pediculus humanus corporis, Pediculus humanus humanus, and Phthirus pubis*), flies (for example, *Hypoderma bovis* and *Stomoxys calcitrans*), horse-flies, trematodes, acanthocephalans, cestodes, nematodes, protozoa, sporozoa, and the like. Preferred examples include animal-parasitic fleas, mites, and ticks, and more preferred examples include animal-parasitic mites and ticks. Examples of public health pests, nuisance pests, stored-grain pests, stored-product pests, and house and household pests against which the pest control agent of the present invention is used include mosquitoes (for example, Aedes *albopictus* and *Culex pipiens pallens),* cockroaches (*Periplaneta fuliginosa, Periplaneta japonica,* and *Blattella germanica*), mites of the family Acaridae (for example, *Tyrophagus putrescentiae*), flies (for example, *Musca domestica,* flesh flies (flies of the family Sarcophagidae), moth flies, flies of the family Drosophilidae, flies of the family Chironomidae), insects of the family Simuliidae, insects of the family Ceratopogonidae, insects of the order Hymenoptera (for example, ants such as *Camponotus japonicus,* and *Solenopsis spp.* and wasps such as *Vespa mandarina*), arthropods of the order Isopoda (for example, *Porcellio scaber, Ligia exotica,* and *Armadillidium vulgare*), insects of the order Hemiptera (for example, *Cimex lectularius*), arthropods of the subphylum Myriapoda (for example, centipedes, house centipedes, and millipedes), arthropods of the order Araneae (for example, *Heteropoda venatoria*), insects of the order Coleoptera (for example, ground beetles), arthropods of the order Collembola (for example, *Onychiurus folsomi*), insects of the order Dermaptera (for example, *Labidura riparia),* insects of the order Orthoptera (for example, insects of the family Rhaphidophoridae), insects of the order Coleoptera (for example, *Callosobruchus chinensis, Sitophilus zeamais, Tenebroidesmauritanicus, Tribolium castaneum,* insects of the family Ptinidae, insects of the family Anobiidae, insects of the family Scolytidae, insects of the family Dermestidae, and Chlorophorus diadema), insects of the order Lepidoptera (for example, insects of the family Pyralidae, and clothes moths), insects of the family Cucujidae, insects of the order Isoptera (for example, *Coptotermes formasanus, Incisitermes minor,* and *Odontotermes formosanus),* insects of the order Thysanura (for example, *Ctenolepisma villosa*), and the like.

For preparation of the pest control agent of the present invention, existing agents can also be blended in addition to the compound represented by the chemical formula (I).

For preparation of the pest control agent of the present invention, a carrier selected depending on how the pest control agent is used can be used in addition to the compound represented by the chemical formula (I).

Examples of usable carriers include liquid carriers, solid carriers, gas carriers, surfactants, dispersing agents, other auxiliary agents for formulation use, and the like.

Examples of the solid carriers include fine powders or particles of clays (kaolin clay, diatomite, bentonite, acid clay, and the like), synthetic hydrated silicon oxide, talc, ceramics, other inorganic minerals (selenite, quartz, sulfur, activated carbon, calcium carbonate, hydrated silica, and the like), and the like; starches; lactose; cellulose; synthetic polymers such as vinyl chloride-based polymers and polyurethane; foods; animal feeds (animal feed cakes, oil cakes; grain powders; coarse grain powders; and the like).

Examples of the liquid carriers include alcohols (methanol, ethanol, isopropanol, polyethylene glycol, propylene glycol, dipropylene glycol, tripropylene glycol, glycerin, and the like), ketones (acetone, methyl ethyl ketone, and the like), aromatic hydrocarbons (benzyl alcohol, benzene, toluene, xylene, ethylbenzene, methylnaphthalene, and the like), aliphatic hydrocarbons (paraffin, n-hexane, cyclohexane, kerosene, lamp oil, and the like), ethers (diethylene glycol monoethyl ether, diethylene glycol monomethyl ether, diisopropyl ether, diethyl ether, dioxane, tetrahydrofuran, and the like), esters (propylene carbonate, ethyl acetate, butyl acetate, benzyl benzoate, isopropyl myristate, fatty acid esters of propylene glycol, and the like), nitriles (acetonitrile, isobutyronitrile, and the like), amides (dimethylformamide, dimethylacetamide, N-methylpyrrolidone, and the like), halogenated hydrocarbons (dichloromethane, trichloroethane, carbon tetrachloride, and the like), animal or vegetable oils such as soybean oil and cottonseed oil, dimethyl sulfoxide, silicone oils, higher fatty acids, glycerol formal, water, and the like.

Examples of the gas carriers include LPG, air, nitrogen, carbon dioxide, dimethyl ether, and the like.

Examples of the surfactants and dispersing agents used for emulsifying, dispersing, spreading, or the like include alkyl sulfuric acid esters, alkyl(aryl)sulfonic acid salts, polyoxyalkylene alkyl(aryl) ethers, polyvalent alcohol esters, lignin sulfonic acid salts, and the like.

In addition, examples of the auxiliary agents used to improve the properties of the preparation include carboxymethyl cellulose, gumarabic, polyethylene glycol, calcium stearate, and the like.

The above-described carriers, surfactants, dispersing agents, and auxiliary agents are used alone or in combination on an as-needed basis.

The pest control agent of the present invention is provided in various forms such as a liquid, a wettable powder, an emulsion, pellets, water dispersible granules, liquefied drops (a spot-on agent, a pour-on agent), a spray, a foam formulation, an aerosol, tablets, granules, fine granules, a powder, capsules, chewables, an injection, suppositories, a cream, a shampoo, a rinse, a resinous formulation, a fumigant, a toxic bait, and the like.

When the pest control agent of the present invention is a control agent against an animal-parasitic pest, preferred forms are a liquid, an emulsion, liquefied drops (a spot-on agent, a pour-on agent), a spray, a foam formulation, an aerosol, tablets, granules, fine granules, a powder, capsules, chewables, an injection, a suppository, a cream, a shampoo,

a rinse, a resinous formulation, a fumigant, a toxic bait, and the like, and particularly preferred forms are a liquid, and liquefied drops (a spot-on agent, a pour-on agent).

The liquid can be blended further with ordinary auxiliary agents for formulation use such as emulsifiers, dispersing agents, spreading agents, wetting agents, suspending agents, stabilizers, preservatives, and propellants. Moreover, ordinary film-forming agents can also be blended. Examples of the surfactants used for emulsifying, dispersing, spreading, or the like include soaps, polyoxyalkylene alkyl(aryl) ethers, polyoxyethylene alkylallyl ethers, polyoxyethylene fatty acid esters, higher alcohols, alkylarylsulfonic acid salts, and the like. Examples of the dispersing agents include casein, gelatin, polysaccharides, lignin derivatives, saccharides, synthetic water-soluble polymers, and the like. Examples of the spreading and wetting agents include glycerin, polyethylene glycol, and the like. Examples of the suspending agents include casein, gelatin, hydroxypropylcellulose, gum arabic, and the like. Examples of the stabilizers include phenol-based antioxidants (BHT, BHA, and the like), amine-based antioxidants (diphenylamine and the like), organic-sulfur-based antioxidants, and the like. Examples of the preservatives include methylparaben, ethylparaben, propylparaben, butylparaben, and the like. The above-described carriers, surfactants, dispersing agents, and the auxiliary agents are used alone or in combination on an as-needed basis. The liquid may further contain a flavor, a fragrance, a synergist, and the like. An appropriate amount of the active ingredient contained in the pest control agent of the present invention is generally 1 to 75% by weight, when the pest control agent is a liquid.

The liquefied drops (the spot-on agent, the pour-on agent) can be prepared as follows. Specifically, the active ingredient of the present invention is dissolved, suspended or emulsified in a liquid carrier applicable onto the skin. Then an absorption enhancer, a coloring agent, a preservative, and the like are added thereto, if needed. Here, examples of the liquid carrier applicable onto the skin include aliphatic hydrocarbons such as alcohols and paraffin, esters such as propylene glycol fatty acid esters, animal or vegetable oils, water, and the like.

The tablets, the granules, the fine granules, the powder, the capsules, and the chewables can be prepared as follows. Specifically, the active ingredient of the present invention is divided into small portions. Then each of the portions is mixed with an excipient such as starch or lactose, and, if needed, a disintegrator such as cellulose, a binder such as gum arabic or hydroxypropylcellulose, and a lubricant such as magnesium stearate or talc are added thereto. Then, the mixture is tableted, molded, pelletized, granulated, or capsulated depending on the formulation form. An appropriate amount of the active ingredient contained in the pest control agent of the present invention is generally 0.1 to 50% by weight.

The carrier used for preparation of the injection needs to be prepared as an aseptic solution. The solution may contain other substances, for example, salt enough to make the solution isotonic to the blood, glucose, or the like. Examples of usable carriers include esters such as glycerides, benzyl benzoate, isopropyl myristate, and derivatives of propylene glycol and fatty acids; and organic solvent such as N-methylpyrrolidone and glycerol formal. An appropriate amount of the active ingredient contained in the pest control agent of the present invention is generally 0.01 to 10% by weight, when the pest control agent is an injection.

Examples of the carrier used for preparation of the cream include non-volatile hydrocarbons (liquid paraffin and the like), fats obtained by hydrogenating lanolin, higher fatty acids, fatty acid esters, animal or vegetable oils, silicone oils, water, and the like. Moreover, emulsifiers, humectants, antioxidants, fragrances, borax, and ultraviolet absorbers are used alone or in combination, if needed. Examples of the emulsifiers include fatty acid sorbitan esters, polyoxyethylene alkyl ethers, fatty acid polyoxyethylene esters, and the like. An appropriate amount of the active ingredient contained in the pest control agent of the present invention is generally 0.5 to 70% by weight, when the pest control agent is a cream.

Examples of the carrier used for preparation of the resinous formulation include vinyl chloride-based polymers and polyurethane. If needed, a plasticizer such as a phthalic acid ester, an adipic acid ester, or stearic acid can be added to such a base material. The active ingredient is kneaded with the base material, and then the mixture is molded by injection molding, extrusion molding, press molding, or the like. Further, an animal ear tag or an insect repellent collar for an animal can be formed through additional appropriate processes such as molding and cutting.

Examples of the carrier used for the toxic bait include foods, animal feeds, attracting substances (grain powders such as wheat flour and maize powder; starches such as corn starch and potato starch; saccharides such as granulated sugar, maltose, and honey; food flavors such as glycerin, onion flavor, and milk flavor; animal powders such as pupa powders and fish powders; and various pheromones), and the like. An appropriate amount of the active ingredient contained in the pest control agent of the present invention is generally 0.0001 to 90% by weight, when the pest control agent is a toxic bait.

The pest control agent of the present invention enables the control of a pest, for example, by orally administering or injecting the pest control agent into the body of an animal to be treated; by administering the pest control agent to the entirety or part of the body surface of an animal to be treated; or by coating with the pest control agent a region where the pest is expected to enter, parasitize, or move about.

The pest control agent of the present invention may be used as it is, or in some cases can be used after diluted with water, a liquid carrier, a commercially available shampoo, rinse, feed, or bedding material for a stable, or the like.

[Examples]

Hereinafter, the present invention will be described specifically on the basis of Examples. However, the present invention is not limited to these Examples.

[Synthesis Examples]

Synthesis Example 1: Compound No.345

[Chem. 7]

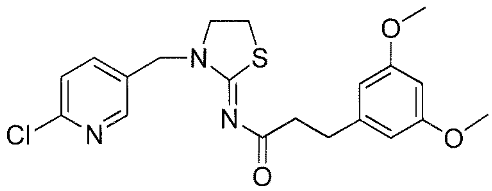

Into 3 ml of anhydrous dichloromethane, 114 mg (0.50 mmol) of 3-(2-chloro-5-pyridinylmethyl)-2-iminothiazolidine synthesized in accordance with the method described in Journal of Medicinal Chemistry 42(12), 2227, (1999), 96 mg (0.50 mmol) of 1-ethyl-3-(3-dimethylaminopropyl)carbodiimide hydrochloride (EDC-HCl), and 65 mg (0.53 mmol) of 4-dimethylaminopyridine (DMAP)were dissolved. To this solution, 105 mg (0.50 mmol) of 3-(3,5-dimethoxyphenyl)propionic acid was added, followed by stirring at room temperature over night. After completion of the reaction, the reaction liquid was diluted with dichloromethane, washed with 1% NaOH aq. and with 1% HCl aq. in this order, and dried over anhydrous magnesium sulfate, followed by evaporation of the solvent. The residue was purified on preparative TLC plates (developed twice on two 0.5-mm plates with hexane:ethyl acetate=1:1). Thus, the target product was obtained. Yield: 134 mg (Percentage Yield: 63%).

Synthesis Example 2: Compound No.309

[Chem. 8]

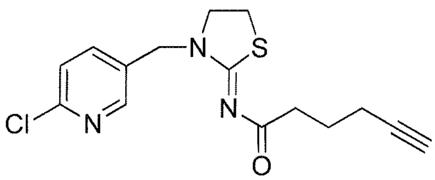

Into 3 ml of anhydrous dichloromethane, 15 mg (0.13 mmol) of 5-hexynoic acid was dissolved. To this solution, 30 mg (0.13 mmol) of 3-(2-chloro-5-pyridinylmethyl)-2-iminothiazolidine, 29 mg (0.15 mmol) of EDC-HCl, and 20 mg (0.16 mmol) of DMAP were added in this order, followed by stirring at room temperature for one hour. After completion of the reaction, the reaction liquid was diluted with dichloromethane, washed with 1% NaOH aq. and 1% HCl aq. in this order, and dried over anhydrous magnesium sulfate, followed by evaporation of the solvent. The residue was purified on a preparative TLC plate (developed on one 0.5-mm plate with 100% Ethyl acetate). Thus, the target product was obtained. Yield: 16 mg (Percentage Yield: 40%).

Synthesis Example 3: Compound No.340

[Chem. 9]

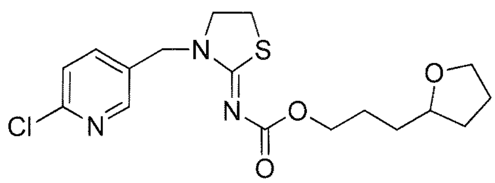

Into 10 ml of anhydrous dichloromethane, 104 mg (0.80 mmol) of 3-(tetrahydrofuran-2-yl)propan-1-ol was dissolved. To this solution, 132 μl (96 mg, 0.96 mmol) of triethylamine and 190 mg (0.80 mmol) of 4-nitrophenyl chloroformate were added in this order, followed by stirring at room temperature for one hour. After completion of the reaction, the reaction liquid was diluted with dichloromethane, washed with 1% HCl aq., and dried over anhydrous magnesium sulfate, followed by concentration under vacuum. The residue was purified by silica gel column chromatography (hexane: ethyl acetate=1:4). Thus, 54 mg of 4-nitrophenyl 3-(tetrahydrofuran-2-yl)propyl carbonate was obtained (Percentage Yield: 34%).

Separately, 28 mg (0.12 mmol) of 3-(2-chloro-5-pyridinylmethyl)-2-iminothiazolidine was dissolved into 7 ml of anhydrous acetonitrile. To this solution, 36 mg (0.12 mmol) of 4-nitrophenyl 3-(tetrahydrofuran-2-yl)propyl carbonate obtained by the above-described method and 20 mg (0.14 mmol) of potassium carbonate were added in this order, followed by stirring at 60°C for six hours. After completion of the reaction, the insoluble materials were removed by vacuum filtration using Celite, and the filtrate was concentrated under vacuum. The residue was dissolved in ethyl acetate, washed with 1% NaOH aq. and with 1% HCl aq. in this order, and dried over anhydrous magnesium sulfate, followed by concentration under vacuum. The residue was purified on a preparative TLC plate (developed on one 0. 5-mm plate with hexane:ethyl acetate=1:3). Thus, the target product was obtained. Yield: 16 mg (Percentage Yield: 35%).

Synthesis Example 4: Compound No.346

[Chem. 10]

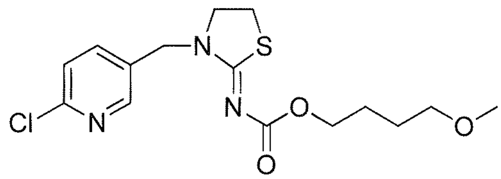

Into 1.0 ml of anhydrous dichloromethane, 92 mg (0.88 mmol) of 4-methoxybutan-1-ol was dissolved. To this solution, 122 μl (89 mg, 0.88 mmol) of triethylamine was added, followed by stirring at 0°C for 30 minutes. To this mixture, 161 mg (0.80 mmol) of 4-nitrophenylchloroformate dissolved in 2 ml of anhydrous dichloromethane was added, followed by stirring at room temperature for one day. After completion of the reaction, the reaction liquid was poured into 25 ml of water, and extracted with chloroform three times. The organic layers combined were washed twice with saturated aqueous sodium hydrogen carbonate, and once with saturated aqueous sodium chloride, and then dried over anhydrous sodium sulfate, followed by evaporation of the solvent. Thus, 188 mg of 4-methoxybutyl 4-nitrophenyl carbonate was obtained (Percentage Yield: 79%).

Into 7 ml of anhydrous acetonitrile, 148 mg (0.50 mmol) of the obtained 4-methoxybutyl 4-nitrophenyl carbonate was dissolved. To this solution, 114 mg (0.50 mmol) of 3-(2-chloro-5-pyridinylmethyl)-2-iminothiazolidine and 80 mg (0.58 mmol) of potassium carbonate were added in this order, followed by stirring at 50°C for 14 hours and at room temperature for 90 hours. After completion of the reaction, the reaction liquid was poured into 30 ml of water, and extracted twice with ethyl acetate. The ethyl acetate layers combined were washed once with water, and once with saturated aqueous sodium chloride, and dried over anhydrous magnesium sulfate, followed by concentration under vacuum. Thus, 222 mg of a crude product was obtained. Of the crude product, 163 mg was purified on preparative TLC plates (developed on two 0.25-mmplates with hexane:ethyl acetate=1:1). Thus, 102 mg of the target product was obtained (Percentage Yield: 78%).

Synthesis Example 5: Compound No.257

[Chem. 11]

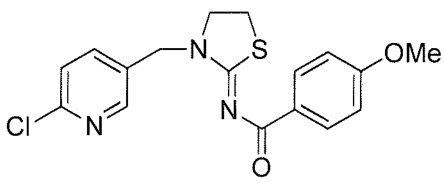

Into 10 ml of anhydrous acetonitrile, 100 mg (0.44 mmol) of 3-(2-chloro-5-pyridinylmethyl)-2-iminothiazolidine was dissolved. To this solution, 87 μl (61 mg, 0.57 mmol) of triethylamine and 82 mg (0.48 mmol) of 4-methoxybenzyl chloride were added in this order, followed by stirring at room temperature overnight. After completion of the reaction, acetonitrile was evaporated under vacuum. Upon addition of methanol, crystals were precipitated. The crystals were collected by filtration, washed well with methanol, and dried. Thus, the target product was obtained. Yield: 112 mg, (Percentage Yield: 71%)

Synthesis Example 6: Compound No.215

[Chem. 12]

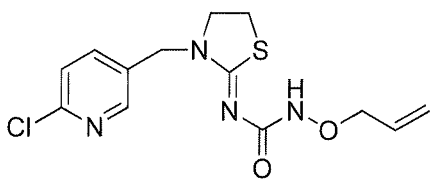

Into 15 ml of acetonitrile, 486 mg (2.0 mmol) of 3-(2-chloro-5-pyridinylmethyl)-2-iminothiazolidine was dissolved, and 260 mg (2.5 mmol) of triethylamine was added thereto. To the mixture being cooled on ice, a solution obtained by dissolving 406 mg (2.0 mmol) of 4-nitrophenyl chloroformate in 5 ml of dichloromethane was added dropwise, followed by reflux by heating for 10 hours. After completion of the reaction, acetonitrile was removed under vacuum. To the residue, 1% HCl aq. and ethyl acetate were added, and the resultant crystals were collected by filtration. The filtrate was washed with 1% HCl aq., water, saturated aqueous sodium hydrogen carbonate, and water in this order, and then dried over anhydrous magnesium sulfate. Thereafter, ethyl acetate was evaporated. Crystals precipitated upon addition of ether were collected by filtration and combined with the aforementioned crystals. Thus, (Z)-4-nitrophenyl 3-((6-chloropyridin-3-ly)methyl)thiazolidin-2-ylidenec arbamate was obtained. Yield: 624 mg (Percentage Yield: 82%).

Into 20 ml of acetonitrile, 395 mg (1 mmol) of (Z)-4-nitrophenyl 3-((6-chloropyridin-3-yl)methyl)thiazolidin-2-ylidenec arbamate obtained by the above-described method and 164 mg (2.0 mmol) of sodium acetate were dissolved. To the solution being cooled on ice, a solution obtained by dissolving o-allylhydroxylamine hydrochloride dissolved into acetonitrile was added dropwise with stirring, followed by reflux by heating for 3 hours. After completion of the reaction, the solvent was evaporated under vacuum, and the residue was dissolved in ethyl acetate. The solution was washed with 1% HCl aq., water, saturated aqueous sodium hydrogen carbonate, and water, then dried over anhydrous magnesium sulfate, and concentrated under vacuum. As a result, crystals were precipitated. The crystals were collected by filtration. Yield: 203 mg (Percentage Yield: 65%).

Synthesis Example 7: Compound No.230

[Chem. 13]

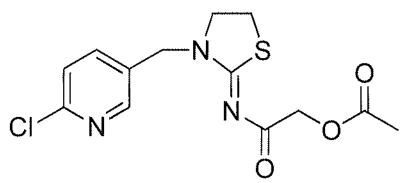

Into 20 ml of anhydrous acetonitrile, 400 mg (1.76 mmol) of 3-(2-chloro-5-pyridinylmethyl)-2-iminothiazolidine was dissolved, and 290 μl (211 mmol, 2.11 mg) of triethylamine was added thereto. To the mixture being cooled on ice, 152 μl (1.76 mmol, 354 mg) of bromoacetyl chloride was added, followed by stirring at room temperature overnight. After completion of the reaction, the reaction liquid was concentrated under vacuum, and the residue was dissolved in dichloromethane. The solution was washed with 1% NaOH aq. and with 1% HCl aq., then dried over anhydrous magnesium sulfate, and concentrated. Then, the residue was purified by silica gel column chromatography (hexane:ethyl acetate=2:3). Thus, (Z)-2-bromo-N-(3-((6-chloropyridin-3-yl)methyl)thiazol idin-2-ylidene)acetamide was obtained. Yield: 321 mg (Percentage Yield: 53%).

Into 2 ml of anhydrous acetonitrile, 66 mg (0.19 mmol) of the (Z)-2-bromo-N-(3-((6-chloropyridin-3-yl)methyl) thiazol idin-2-ylidene) acetamide obtained by the above-described method was dissolved. To this solution, 20 mg (0.22 mmol) of potassium acetate and 32 mg (0.10 mmol) of tetrabutylammonium bromide were added, followed by reflux by heating for 13 hours. After completion of the reaction, the solvent was evaporated under vacuum. As a result, crystals were precipitated. Chloroform was added to the crystals, and the insoluble substance was collected by filtration. Thus, the target product was obtained. Moreover, the filtrate was washed with water, then dried over anhydrous magnesium sulfate, and concentrated under vacuum. Crystals formed upon addition of hexane were collected by filtration and combined with the above-described crystals. Yield: 43 mg (Percentage Yield: 66%).

The compounds listed in Tables 2 to 8 were similarly synthesized. The results of synthesis and materials properties are shown in Tables 9 to 19.

[Table 9]

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 4 | HOOC-C(Me)(CF3)2 | 3-(2-chloro-5-pyridinylmethyl)-2-iminothiazolidine | 1,2 | 1.70 (3H, m), 3.22 (2H, t), 3.67 (2H, t), 4.84 (2H, s), 7.35 (1H, d), 7.65 (1H, dd), 8.33 (1H, d | 1640, 1542 |
| 7 | (4-chlorophenyl)-COCl | 3-(2-chloro-5-pyridinylmethyl)-2-iminothiazolidine | 5 | 3.20 (2H, t), 3.63 (2H, t), 4.97 (2H, s), 7.32 (1H, d), 7.38 (2H, d), 7.69 (1H, dd), 8.19 (2H, d), 8.39 (1H, d) | m/z=366 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 8 | HOOC-(4-methylphenyl) | 3-(2-chloro-5-pyridinylmethyl)-2-iminothiazolidine | 1,2 | 2.41 (3H, s), 3.18 (2H, t), 3.60 (2H, t), 4.97 (2H, s), 7.22 (2H, d), 7.31 (1H, d), 7.72 (1H, d), 8.15 (2H, dd), 8.39 (1H, d) | m/z=346 (M+H) |
| 15 | HOOC-(6-chloro-3-pyridyl) | 3-(2-chloro-5-pyridinylmethyl)-2-iminothiazolidine | 1,2 | 3.23 (2H, t), 3.67 (2H, t), 4.96 (2H, s), 7.31 (1H, d), 7.39 (1H, d), 7.67 (1H, dd), 8.40 (2H, m), 9.22 (1H, d) | 1628 (C=O) |
| 18 | (2-pyridazinyl)-COOH | 3-(2-chloro-5-pyridinylmethyl)-2-iminothiazolidine | 1,2 | 3.27 (2H, t), 3.71 (2H, t), 5.00 (2H, s), 7.35 (1H, d), 7.67 (1H, dd), 8.13 (1H, dd), 8.39 (1H, d), 9.38 (1H, dd), 9.87 (1H, d) | m/z=334 (M+H) |
| 20 | (3-bromo-5-pyridyl)-COCl | 3-(2-chloro-5-pyridinylmethyl)-2-iminothiazolidine | 5 | 3.24 (2H, t), 3.67 (2H, t), 4.98 (2H, s), 7.34 (1H, d), 7.67 (1H, dd), 8.38 (1H, s), 8.59 (1H, s), 8.78 (1H, s), 9.35 (1H, s) | 1624 (C=O) |
| 46 | ClCOOCH2CH2OMe | 3-(2-chloro-5-pyridinylmethyl)-2-iminothiazolidine | 5 | 3.17 (2H, t), 3.43 (3H, s), 3.55 (2H, t), 3.68 (2H, t), 4.32 (2H, t), 4.84 (2H, s), 7.32 (1H, d), 7.66 (1H, dd), 8.30 (1H, d) | 1657 (C=O) |

(continued)

| Compound No. | Chemical Precursor 1 | Chemical Precursor 2 | Synthesis Example | NMR ($CDCl_3$, $\delta$, ppm) | IR (KBr, ν, $cm^{-1}$) or MS |
|---|---|---|---|---|---|
| 53 | HO-3-naphthalene | 3-(2-chloro-5-pyridinylmethyl)-2-iminothiazolidine | 3,4 | 3.23 (2H, t), 3.74 (2H, t), 4.80 (2H, s), 7.30 (1H, d), 7.35 (1H, m), 7.48 (2H, m), 7.61 (1H, m), 7.69 (1H, m), 7.86 (3H, m), 8.37 (1H, s) | 1664, 1546 |
| 65 | (2-pyrazinyl)-COCl | 3-(2-chloro-5-pyridinylmethyl)-2-iminoimidazolidine | 5 | 3.48 (2H, t), 3.78 (2H, t), 4.73 (2H, s), 7.32 (1H, d), 7.72 (1H, d), 8.38 (1H, s), 8.64 (1H, d), 8.70 (1H, d), 8.91 (1H, s), 9.52 (1H, s) | 1.628, 1606 |
| 73 | ClCH2COCl | 3-(2-chloro-5-pyridinylmethyl)-2-iminothiazolidine | 5 | 3.18 (2H, t), 7.62 (2H, t), 4.22 (2H, s), 4.82 (2H, s), 7.33 (1H, d), 7.69 (1H, dd), 8.35 (1H, d) | m/z=304 (M+H) |
| 75 | BrCOCH2Br | 3-(2-chloro-5-pyridinylmethyl)-2-iminothiazolidine | 5 | 3.18 (2H, t), 3.62 (2H, t), 4.02 (2H, s), 4.83 (2H, s), 7.35 (1H, d), 7.72 (1H, dd), 8.36 (1H, d) | 1540, 1532 |
| 76 | CHF2COOH | 3-(2-chloro-5-pyridinylmethyl)-2-iminothiazolidine | 1,2 | 3.23 (2H, t), 3.68 (2H, t), 4.87 (2H, s), 5.94 (1H, t), 7.34 (1H, d), 7.70 (1H, d), 8.36 (1H, s) | 1564, 1548 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 78 | MeOCH2COOH | 3-(2-chloro-5-pyridinylmethyl)-2-iminothiazolidine | 1,2 | 3.15 (2H, t), 3.48 (3H, s), 3.57 (2H, t), 4.17 (2H, s), 4.80 (2H, s), 7.32 (1H, d), 7.64 (1H, dd), 8.34 (1H, d) | m/z=300 (M+H) |
| 104 | BrCOCH2Br imidazole | 3-(2-chloro-5-pyridinylmethyl)-2-iminothiazolidine | 7 | 3.17 (2H, t), 3.60 (2H, t), 4.68 (2H, s), 4.78 (2H, s), 6.98 (1H, s), 7.08 (1H, s), 7.32 (1H, d), 7.42 (1H, dd), 7.53 (1H, s), 8.26 (1H, d) | 1729, 1636, 1536 |
| 111 | MeOCH2CH2NH2 | 3-(2-chloro-5-pyridinylmethyl)-2-iminothiazolidine | 6 | 3.09 (2H, t), 3.37 (2H, s), 3.47 (4H, m), 3.50 (2H, t), 4.71 (2H, s), 7.31 (1H, d), 7.62 (1H, dd), 8.31 (1H, d) | 1607, 1578, 1531 |
| 113 | EtOCOCl | 3-(2-chloro-5-pyridinylmethyl)-2-iminothiazolidine | 5 | 1.36 (3H, t), 3.18 (2H, t), 3.63 (2H, t), 4.24 (2H, q), 4.86 (2H, s), 7.46 (1H, s) | 1664, 1552, 1529 |
| 117 | CCl3CH2OCOCl | 3-(2-chloro-5-pyridinylmethyl)-2-iminothiazolidine | 5 | 3.21 (2H, t), 3.63 (2H, t), 4.84 (2H, s), 4.85 (2H, s), 7.33 (1H, d), 7.67 (1H, dd), 8.33 (1H, d) | 1668, 1536 |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 124 | HO-CH(Me)CF3 | 3-(2-chloro-5-pyridinylmethyl)-2-iminothiazolidine | 3,4 | 1.45, 1.46 (3H, s), 3.19 (2H, t), 3.61 (2H, t), 4.81 (1H, d), 4.85 (1H, d) 5.31 (1H, m), 7.33 (1H, d), 7.66 (1H, d), 8.32 (1H, d) | 1668, 1543 |
| 128 | CH3(CH2)3COCl | 3-(2-chloro-5-pyridinylmethyl)-2-iminothiazolidine | 5 | 0.96 (3H, t), 1.44 (2H, m), 1.72 (2H, m), 3.17 (2H, t), 3.37 (2H, t), 4.19 (2H, t), 4.86 (2H, s), 7.46 (1H, s) | 1661, 1550, 1528 |
| 129 | HO-CH2CHMe2 | 3-(2-chloro-5-pyridinylmethyl)-2-iminothiazolidine | 3,4 | 0.96 (6H, d), 2.04 (1H, m), 3.15 (2H, t), 3.56 (2H, t), 3.95 (2H, d), 4.82 (2H, s), 7.32 (1H, d), 7.66 (1H, dd), 8.32 (1H, d) | 1658, 1550 |
| 131 | t-BuOH | 3-(2-chloro-5-pyridinylmethyl)-2-iminothiazolidine | 3,4 | 1.52 (9H, s), 3.12 (2H, t), 3.51 (2H, t), 4.81 (2H, s), 7.32 (1H, d), 7.63 (1H, dd), 8.31 (1H, d) | 1653, 1560 |
| 134 | HO-CH2CF2CF2CF3 | 3-(2-chloro-5-pyridinylmethyl)-2-iminothiazolidine | 3,4 | 3.21 (2H, t), 3.64 (2H, t), 4.68 (2H, m), 4.82 (2H, s), 7.33 (1H, d), 7.67 (1H, d), 8.33 (1H, s) | 1681, 1552 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 139 | HO-CH2-cyclopropyl | 3-(2-chloro-5-pyridinylmethyl)-2-iminothiazolidine | 3,4 | 0.32 (2H, m), 0.58 (2H, m), 1.24 (1H, m), 3.16 (2H, t), 3.56 (2H, t), 4.00 (2H, d), 4.81 (2H, s), 7.32 (1H, d), 7.65 (1H, d), 8.32 (1H, s) | 1662, 1550 |

[Table 10]

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 140 | HO-CH2-2-oxiranyl | 3-(2-chloro-5-pyridinylmethyl)-2-iminothiazolidine | 3,4 | 2.68 (1H, dd), 2.84 (1H, dd), 3.18 (2H, t), 3.30 (1H, m), 3.61 (2H, t), 4.11 (1H, dd), 4.37 (1H, dd), 4.81 (2H, s), 7.33 (1H, d), 7.77 (1H, dd), 8.32 (1H, d) | 1666, 1550 |
| 141 | HO-cyclobutyl | 3-(2-chloro-5-pyridinylmethyl)-2-iminothiazolidine | 3,4 | 1.61 (1H, m), 1.80 (1H, m), 2.19 (2H, m), 2.39 (2H, m), 3.15 (2H, t), 3.56 (2H, t), 4.82 (2H, s), 5.02 (1H, m), 7.32 (1H, d), 7.64 (1H, dd), 8.32 (1H, d) | 1664, 1548 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 143 | ClCOO-cycopentyl | 3-(2-chloro-5-pyridinylmethyl)-2-iminothiazolidine | 5 | 1.51-1.97 (8H, m), 3.14 (2H, t), 3.54 (2H, t), 4.81 (2H, s), 5.15 (1H, m), 7.32 (1H, d), 7.63 (1H, dd), (8.32 (1H, d) | 1650, 1546 |
| 144 | HO-CH2-3-tetrahydrofuranyl | 3-(2-chloro-5-pyridinylmethyl)-2-iminothiazolidine | 3,4 | 1.65 (1H, m), 2.06 (1H, m), 2.69 (1H, m), 3.17 (2H, t), 3.58 (2H, t), 3.61 (1H, m), 3.76 (1H, m), 4.08 (1H, m), 4.17 (1H, m), 7.33 (1H, d), 7.64 (1H, dd), 8.32 (1H, d) | 1665, 1550 |
| 147 | HO-CH2-2-tetrahydropyranyl | 3-(2-chloro-5-pyridinylmethyl)-2-iminothiazolidine | 3,4 | 1.30-1.92 (6H, m), 3.15 (2H, t), 3.44 (1H, td), 3.55 (2H, t), 3.60 (1H, m), 3.95 (1H, m), 4.23 (2H, d), 4.82 (2H, s), 7.35 (1H, d), 7.66 (1H, dd), 8.30 (1H, d) | 1661, 1543 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 153 | HOOC-CH2-3-fruanyl | 3-(2-chloro-5-pyridinylmethyl)-2-iminothiazolidine | 3,4 | 3.16 (2H, t), 3.57 (2H, t), 4.79 (2H, s), 5.07 (2H, s), 6.50 (1H, s), 7.30 (1H, d), 7.38 (1H, d), 7.52 (1H, s), 7.63 (1H, dd), 8.30 (1H, d) | 1657, 1557 |
| 154 | HOOC-CH2-2-thienyl | 3-(2-chloro-5-pyridinylmethyl)-2-iminothiazolidine | 1,2 | 3.11 (2H, t), 3.55 (2H, t), 3.98 (2H, s), 4.78 (2H, s), 6.93 (2H, m), 7.17 (1H, dd), 7.26 (1H, m), 7.53 (1H, dd), 8.29 (1H, d) | m/z=352 (M+H) |
| 155 | HOOC-CH2-3-thienyl | 3-(2-chloro-5-pyridinylmethyl)-2-iminothiazolidine | 1,2 | 3.11 (2H, t), 3.54 (2H, t), 3.81 (2H, s), 4.75 (2H, s), 7.07 (1H, dd), 7.23 (1H, d), 7.25 (2H, m), 7.47 (1H, dd), 8.27 (1H, d) | m/z=352 (M+H) |
| 156 | (3-pyridyl)-OH | 3-(2-chloro-5-pyridinylmethyl)-2-iminothiazolidine | 3,4 | 3.23 (2H, t), 3.67 (2H, t), 4.84 (2H, s), 7.30-7.37 (2H, m), 7.58 (1H, dd), 7.67 (1H, dd), 8.34 (1H, d), 8.45 (1H, dd), 8.53 (1H, d) | 1682, 1548 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 160 | CH(Me)2OCOCl | 3-(2-chloro-5-thiazolylmethyl)-2-iminothiazolidine | 5 | 1.33 (6H, d), 3.16 (2H, t), 3.62 (2H, t), 4.87 (2H, s), 5.03 (1H, sept), 7.45 (1H, s9 | 1667, 1550, 1530 |
| 163 | PhOCOCl | 3-(2-chloro-5-thiazolylmethyl)-2-iminothiazolidine | 5 | 3.21 (2H, t), 3.69 (2H, t), 4.89 (2H, s), 7.19-7.47 (6H, m) | |
| 167 | HO-CHMe2 | 3-(2-chloro-5-pyridinylmethyl)-2-iminopyrrolidine | 3,4 | 1.31 (6H, d), 2.04 (2H, m), 3.10 (2H, t), 3.32 (2H, t), 4.69 (2H, s), 4.94 (1H, sept), 7.30 (1H, d), 7.62 (1H, d), 8.30 (1H, d) | 1671, 1587 |
| 171 | HO-CH2C=CH | 3-(2-chloro-5-pyridinylmethyl)-2-iminothiazolidine | 3,4 | 2.47 (1H, t), 3.19 (2H, t), 3.61 (2H, t), 4.76 (2H, d), 4.81 (2H, s), 7.33 (1H, d), 7.67 (1H, dd), 8.31 (1H, d) | 1658, 1550 |
| 174 | (2,4,6-trimethylphenyl)-COCl | 3-(2-chloro-5-pyridinylmethyl)-2-iminothiazolidine | 5 | 2.27 (9H, s), 3.18 (2H, t), 3.59 (2H, t), 4.81 (2H, s), 6.83 (2H, s), 7.28 (1H, d), 7.59 (1H, dd), 8.29 (1H, d) | |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 175 | 4-diphenyl-COCl | 3-(2-chloro-5-pyridinylmethyl)-2-iminothiazolidine | 5 | 3.19 (2H, t), 3.63 (2H, t), 4.99 (2H, s), 7.30-7.50 (4H, m), 7.65 (4H, m), 7.73 (1H, dd), 8.32 (2H, d), 8.40 (1H, d) | 1620, 1526, 1407, 1279 |
| 176 | tBuCH2COCl | 3-(2-chloro-5-pyridinylmethyl)-2-iminothiazolidine | 5 | 1.05 (9H, s), 2.40 (2H, s), 3.11 (2H, t), 3.52 (2H, t), 4.80 (2H, s), 7.30 (1H, d), 7.64 (1H, dd), 8.34 (1H, d) | 1625, 1518, 1458, 1400 |
| 177 | HOOC-(4-t-butylphenyl) | 3-(2-chloro-5-pyridinylmethyl)-2-iminothiazolidine | 1,2 | 1.34 (9H, s), 3.17 (2H, t), 3.61 (2H, t), 4.90 (2H, s), 7.30 (1H, d), 7.45 (2H, d), 7.72 (1H, dd), 8.19 (2H, d), 8.40 (1H, d) | 1621, 1529, 1404, 1286 |
| 178 | HOOC-2-diphenyl | 3-(2-chloro-5-pyridinylmethyl)-2-iminothiazolidine | 1,2 | 3.04 (2H, t), 3.38 (2H, t), 4.13 (2H, s), 7.17-7.50 (10H, m), 7.95 (1H, d), 8.08 (1H, s) | 1621, 1528, 1404 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 179 | HOOC-1-naphthyl | 3-(2-chloro-5-pyridinylmethyl)-2-iminothiazolidine | 1,2 | 3.25 (2H, t), 3.75 (2H, t), 4.93 (2H, s), 7.52 (4H, m), 7.81 (1H, dd), 7.95 (1H, dd), 8.05 (1H, d), 8.27 (1H, d), 8.43 (1H, d), 8.89 (1H, m) | 1525, 1402, 1238 |
| 180 | HOOC-2-naphthyl | 3-(2-chloro-5-pyridinylmethyl)-2-iminothiazolidine | 1,2 | 3.21 (2H, t), 3.64 (2H, t), 5.04 (2H, s), 7.33 (1H, d), 7.51 (1H, dd), 7.5 (1H, dd), 7.76 (1H, dd), 7.87 (2H, d), 7.96 (1H, d), 8.30 (1H, dd), 8.44 (1H, d), 8.82 (1H, s) | 1615, 1526, 1402 |
| 181 | (2-nitro-3-chlorophenyl)-COOH | 3-(2-chloro-5-pyridinylmethyl)-2-iminothiazolidine | 1,2 | 3.28 (2H, t), 3.74 (2H, t), 4.90 (2H, s), 7.45 (1H, d), 7.66 (1H, td), 7.77 (1H, d), 7.81 (1H, d), 8.17 (1H, d), 8.41 (1H, s) | 1623, 1545, 1524, 1398 |

(continued)

| Compound No. | Chemical Precursor 1 | Chemical Precursor 2 | Synthesis Example | NMR ($CDCl_3$, $\delta$, ppm) | IR (KBr, ν, $cm^{-1}$) or MS |
|---|---|---|---|---|---|
| 182 | (2-iodophenyl)-COOH | 3-(2-chloro-5-pyridinylmethyl)-2-iminothiazolidine | 1,2 | 3.15 (2H, t), 3.62 (2H, t), 4.90 (2H, s), 7.07 (1H, dd), 7.32 (1H, d), 7.38 (1H, dd), 7.67 (1H, dd), 7.89 (1H, d), 7.95 (1H, d), 8.36 (1H, d) | 1620, 1523, 1404 |
| 183 | (3-quinolinyl)-COOH | 3-(2-chloro-5-pyridinylmethyl)-2-iminothiazolidine | 1,2 | 3.26 (2H, t), 3.81 (2H, t), 5.08 (2H, s), 7.49 (1H, d), 7.60 (1H, dd), 7.85 (1H, dd), 7.90 (1H, dd), 8.23 (1H, s), 8.51 (1H, d), 9.04 (1H, d), 9.53 (1H, d) | 1626, 1614, 1588, 1523, 1456, 1399 |

[Table 11]

| Compound No. | Chemical Precursor 1 | Chemical Precursor 2 | Synthesis Example | NMR ($CDCl_3$, $\delta$, ppm) | IR (KBr, ν, $cm^{-1}$) or MS |
|---|---|---|---|---|---|
| 184 | HOOC-CH2OEt | 3-(2-chloro-5-pyridinylmethyl)-2-iminothiazolidine | 1,2 | 1.27 (3H, t), 3.15 (2H, t), 3.56 (2H, t), 3.63 (2H, q), 4.21 (2H, s), 4.80 (2H, s), 7.32 (1H, d), 7.65 (1H, dd), 8.34 (1H, d) | 1648, 1587 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 185 | HOOC-4-fluorophenyl | 3-(2-chloro-5-pyridinylmethyl)-2-iminothiazolidine | 1,2 | 3.19 (2H, t), 3.62 (2H, t), 4.96 (2H, s), 7.07-7.27 (2H, m), 7.33 (1H, d), 7.70 (1H, dd), 8.28 (2H, m), 8.40 (1H, d) | 1622, 1523 |
| 186 | HOOC-cyclopentyl | 3-(2-chloro-5-pyridinylmethyl)-2-iminothiazolidine | 1,2 | 1. 58 (2H, m), 1.71 (2H, m), 1.82-1.94 (4H, m), 2.90 (1H, m), 3.12 (2H, t), 3.55 (2H, t), 4.84 (2H, s), 7.32 (1H, d), 7.66 (1H, dd), 8.36 (1H, d) | 1626, 1568 |
| 187 | HOOC-1-cyclopentenyl | 3-(2-chloro-5-pyridinylmethyl)-2-iminothiazolidine | 1,2 | 1.97 (2H, t), 2.52 (2H, m), 2.64 (2H, m), 3.12 (2H, t), 3.53 (2H, ), 4.84 (2H, s), 6.90 (1H, m), 7.31 (1H, d), 7.67 (1H, dd), 8.37 (1H, d) | 1633, 1600, 1532 |
| 188 | HOOC-3-cyclopentenyl | 3-(2-chloro-5-pyridinylmethyl)-2-iminothiazolidine | 1,2 | 2.61-2.77 (4H, m), 3.12 (2H, t), 3.28 (1H, m), 3.56 (2H, t), 4.81 (2H, s), 5.67 (2H, m), 7.31 (1H, d), 7.66 (1H, dd), 8.35 (1H, d) | 1632, 1527 |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 189 | CH3(CH2)16COCl | 3-(2-chloro-5-pyridinylmethyl)-2-iminothiazolidine | 5 | 0.87 (3H, t), 1.24 (28H, m), 1.66 (2H, m), 2.47 (2H, t), 3.10 (2H, t), 3.50 (2H, t), 4.80 (2H, s), 7.31 (1H, d), 7.65 (1H, dd), 8.33 (1H, d) | 2918, 2849, 1529, 1399 |
| 190 | BrCOCH2Br, pyrazole | 3-(2-chloro-5-pyridinylmethyl)-2-iminothiazolidine | 7 | 3.14 (2H, t), 3.57 (2H, t), 4.68 (2H, s), 5.03 (2H, s), 6.30 (1H, t), 7.30 (1H, d), 7.52 (3H, m), 8.25 (1H, d) | 1647, 1534 |
| 191 | BrCOCH2Br, rodanine | 3-(2-chloro-5-pyridinylmethyl)-2-iminothiazolidine | 7 | 3.18 (2H, t), 3.62 (2H, t), 3.98 (2H, s), 4.35 (2H, s), 4.82 (2H, s), 7.36 (1H, d), 7.68 (1H, dd), 8.33 (1H, d) | |
| 192 | BrCOCH2Br, rodanine | 3-(2-chloro-5-pyridinylmethyl)-2-iminothiazolidine | 7 | 3.19 (2H, t), 3.63 (2H, t), 3.98 (2H, s), 4.35 (2H, s), 4.82 (2H, s), 7.36 (1H, d), 7.69 (1H, dd), 8.33 (1H, d) | 1728, 1706, 1626, 1542 |
| 193 | BrCOCH2Br, 3-methyl-1,2,4-oxaziazolin-5-on potassium salt | 3-(2-chloro-5-pyridinylmethyl)-2-iminothiazolidine | 7 | 2.20 (3H, s), 3.21 (2H, t), 3.65 (2H, t), 4.39 (2H, s), 4.76 (2H, s), 7.35 (1H, d), 7.59 (1H, dd), 8.29 (1H, d) | 1772, 1641, 1545 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 194 | HO-CH2-1-cyclopentyl | 3-(2-chloro-5-pyridinylmethyl)-2-iminothiazolidine | 3,4 | 1.91 (2H, t), 2.34 (2H, m), 3.17 (2H, t), 3.58 (2H, t), 4.73 (2H, s), 4.82 (2H, s), 5.71 (1H, m), 7.32 (1H, d), 7.6 (1H, dd), 8.32 (1H, d) | 1546 |
| 195 | HO-CH2-3-cyclopentyl | 3-(2-chloro-5-pyridinylmethyl)-2-iminothiazolidine | 3,4 | 2.15 (2H, m), 2.53 (2H, m), 2.73 (1H, m), 3.16 (2H, t), 3.57 (2H, t), 4.08 (2H, d), 4.85 (2H, s), 5.65 (2H, m), 7.33 (1H, d), 7.65 (1H, dd), 8.32 (1H, d) | 1654, 1550 |
| 196 | tBuCH(Br)COCl | 3-(2-chloro-5-pyridinylmethyl)-2-iminothiazolidine | 5 | 1.20 (9H, s), 3.16 (2H, td), 3.61 (2H, t), 4.34 (1H, s), 4.70 (1H, d), 4.90 (1H, d), 7.32 (1H, d), 7.73 (1H, dd), 8.35 (1H, d) | 1627, 1526, 1408 |
| 197 | tBuCH2COCl | 3-(2-chloro-5-thiazolylmethyl)-2-iminothiazolidine | 5 | 1.08 (9H, s), 2.44 (2H, s), 3.12 (2H, t), 3.58 (2H, t), 4.83 (2H, s), 7.47 (1H, s) | 1627, 1513, 1399, 1231 |
| 198 | (4-t-butylphenyl)-COOH | 3-(2-chloro-5-thiazolylmethyl)-2-iminothiazolidine | 1,2 | 1.34 (9H, s), 3.18 (2H, t), 3.66 (2H, t), 4.99 (2H, s), 7.47 (1H, s), 7.49 (2H, d), 8.24 (2H, d) | 1618, 1520, 1408 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 199 | (3-iodophenyl)-COOH | 3-(2-chloro-5-pyridinylmethyl)-2-iminothiazolidine | 1,2 | 3.19 (2H, t), 3.63 (2H, t), 4.99 (2H, s), 7.17 (1H, dd), 7.33 (1H, d), 7.68 (1H, dd), 7.82 (1H, d), 8.20 (1H, d), 8.39 (1H, s), 8.58 (1H, d) | 2360, 1618, 1527, 1417 |
| 201 | cyclohexyl-CH2COCl | 3-(2-chloro-5-pyridinylmethyl)-2-iminothiazolidine | 5 | 0.90-1.31 (4H, m), 1.60-1.76 (4H, m), 1.90 (1H, m), 2.35 (2H, d), 3.12 (2H, t), 3.51 (2H, t), 4.80 (2H, s), 7.30 (1H, d), 7.64 (1H, dd), 8.34 (1H, d) | 2923, 1632, 1526, 1460, 1403 |
| 202 | (3,5-dimethylphenyl)-COOH | 3-(2-chloro-5-pyridinylmethyl)-2-iminothiazolidine | 1,2 | 2.36 (6H, s), 3.15 (2H, t), 3.59 (2H, t), 4.96 (2H, s), 7.14 (1H, s), 7.30 (1H, d), 7.70 (1H, dd), 7.86 (2H, s), 8.38 (1H, d) | 1526, 1430 |
| 203 | (2,3-dimethylphenyl)-COOH | 3-(2-chloro-5-pyridinylmethyl)-2-iminothiazolidine | 1,2 | 2.32 (3H, s), 2.56 (3H, s), 3.17 (2H, t), 3.59 (2H, t), 4.89 (2H, s), 7.11 (1H, dd), 7.23 (1H, d), 7.30 (1H, d), 7.65 (1H, dd), 7.70 (1H, d), 8.35 (1H, d) | 1623, 1524, 1457, 1457, 1401 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 204 | phenyl-CH(Me)-COCl | 3-(2-chloro-5-pyridinylmethyl)-2-iminothiazolidine | 5 | 1.56 (3H, d), 3.08 (2H, m), 3.49 (2H, m), 3.85 (1H, q), 4.51 (1H, d), 4.82 (1H, d), 7.09 (1H, d), 7.20-7.38 (6H, m), 8.20 (1H, d) | 1634, 1530, 1457, 1401 |
| 205 | phenyl-CH(Me)-COCl | 3-(2-chloro-5-thiazolylmethyl)-2-iminothiazolidine | 5 | 164 (3H, d), 3.08 (2H, m), 3.49 (2H, m), 3.85 (1H, q), 4.51 (1H, d), 4.82 (1H, d), 7.45 (1H, s) | 1634, 1530, 1458, 1401 |
| 206 | HO-CH2-2-tetrahydrofran y l | 3-(2-chloro-5-pyridinylmethyl)-2-iminothiazolidine | 3,4 | 1.65 (1H, m), 1.85-2.05 (3H, m), 3..16 (2H, t), 3.57 (2H, t), 3.78 (1H, dd), 3.89 (1H, dd), 4.10-4.23 (3H, m), 4.80 (2H, s), 7.32 (1H, d), 7.66 (1H, dd), 8.30 (1H, d) | 1657, 1549 |

[Table 12]

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 207 | HOOC-CH2CH2OEt | 3-(2-chloro-5-pyridinylmethyl)-2-iminothiazolidine | 1,2 | 1.19 (3H, t), 2.78 (2H, t), 3.12 (2H, t), 3.53 (4H, m), 3.78 (2H, t), 4.81 (2H, t), 7.31 (1H, d), 7.66 (1H, dd), 8.33 (1H, d) | |
| 208 | HO-CH2-3-tetrahydrofranyl | 3-(2-chloro-5-thiazolylmethyl)-2-iminothiazolidine | 3,4 | 1.71 (2H, m), 2.07 (1H, m), 2.71 (1H, m), 3.18 (2H, t), 3.64 (2H, t), 3.78 (1H, m), 3.89 (2H, m), 4.10 (1H, dd), 4.20 (1H, dd), 4.86 (2H, s), 7.46 (1H, s) | 1661, 1549, 1527 |
| 209 | HO-3-thietanyl | 3-(2-chloro-5-pyridinylmethyl)-2-iminothiazolidine | 3,4 | 3.17 (2H, t), 3.34 (2H, m), 3.59 (4H, m), 3.64 (1H, m), 7.33 (1H, d), 7.63 (1H, dd), 8.32 (1H, d) | |
| 210 | HO-3-thietanyl | 3-(2-chloro-5-thiazolylmethyl)-2-iminothiazolidine | 3,4 | 3.19 (2H, t), 3.36 (2H, t), 3.65 (4H, m), 4.86 (2H, s), 5.64 (1H, m), 7.46 (1H, s) | |
| 211 | ClCOO-n-pentyl | 3-(2-chloro-5-pyridinylmethyl)-2-iminothiazolidine | 5 | 0.90 (3H, t), 1.36 (4H, m), 1.73 (2H, m), 3.15 (2H, t), 3.56 (2H, t), 4.16 (2H, t), 4.81 (2H, s), 7.31 (1H, d), 7.65 (1H, dd), 8.31 (1H, d) | 1660, 1561 |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 212 | HO-CH2CH2SOOMe | 3-(2-chloro-5-pyridinylmethyl)-2-iminothiazolidine | 3,4 | 3.01 (3H, s), 3.20 (2H, t), 3.39 (2H, t), 3.61 (2H, t), 4.56 (2H, t), 4.78 (2H, s), 7.32 (1H, d), 7.62 (1H, dd), 8.31 (1H, d) | |
| 213 | HOOC-2,2-difluorocyclopropyl | 3-(2-chloro-5-pyridinylmethyl)-2-iminothiazolidine | 1,2 | 1.68 (1H, m), 2.13 (1H, m), 2.64 (1H, m), 3.15 (2H, t), 3.61 (2H, t), 4.74 (1H, d), 4.91 (1H, d), 7.35 (1H, d), 7.76 (1H, dd), 8.34 (1H, d) | |
| 214 | (CH3ONHCH3)* HCl | 3-(2-chloro-5-pyridinylmethyl)-2-iminothiazolidine | 6 | 3.14 (2H, t), 3.29 (3H, s), 3.57 (2H, t), 3.73 (3H, s), 4.77 (2H, s), 7.33 (1H, d), 7.66 (1H, dd), 8.33 (1H, d) | |
| 215 | (H2NOCH2CH=CH2)* HCl | 3-(2-chloro-5-pyridinylmethyl)-2-iminothiazolidine | 6 | 3.14 (2H, t), 3.54 (2H, t), 4.43 (2H, d), 4.71 (2H, s), 5.28 (1H, d), 5.34 (1H, d), 5.99 (1H, m), 7.31 (1H, d), 7.61 (1H, dd), 8.30 (1H, d) | |
| 216 | HOOC-CH2CH2CF3 | 3-(2-chloro-5-pyridinylmethyl)-2-iminothiazolidine | 1,2 | 2.49 (2H, m), 2.72 (2H, m), 3.15 (2H, m), 3.62 (2H, m), 4.84 (2H, s), 7.32 (1H, d), 7.67 (1H, dd), 8.33 (1H, d) | |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 217 | HOOC-CH2CH2NHCOCH3 | 3-(2-chloro-5-pyridinylmethyl)-2-iminothiazolidine | 1,2 | 1.94 (3H, s), 2.71 (2H, t), 3.16 (2H, t), 3.54 (2H, t), 3.56 (2H, t), 4.81 (2H, s), 6.30 (1H, br s), 7.33 (1H, d), 7.63 (1H, dd), 8.33 (1H, d) | |
| 218 | HO-CH2CH2OMe | 3-(2-chloro-5-thiazolylmethyl)-2-iminothiazolidine | 3,4 | 2.80 (2H, t), 3.13 (2H, t), 3.38 (3H, s), 3.62 (2H, t), 3.77 (2H, t), 4.85 (2H, s), 7.46 (1H, s) | |
| 219 | HO-3-oxetanyl | 3-(2-chloro-5-thiazolylmethyl)-2-iminothiazolidine | 3,4 | 3.28 (2H, t), 3.76 (2H, t), 4.85 (2H, m), 4.90 (2H, s), 5.04 (2H, m), 5.22 (1H, m), 7.54 (1H, s) | |
| 220 | HO-CH2C≡CMe | 3-(2-chloro-5-pyridinylmethyl)-2-iminothiazolidine | 3,4 | 1.85 (3H, t), 3.17 (2H, t), 3.58 (2H, t), 4.73 (2H, t), 4.81 (2H, s), 7.31 (1H, d), 7.66 (1H, dd), 8.31 (1H, d) | |
| 221 | HOOC-C≡C-CH3 | 3-(2-chloro-5-pyridinylmethyl)-2-iminothiazolidine | 1,2 | 2.00 (3H, s), 3.18 (2H, t), 3.61 (2H, t), 4.85 (2H, s), 7.33 (1H, d), 7.68 (1H, dd), 8.34 (1H, d) | |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 222 | HOOC-CH=CH-CH3 | 3-(2-chloro-5-pyridinylmethyl)-2-iminothiazolidine | 1,2 | 1.89 (3H, d), 3.12 (2H, t), 3.56 (2H, t), 4.84 (2H, s), 6.13 (1H, m), 7.06 (1H, m), 7.31 (1H, d), 7.66 (1H, dd), 8.35 (1H, d) | |
| 223 | HOOC-CH2CH=CH2 | 3-(2-chloro-5-pyridinylmethyl)-2-iminothiazolidine | 1,2 | 1.89 (2H, dd), 3.12 (2H, t), 3.26 (1H, m), 3.56 (2H, t), 4.86 (2H, s), 6.08 (1H, m), 7.07 (1H, m), 7.35 (1H, d), 7.71 (1H, dd), 8.36 (1H, d) | |
| 224 | HO-1-pyrrolidinyl-2,5-dione | 3-(2-chloro-5-pyridinylmethyl)-2-iminothiazolidine | 3,4 | 2.76 (4H, s), 3.35 (2H, t), 3.82 (2H, t), 4.81 (2H, s), 7.52 (1H, d), 7.79 (1H, d), 8.39 (1H, d) DMSO-d6 | 1731, 1552 |
| 225 | EtOCOCH2NCO | 3-(2-chloro-5-pyridinylmethyl)-2-iminothiazolidine | another methods | 1.23 (3H, t), 3.08 (2H, m), 3.57 (2H, m), 3.82 (2H, s), 4.10 (2H, m), 4.74 (2H, s), 7.37 (1H, m), 7.77 (1H, m), 8.41 (1H, m) | 1742, 1610, 1578, 1531 |
| 226 | HO-(CH2)5CH3 | 3-(2-chloro-5-pyridinylmethyl)-2-iminothiazolidine | 3,4 | 0.89 (3H, t), 1.31 (6H, m), 1.40 (2H, m), 1.71 (2H, m), 3.15 (2H, t), 3.56 (2H, t), 4.16 (2H, t), 4.81 (2H, s), 7.31 (1H, d), 7.65 (1H, dd), 8.31 (1H, d) | 1724, 1657, 1552 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 227 | HO-CH2tBu | 3-(2-chloro-5-pyridinylmethyl)-2-iminothiazolidine | 3,4 | 0.98 (9H, S), 3.15 (2H, t), 3.56 (2H, t), 3.90 (2H, s), 4.82 (2H, s), 7.32 (1H, d), 7.66 (1H, dd), 8.32 (1H, d) | 1661, 1546 |
| 228 | HO-CH2-Crownether(18-C-6) | 3-(2-chloro-5-pyridinylmethyl)-2-iminothiazolidine | 3,4 | 3.16 (2H, t), 3.57 (2H, t), 3.70 (22H, m), 3.85 (2H, m), 4.23 (2H, dd), 4.80 (2H, dd), 7.32 (1H, d), 7.65 (1H, dd), 8.31 (1H, d) | 1663, 1550 |
| 229 | HOOC-CH2-2-furanyl | 3-(2-chloro-5-pyridinylmethyl)-2-iminothiazolidine | 3,4 | 3.12 (2H, t), 3.56 (2H, t), 3.83 (2H, s), 4.73 (2H, s), 6.20 (1H, d), 6.33 (1H<d), 7.27 (1H, d), 7.34 (1H, d), 7.55 (1H, dd), 8.28 (1H, d) | |

[Table 13]

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 230 | BrCOCH2Br, CH3COOK | 3-(2-chloro-5-pyridinylmethyl)-2-iminothiazolidine | 7 | 2.17 (3H, s), 3.16 (2H, t), 3.57 (2H, t), 4.76 (2H, s), 4.79 (2H, s), 7.33 (1H, d), 7.63 (1H, dd), 8.33 (1H, d) | 1744, 1660, 1537, 1417 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 231 | BrCOCH2Br, morpholine | 3-(2-chloro-5-pyridinylmethyl)-2-iminothiazolidine | 7 | 2.62 (4H, m), 3.14 (2H, t), 3.37 (2H, s), 3.56 (2H, t), 3.77 (4H, t), 4.80 (2H, s), 7.32 (1H, d), 7.64 (1H, dd), 8.34 (1H, d) | 1730, 1646, 1531, 1460, 1409 |
| 232 | BrCOCH2Br, 1,2,4-triazole | 3-(2-chloro-5-pyridinylmethyl)-2-iminothiazolidine | 7 | 3.18 (2H, t), 3.60 (2H, t), 4.68 (2H, s), 5.07 (2H, s), 7.32 (1H, d), 7.50 (1H, dd), 7.95 (1H, s), 8.21 (1H, d), 8.27 (1H, d) | 1632, 1534 |
| 233 | BrCOCH2Br, HONHCOOEt | 3-(2-chloro-5-pyridinylmethyl)-2-iminothiazolidine | 7 | 1.28 (3H, t), 3.19 (2H, t), 3.60 (2H, t), 4.20 (2H, q), 4.56 (2H, s), 4.80 (2H, s), 7.33 (1H, d), 7.61 (1H, dd), 8.32 (1H, d) | 1733, 1707, 1647, 1596, 1534 |
| 234 | HOOC-5-(2H-pyranyl-2-one) | 3-(2-chloro-5-pyridinylmethyl)-2-iminothiazolidine | 1,2 | 3.22 (2H, t), 3.64 (2H, t), 4.89 (2H, s), 6.32 (1H, d), 7.34 (1H, d), 7.61 (1H, d), 8.02 (1H, d), 8.36 (1H, s), 8.51 (1H, s) | 1730, 1638, 1528 |
| 235 | BrCOCH2Br, imidazole· HCl | 3-(2-chloro-5-pyridinylmethyl)-2-iminothiazolidine | 7 | 3.12 (2H, t), 3.56 (2H, t), 3.83 (2H, d), 4.77 (2H, s), 6.92 (aH, d), 7.30 (1H, m), 7.57 (2H m), 8.29 (1H, d) | 1621, 1530 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 236 | BrCOCH2Br, thiazolidinedione | 3-(2-chloro-5-pyridinylmethyl)-2-iminothiazolidine | 7 | 3.16 (2H, t), 3.59 (2H, t), 4.00 (2H, s), 4.47 (2H, s), 4.79 (2H, s), 7.36 (1H, d), 7.63 (1H, dd), 8.32 (1H, d) | 1748, 1683, 1644, 1537 |
| 237 | BrCOCH2Br, imidazolidine-2,4-dione | 3-(2-chloro-5-pyridinylmethyl)-2-iminothiazolidine | 7 | 3.19 (2H, t), 3.68 (2H, t), 3.96 (2H, s), 4.11 (2H, s), 4.78 (2H, s), 7.52 (1H, d), 7.80 (1H, dd), 8.09 (1H, s), 8.40 (1H, d) DMSO-d6 | 1713, 1529 |
| 242 | H2N-CH2-(3-pyridyl) | 3-(2-chloro-5-pyridinylmethyl)-2-iminothiazolidine | 6 | 3.11 (2H, t), 3.51 (2H, t), 4.52 (2H, d), 4.70 (2H, s), 7.19-7.31 (3H, m), 7.60 (1H, dd), 7.70 (1H, d), 8.31 (1H, d), 8.50 (1H, d), 8.56 (1H, s) | 1626, 1524 |
| 243 | H2N-CH2CH2SMe | 3-(2-chloro-5-pyridinylmethyl)-2-iminothiazolidine | 6 | 2.13 (3H, s), 2.69 (2H, t), 3.10 (2H, t), 3.49 (4H, m), 4.76 (2H, s), 5.61 (br s), 7.30 (1H, d), 7.61 (1H, dd), 8.31 (1H, d) | 1605, 1577, 1521, 1507 |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 245 | HOOC-CH=CH-phenyl | 3-(2-chloro-5-pyridinylmethyl)-2-iminothiazolidine | 1,2 | 3.14 (2H, t), 3.58 (2H, t), 4.90 (2H, s), 6.73 (1H, d), 7.35 (4H, m), 7.56 (2H, m), 7.70 (1H, dd), 7.79 (1H, d), 8.38 (1H, d) | 1637, 1593, 1529 |
| 246 | HO-CH2CH=CH-phenyl | 3-(2-chloro-5-pyridinylmethyl)-2-iminothiazolidine | 3,4 | 3.17 (2H, t), 3.57 (2H, t), 4.81 (2H, s), 4.84 (2H, d), 6.39 (1H, m), 6.78 (!H, d), 7.20-7.41 (6H, m), 7.66 (1H, dd), 8.31 (1H, d) | 1669, 1654, 1546 |
| 247 | 1H-benzo[ d][ 1,3]oxazin-4 (2H)-one | 3-(2-chloro-5-pyridinylmethyl)-2-iminothiazolidine | another methods | 2.26 (3H, s), 3.22 (2H, t), 3.65 (2H, t), 4.96 (2H, s), 7.05 (1H, m), 7.32 (1H, d), 7.49 (1H, td), 7.69 (1H, dd), 8.38 (1H, d), 8.43 (1H, dd), 8.70 (1H, d) | 1682, 1592, 1517, 1460, 1395 |
| 248 | 8-methyl-1H-benzo[ d][ 1,3] oxazin-4(2H)-one | 3-(2-chloro-5-pyridinylmethyl)-2-iminothiazolidine | another methods | 2.22 (3H, s), 2.34 (3H, s), 3.19 (2H, t), 3.62 (2H, t), 4.91 (2H, s), 7.13 (1H, m), 7.34 (2H, m), 7.68 (1H, dd), 8.08 (1H, d), 8.34 (1H, d) | 1532, 1460, 1407 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 249 | HOOC-3-tetrahydrofuranyl | 3-(2-chloro-5-pyridinylmethyl)-2-iminothiazolidine | 1,2 | 2.14 (1H, m), 2.28 (1H, m), 3.15 (2H, t), 3.25 (1H, m), 3.58 (2H, t), 3.80-4.05 (4H, m), 4.82 (2H, d×2), 7.33 (1H, d), 7.64 (1H, dd), 8.34 (1H,d) | 1629, 1529, 1411 |
| 251 | HO-CH2CH2CH2NO2 | 3-(2-chloro-5-pyridinylmethyl)-2-iminothiazolidine | 3,4 | 2.42 (2H, m), 3.18 (2H, t), 3.59 (2H, t), 4.28 (2H, t), 4.54 (2H, t), 4.80 (2H, s), 7.35 (1H, d), 7.65 (1H, dd), 8.31 (1H, d) | |
| 252 | (4-idophenyl)-COCl | 3-(2-chloro-5-pyridinylmethyl)-2-iminothiazolidine | 5 | 3.18 (2H, t), 3.61 (2H, t), 4.94 (2H, s), 7.31 (1H, d), 7.66 (1H, dd), 7.75 (2H, d), 7.96 (2H, d), 8.37 (1H, d) | 1530, 1405, 1278 |
| 253 | (4-chlorophenyl)-O-C(Me)2-COOH | 3-(2-chloro-5-pyridinylmethyl)-2-iminothiazolidine | 1,2 | 1.57 (6H, s), 3.14 (2H, t), 3.55 (2H, t), 4.68 (2H, s), 6.70 (2H, d), 7.08 (1H, d), 7.17 (3H, m), 8.21 (1H, s) | 1635, 1530, 1489, 1463, 1402 |
| 254 | HO-CH2CH2SiMe3 | 3-(2-chloro-5-pyridinylmethyl)-2-iminothiazolidine | 3,4 | 0.05 (9H, s), 1.09 (2H, t), 3.13 (2H, t), 3.54 (2H, t), 4.24 (2H, t), 4.79 (2H, s), 7.30 (1H, d), 7.64 (1H, dd), 8.30 (1H, d) | 2951, 1665, 1541 |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 255 | HO-CH2CH2morpholinyl | 3-(2-chloro-5-pyridinylmethyl)-2-iminothiazolidine | 3,4 | 2.65 (4H, m), 2.78 (2H, t), 3.15 (2H, t), 3.56 (2H, t), 3.74 (4H, m), 4.29 (2H, t), 4.78 (2H, s), 7.29 (1H, d), 7.62 (1H, dd), 8.31 (1H, d) | 1705, 1550, 1459 |
| 256 | (2-methoxyphenyl)-COOH | 3-(2-chloro-5-pyridinylmethyl)-2-iminothiazolidine | 1,2 | 3.16 (2H, t), 3.58 (2H, t), 3.91 (3H, s), 4.91 (2H, s), 6.97 (2H, m), 7.31 (1H, d), 7.42 (1H, td), 7.72 (1H, dd), 8.00 (1H, dd), 8.38 (1H, d) | m/z=362 (M+H) |
| 257 | (4-methoxyphenyl)-COCl | 3-(2-chloro-5-pyridinylmethyl)-2-iminothiazolidine | 5 | 3.17 (2H, t), 3.60 (2H, t), 3.86 (3H, s), 4.96 (2H, s), 6.91 (2H, d), 7.31 (1H, d), 7.71 (1H, dd), 8.22 (2H, d), 8.40 (1H, d) | m/z=362 (M+H) |
| 258 | HOOC-3-thienyl | 3-(2-chloro-5-pyridinylmethyl)-2-iminothiazolidine | 1,2 | 3.17 (2H, d), 3.60 (2H, t), 4.92 (2H, s), 7.27 (1H, m), 7.33 (1H, d), 7.64 (1H, d), 7.70 (1H, dd), 8.21 (1H, d), 8.38 (1H, d) | m/z=338 (M+H) |

[Table 14]

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 259 | (2-chlorophenyl)-COCl | 3-(2-chloro-5-pyridinylmethyl)-2-iminothiazolidine | 5 | 3.21 (2H, t), 3.62 (2H, t), 4.91 (2H, s), 7.26 - 7.36 (3H, m), 7.42 (1H, d), 7.68 (1H, dd), 7.93 (1H, dd), 8.36 (1H, d) | m/z=366 (M+H) |
| 260 | 2-thienyl-COCl | 3-(2-chloro-5-pyridinylmethyl)-2-iminothiazolidine | 5 | 3.18 (2H, t), 3.63 (2H, t), 4.90 (2H, s), 7.10 (1H, m), 7.31 (1H, d), 7.49 (1H, m), 7.76 (1H, dd), 7.88 (1H, dd), 8.41 (1H, d) | m/z=366 (M+H) |
| 261 | HOOC-(4-cyclohexyl)Ph | 3-(2-chloro-5-pyridinylmethyl)-2-iminothiazolidine | 1,2 | 1.20-1.32 (1H, m), 1.36-1.48 (4H, m), 1.75 (1H, m), 1.83 - 1.89 (4H, m), 2.56 (1H, m), 3.17 (2H, t), 3.60 (2H, t), 4.97 (2H, s), 7.25 (2H, d), 7.31 (1H, d), 7.72 (1H, dd), 8.18 (2H, d), 8.39 (1H, d) | m/z=338 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 262 | HOOC-2-benzofuranyl | 3-(2-chloro-5-pyridinylmethyl)-2-iminothiazolidine | 1,2 | 3.22 (2H, t), 3.65 (2H, t), 4.97 (2H, s), 7.27 (2H, d), 7.34 (1H, d), 7.41 (1H, td), 7.60 (1H, m), 7.66 (1H, d), 7.77 (1H, dd), 8.34 (1H, d) | m/z=414 (M+H) |
| 263 | HOOC-C≡C-Ph | 3-(2-chloro-5-pyridinylmethyl)-2-iminothiazolidine | 1,2 | 3.18 (2H, t), 3.61 (2H, t), 4.90 (2H, s), 7.33-7.42 (4H, m), 7.60 (2H, m), 7.72 (1H, dd), 8.36 (1H, d) | m/z=356 (M+H) |
| 264 | HOOC-(3-methoxyphenyl) | 3-(2-chloro-5-pyridinylmethyl)-2-iminothiazolidine | 1,2 | 3.19 (2H, t), 3.62 (2H, t), 3.85 (3H, s), 4.97 (2H, s), 7.08 (1H, dd), 7.26-7.35 (2H, m), 7.72 (1H, dd), 7.83 (1H, m), 7.89 (1H, d), 8.39 (1H, d) | m/z=362 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 265 | HOOC-(4-ethylphenyl) | 3-(2-chloro-5-pyridinylmethyl)-2-iminothiazolidine | 1,2 | 1.26 (2H, t), 2.70 (2H, t), 3.18 (2H, t), 3.60 (2H, t), 4.97 (2H, s), 7.25 (2H, d), 7.32 (1H, d), 7.18 (1H, dd), 8.18 (2H, d), 8.40 (1H, d) | m/z=360 (M+H) |
| 266 | HOOC-(4-cyanophenyl) | 3-(2-chloro-5-pyridinylmethyl)-2-iminothiazolidine | 1,2 | 3.23 (2H, t), 3.66 (2H, t), 4.98 (2H, s), 7.34 (1H, d), 7.66 (1H, dd), 7.72 (2H, d), 8.33 (2H, d), 8.40 (1H, d) | m/z=357 (M+H) |
| 267 | HOOC-(3-methylphenyl) | 3-(2-chloro-5-pyridinylmethyl)-2-iminothiazolidine | 1,2 | 2.40 (3H, s), 3.18 (2H, t), 3.61 (2H, t), 4.98 (2H, s), 7.32 (3H, m), 7.72 (1H, dd), 8.07 (2H, m), 8.40 (1H, d) | m/z=346 (M+H) |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 268 | HOOC-(2-fluorophenyl) | 3-(2-chloro-5-pyridinylmethyl)-2-iminothiazolidine | 1,2 | 3.19 (2H, t), 3.62 (2H, t), 4.92 (2H, s), 7.11 (1H, dd), 7.17 (1H, m), 7.31 (1H, d), 7.45 (1H, m), 7.73 (1H, dd), 8.10 (1H, td), 8.39 (1H, d) | m/z=350 (M+H) |
| 269 | HOOC-(3-cyanophenyl) | 3-(2-chloro-5-pyridinylmethyl)-2-iminothiazolidine | 1,2 | 7.23 (2H, t), 7.66 (2H, t), 5.00 (2H, s), 7.35 (1H, d), 7.56 (1H, t), 7.67 (1H, dd), 7.77 (1H, d), 8.39 (1H, d), 8.45 (1H, d), 8.56 (1H, s) | m/z=357 (M+H) |
| 270 | (2-methylphenyl)-COCl | 3-(2-chloro-5-pyridinylmethyl)-2-iminothiazolidine | 5 | 2.67 (3H, s), 3.18 (2H, t), 3.59 (2H, t), 4.92 (2H, s), 7.22 (2H, m), 7.33 (2H, m), 7.68 (1H, dd), 8.12 (1H, d), 8.37 (1H, d) | m/z=346 (M+H) |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 271 | HOOC-(2,3-dihydro-1H-indenyl) | 3-(2-chloro-5-pyridinylmethyl)-2-iminothiazolidine | 1,2 | 3.12 (2H, t), 3.23 (2H, dd), 3.32 (2H, dd), 3.48 (1H, m), 3.57 (2H, t), 4.77 (2H, s), 7.12 (2H, m), 7.19 (2H, m), 7.27 (1H, d), 7.53 (1H, dd), 8.31 (1H, d) | m/z=372 (M+H) |
| 272 | HOOC-(4-SO2Me-phenyl) | 3-(2-chloro-5-pyridinylmethyl)-2-iminothiazolidine | 1,2 | 3.23 (2H, t), 3.67 (2H, t), 4.99 (2H, s), 7.34 (1H, dd), 7.67 (1H, dd), 8.00 (2H, dd), 8.41 (2H, dd), 8.43 (1H, d) | m/z=410 (M+H) |
| 273 | HOOC-(3-chlorophenyl) | 3-(2-chloro-5-pyridinylmethyl)-2-iminothiazolidine | 1,2 | 3.20 (2H, t), 3.63 (2H, t), 4.98 (2H, s), 7.35 (2H, dd), 7.48 (1H, dd), 7.70 (1H, dd), 8.11 (1H, dd), 8.23 (1H, t), 8.40 (1H, d) | m/z=366 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 274 | HOOCCH2CH(Me)2 | 3-(2-chloro-5-pyridinylmethyl)-2-iminothiazolidine | 1,2 | 0.95 (6H, d) 2.20 (1H, m), 2.36 (2H, d), 3.11 (2H, t), 3.53 (2H, t), 4.80 (2H, s), 7.31 (1H, d), 7.63 (1H, dd), 8.34 (1H, d) | m/z=312 (M+H) |
| 275 | HOOC-(3-nitrophenyl) | 3-(2-chloro-5-pyridinylmethyl)-2-iminothiazolidine | 1,2 | 3.24 (2H, t), 3.69 (2H, t), 5.01 (2H, s), 7.35 (1H, d), 7.62 (1H, m), 7.71 (1H, dd), 8.36 (1H, dd), 8.41 (1H, d), 8.55 (1H, d), 9.08 (1H, d) | m/z=377 (M+H) |
| 276 | (4-nitrophenyl)-OCOCl | 3-(2-chloro-5-pyridinylmethyl)-2-iminothiazolidine | 5 | 3.24 (2H, t), 3.67 (2H, t), 5.00 (2H, s), 7.34 (1H, d), 7.68 (1H, dd), 8.26 (2H, dd), 8.39 (3H, m) | m/z=377 (M+H) |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 277 | HOOC-CH2OCH(Me)2 | 3-(2-chloro-5-pyridinylmethyl)-2-iminothiazolidine | 1,2 | 3.15 (2H, t), 3.57 (2H, t), 3.74 (1H, sept), 4.22 (2H, s), 4.81 (2H, s), 7.33 (1H, d), 7.65 (1H, dd), 8.34 (1H, d) | m/z=328 (M+H) |
| 278 | HOOC-{ 3-(1,2,3,4-tetrahydronaphthalenyl)] | 3-(2-chloro-5-pyridinylmethyl)-2-iminothiazolidine | 1,2 | 1.91 (1H, m), 2.24 (1H, m), 2.88 (3H, m), 3.06 (2H, m), 3.13 (2H, t), 3.55 (2H, t), 4.81 (2H, d), 7.09 (4H, m), 7.30 (1H, d), 7.59 (1H, dd), 8.33 (1H, d) | m/z=386 (M+H) |
| 279 | HOOC-CH2OtBu | 3-(2-chloro-5-pyridinylmethyl)-2-iminothiazolidine | 1,2 | 1.25 (9H, s), 3.13 (2H, t), 3.55 (2H, t), 4.17 (2H, s), 4.79 (2H, s), 7.32 (1H, d), 7.67 (1H, dd), 8.34 (1H, d) | m/z=342 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 280 | HOOC-(3-fluorophenyl) | 3-(2-chloro-5-pyridinylmethyl)-2-iminothiazolidine | 1,2 | 3.20 (2H, t), 3.63 (2H, t), 4.97 (2H, s), 7.20 (1H, td), 7.34 (1H, d), 7.40 (1H, m), 7.70 (1H, dd), 7.93 (1H, dd), 8.04 (1H, dd), 8.40 (1H, d) | m/z=350 (M+H) |

[Table 15]

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 281 | HOOC-C(cyclopropyl)-Ph | 3-(2-chloro-5-pyridinylmethyl)-2-iminothiazolidine | 1,2 | 1.21 (2H, t), 1.68 (2H, t), 3.08 (2H, t), 3.52 (2H, t), 4.47 (2H, s), 7.17 (3H, m), 7.12-7.28 (4H, m), 7.44 (2H, m), 8.11 (1H, d) | m/z=372 (M+H) |
| 282 | HOOC-C(Me)2-Ph | 3-(2-chloro-5-pyridinylmethyl)-2-iminothiazolidine | 1,2 | 1.62 (6H, s), 3.06 (2H, t), 3.50 (2H, t), 4.57 (2H, s), 7.00 (2H, m), 7.19-7.37 (4H, m), 8.12 (1H, d) | m/z=374 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 283 | HOOC-(CH2CH2)-Ph | 3-(2-chloro-5-pyridinylmethyl)-2-iminothiazolidine | 1,2 | 2.82 (2H, t), 3.02 (2H, t), 3.11 (2H, t), 3.52 (2H, t), 4.78 (2H, s), 7.16-7.32 (5H, m), 7.57 (1H, dd), 8.32 (1H, d) | m/z=360 (M+H) |
| 284 | HOOC-3-pyrimidinyl | 3-(2-chloro-5-pyridinylmethyl)-2-iminothiazolidine | 1,2 | 3.25 (2H, t), 3.69 (2H, t), 4.98 (2H, s), 7.34 (1H, d), 7.67 (1H, dd), 8.38 (1H, d), 9.31 (1H, d), 9.46 (1H, d) | m/z=334 (M+H) |
| 285 | HOOC-CH2NHCOOtBu | 3-(2-chloro-5-pyridinylmethyl)-2-iminothiazolidine | 1,2 | 1.45 (9H, s), 3.16 (2H, t), 3.58 (2H, t), 4.06 (1H, d), 4.81 (2H, s), 7.32 (1H, d), 7.65 (1H, dd), 8.31 (1H, d) | m/z=385 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 286 | HOOC-2-oxirane | 3-(2-chloro-5-pyridinylmethyl)-2-iminothiazolidine | 1,2 | 2.93 (1H, dd), 3.00 (1H, dd), 3.17 (2H, t) 3.56 (1H, dd), 3.60 (2H, t), 4.81 (1H, d), 4.86 (1H, d), 7.34 (!H, d), 7.67 (1H, dd), 8.34 (1H, d) | m/z=298 (M+H) |
| 287 | (3-bromophenyl)-COCl | 3-(2-chloro-5-pyridinylmethyl)-2-iminothiazolidine | 5 | 3.20 (2H, t), 3.63 (2H, t), 4.97 (2H, s), 7.28-7.35 (2H, m), 7.63 (1H, dd), 7.69 (1H, dd), 8.18 (1H, d), 8.38 (2H, m) | m/z=410 (M+H) |
| 288 | HOOC-3-azetydine | 3-(2-chloro-5-pyridinylmethyl)-2-iminothiazolidine | 1,2 | 3.14 (2H, t), 3.49 (1H, m), 3.58 (2H, t), 3.74 (2H, m), 2.97 (2H, m), 4.81 (2H, s), 7.31 (1H, d), 7.63 (1H, dd), 8.33 (1H, d) | m/z=311 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 289 | (2-pyridyl)-COCl | 3-(2-chloro-5-pyridinylmethyl)-2-iminothiazolidine | 5 | 3.21 (2H, t), 33.64 (2H, t), 5.00 (2H, s), 7.32 (1H, d), 7.40 (1H, m), 7.79 (2H, m), 8.29 (1H, d), 8.42 (1H, s), 8.77 (1H, d) | m/z=333 (M+H) |
| 290 | HOOC-4-pyridyl | 3-(2-chloro-5-pyridinylmethyl)-2-iminothiazolidine | 1,2 | 3.23 (2H, t), 3.66 (2H, t), 4.98 (2H, s), 7.32 (1H, m), 7.67 (1H, dd), 8.30 (2H, m), 8.40 (1H, d), 8.74 (2H, m) | m/z=333 (M+H) |
| 291 | HOOC-(5-chloro-2-thienyl) | 3-(2-chloro-5-pyridinylmethyl)-2-iminothiazolidine | 1,2 | 3.21 (2H, t), 3.63 (2H, t), 4.87 (2H, s), 6.93 (1H, t), 7.35 (1H, dd), 7.64 (1H, m), 7.73 (1H, dd), 8.40 (1H, d) | |
| 292 | (2-fluorophenyl)-COOH | 3-(2-chloro-5-thiazolylmethyl)-2-iminothiazolidine | 1,2 | 3.21 (2H, t), 3.68 (2H, t), 4.99 (2H, s), 7.13 (1H, dd), 7.21 (1H, m), 7.48 (1H, m), 7.50 (1H, s), 8.17 (1H, td) | |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 293 | PhCOCl | 3-(2-chloro-5-pyridinylmethyl)-2-iminoimidazolidine | 5 | 3.41 (2H, t), 3.71 (2H, t), 4.71 (2H, s), 7.31 (1H, d), 7.40 (2H, m), 7.46 (1H, m), 7.73 (1H, dd), 8.24 (2H, m), 8.38 (1H, d), 8.89 (1H, br s) | m/z=315 (M+H) |
| 294 | (2-fluorophenyl)-COOH | 3-(2-chloro-5-pyridinylmethyl)-2-iminoimidazolidine | 1,2 | 3.41 (2H, t), 3.71 (2H, t), 4.65 (2H, s), 7.05-7.16 (2H, m), 7.32 (1H, d), 7.39 (1H, m), 7.73 (1H, dd), 8.01 (1H, td), 8.37 (1H, d), 8.81 (1H, be s) | |
| 295 | PhCOCl | 3-(2-trifluoromethyl-5-pyridinylmethyl)-2-iminothiazolidine | 5 | 3.20 (2H, t), 3.63 (2H, t), 5.06 (2H, s), 7.42 (2H, m), 7.51 (1H, m), 7.67 (1H, d), 7.90 (1H, d), 8.25 (2H, d), 8.74 (1H, s) | m/z=366 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 296 | (2-fluorophenyl)-COOH | 3-(2-trifluoromethyl-5-pyridinylmethyl)-2-iminothiazolidine | 1,2 | 3.21 (2H, t), 3.66 (2H, t), 5.03 (2H, s), 7.10 (1H, dd), 7.19 (1H, m), 7.45 (1H, m), 7.69 (1H, d), 7.94 (1H, d), 8.08 (1H, td), 8.74 (1H, s) | m/z=384 (M+H) |
| 297 | PhOCOCl | 3-(2-trifluoromethyl-5-pyridinylmethyl)-2-iminothiazolidine | 5 | 3.21 (2H, t), 3.65 (2H, t), 4.93 (2H, s), 7.18 (3H, m), 7.37 (2H, m), 7.69 (1H, d), 7.85 (1H, dd), 8.68 (1H, d) | m/z=382 (M+H) |
| 298 | (2-trifluoromethylphenyl)-COCl | 3-(2-chloro-5-pyridinylmethyl)-2-iminothiazolidine | 5 | 2.65 (3H, s). 4.58 (2H, s), 7.33 (1H, d), 7.43 (2H, m), 7.50 (1H, m), 7.65 (1H, dd), 8.23 (2H, m), 8.38 (1H, d) | m/z=400 (M;H) |
| 299 | PhCOCl | 3-(5,6-dichloro-3-pyridinylmethyl)-2-iminothiazolidine | 5 | 3.29 (2H, t), 3.63 (2H, t), 4.96 (2H, s), 7.43 (2H, m), 7.51 (1H, m), 7.82 (1H, d), 8.24 (2H, m), 8.31 (1H, d) | m/z=366 (M;H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 300 | PhCOCl | 3-(6fluoro-3-pyridinylmethyl)-2-iminothiazolidine | 5 | 3.17 (2H, t), 3.61 (2H, t), 4.98 (2H, s), 6.93 (1H, dd), 7.43 (2H, m), 7.51 (1H, m), 7.86 (1H, td), 8.22 (1H, d), 8.28 (2H, d) | m/z=316 (M;H) |
| 301 | PhCOCl | 3-(6-chloro-5-fluoro-3-pyridinylmethyl)-2-iminothiazolidine | 5 | 3.20 (2H, t), 3.63 (2H, t), 4.98 (2H, s), 7.41-7.58 (4H, m), 8.24 (2H, m) | m/z=350 (M;H) |
| 302 | PhCOCl | 3-(6-bromo-3-pyridinylmethyl)-2-iminothiazolidine | 5 | 3.15 (2H, t), 3.59 (2H, t), 4.93 (2H, s), 7.42-7.62 (5H, m), 8.24 (2H, m), 8.36 (1H, d) | m/z=376 (M;H) |
| 303 | HO-CH2CH2C≡CH | 3-(2-chloro-5-pyridinylmethyl)-2-iminothiazolidine | 3,4 | 2.00 (1H, d), 2.63 (2H, td), 3.18 (2H, t), 3.58 (2H, t), 4.28 (2H, t), 4.83 (2H, s), 7.32 (1H, d), 7.65 (1H, dd), 8.31 (1H, d) | m/z=324 (M;H) |

[Table 16]

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 304 | HOOC-CH2CH2-(2-methoxyphenyl) | 3-(2-chloro-5-pyridinylmethyl)-2-iminothiazolidine | 1,2 | 2.80(1H,m), 3.00(1H,m), 3.11(2H,m), 3.81(3H,s), 4.12(2H,s), 6.85(2H,m), 7.17(2H,m), 7.30(1H,t), 7.62(1H,m), 8.33(1H,s) | m/z=390 (M+H) |
| 305 | HOOC-CH2CH2-(2-methoxyphenyl) | 3-(2-chloro-5-pyridinylmethyl)-2-iminothiazolidine | 1,2 | 2.85(1H,t), 3.04(1H,m), 3.14(1H,t) 3.60(1H,t), 3.83(3H,s), 4.13(1H,q) 4.83(2H,s), 6.85(1H,d), 6.89(1H,d), 7.20(2H,m), 7.45(1H,s) | m/z=396 (M+H) |
| 306 | HO-CH2CH2CH2C≡CH | 3-(2-chloro-5-pyridinylmethyl)-2-iminothiazolidine | 3,4 | 1.96 (3H, m), 2.33 (2H, td), 3.16 (2H, t), 3.57 (2H, t), 4.27 (2H, t), 4.85 (2H, s), 7.32 (1H, d), 7.64 (1H, dd), 8.31 (1H, d) | m/z=338 (M;H) |
| 307 | HO-CH2CH2CH2CH2C≡CH | 3-(2-chloro-5-pyridinylmethyl)-2-iminothiazolidine | 3,4 | 1.64 (2H, m), 1.84 (2H, m), 1.94 (1H, d), 2.23 (2H, td), 3.16 (2H, t), 3.56 (2H, t), 4.19 (2H, t), 4.81 (2H, s), 7.31 (1H, d), 7.64 (1H, dd), 8.32 (1H, d) | m/z=352 (M;H) |

(continued)

| Compound No. | Chemical Precursor 1 | Chemical Precursor 2 | Synthesis Example | NMR ($CDCl_3$, δ, ppm) | IR (KBr, ν, $cm^{-1}$ ) or MS |
|---|---|---|---|---|---|
| 308 | HOOC-CH2CH2C≡CH | 3-(2-chloro-5-pyridinylmethyl)-2-iminothiazolidine | 1,2 | 1.96 (3H, m), 2.56 (2H, td), 2.73 (2H, t), 3.13 (2H, t), 3.56 (2H, t), 4.82 (2H, t), 7.32 (1H, d), 7.67 (1H, dd), 8.34 (1H, d) | m/z=308 (M;H) |
| 309 | HOOC-CH2CH2CH2C≡CH | 3-(2-chloro-5-pyridinylmethyl)-2-iminothiazolidine | 1,2 | 1.92 (3H, m), 2.27 (2H, t), 2.61 (2H, t), 3.12 (2H, t), 3.53 (2H, t), 4.80 (2H, s), 7.31 (1H, d), 7.65 (1H, dd), 8.31 (1H, d) | m/z=322 (M;H) |
| 310 | HO-CH2CH2CH2CH2CH=CH2 | 3-(2-chloro-5-pyridinylmethyl)-2-iminothiazolidine | 3,4 | 1.50 (2H, m), 1.75 (2H, m), 2.09 (2H, q), 3.16 (2H, t), 3.56 (2H, t), 4.17 (2H, t), 4.81 (2H, s), 4.96 (1H, m), 5.02(1H, m), 5.81 (1H, m), 7.29 (1H, d), 7.64 (1H, dd), 8.31 (1H, d) | m/z = 354 (M+H) |
| 311 | HO-CH2CH2CH2CH2CH=CH2 | 3-(2-chloro-5-thiazolylmethyl)-2-iminothiazolidine | 3,4 | 1.51 (2H, m), 1.76 (2H, m), 2.13 (2H, m), 3.17 (2H, t), 3.63 (2H, t), 4.21 (2H, t), 4.96 (1H, m), 5.04 (1H, m), 5.80 (1H, m), 7.45 (1H, s) | m/z = 360 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 312 | HOOC-CH2CH2CH2CH2C≡CH | 3-(2-chloro-5-pyridinylmethyl)-2-iminothiazolidine | 1,2 | 1.60 (2H, m), 1.77 (2H, m), 1.94 (1H, t), 2.22 (2H, td), 2.50 (2H, t), 3.13 (2H, t), 3.53 (2H, t), 4.80 (2H, s), 7.31 (1H, d), 7.65 (1H, dd), 8.33 (1H, d) | m/z = 336 (M+H) |
| 313 | HOOC-CH2CH2CH2CH2C≡CH | 3-(2-chloro-5-thiazolylmethyl)-2-iminothiazolidine | 1,2 | 1.60 (2H, m), 1.84 (2H, m), 1.95 (1H, t), 2.25 (2H, ts), 2.56 (2H, t), 3.14 (2H, t), 3.60 (2H, t), 4.83 (2H, s), 7.45 (1H, s) | m/z = 342 (M+H) |
| 314 | HO-CH2CH2CH2CH=CH2 | 3-(2-chloro-5-pyridinylmethyl)-2-iminothiazolidine | 3,4 | 1.83 (2H, m), 2.17 (2H, m), 3.16 (2H, t), 3.58 (2H, t), 4.18 (2H, t), 4.98 (1H, m), 5.04 (1H, m), 5.83 (1H, m), 7.33 (1H, d), 7.65 (1H, dd), 8.32 (1H, d) | m/z = 340 (M+H) |
| 315 | HO-CH2CH2CH2CH=CH2 | 3-(2-chloro-5-thiazolylmethyl)-2-iminothiazolidine | 3,4 | 1.84 (2H, m), 2.17 (2H, m), 3.17 (2H, t), 3.63 (2H, t), 4.20 (2H, t), 4.86 (2H, s), 4.99 (1H, m), 5.06 (1H, m), 5.85 (1H, m), 7.45 (1H, s) | m/z = 346 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 316 | HOOC-CH2CH2CH2CH2CH=CH2 | 3-(2-chloro-5-pyridinylmethyl)-2-iminothiazolidine | 1,2 | 1.44 (2H, m), 1.70 (2H, m), 2.07 (2H, m), 2.49 (2H, t), 3.11 (2H, t), 3.53 (2H, t), 4.80 (2H, s), 4.94 (1H, m), 5.00 (1H, m), 5.82 (1H, m), 7.40 (1H, d), 7.64 (1H, dd), 8.34 (1H, d) | m/z = 338 (M+H) |
| 317 | HOOC-CH2CH2CH2CH2CH=CH2 | 3-(2-chloro-5-thiazolylmethyl)-2-iminothiazolidine | 1,2 | 1.40 (2H, m), 1.74 (2H, m), 2.10 (2H, m), 2.54 (2H, t), 3.13 (2H, t), 3.59 (2H, t), 4.83 (2H, s), 4.95 (1H, m), 5.03 (1H, m), 5.83 (1H, m), 7.45 (1H, s) | m/z = 344 (M+H) |
| 318 | HOOC-CH2CH2CH2CH=CH2 | 3-(2-chloro-5-pyridinylmethyl)-2-iminothiazolidine | 1,2 | 1.78 (2H, m), 2.11 (2H, m), 2.50 (2H, t), 3.11 (2H, t), 3.53 (2H, t), 4.80 (2H, s), 4.96 (1H, m), 5.03 (1H, m), 5.82 (1H, m), 7.33 (1H, d), 7.64 (1H, dd), 8.34 (1H, d) | m/z = 324 (M+H) |
| 319 | HOOC-CH2CH2CH2CH=CH2 | 3-(2-chloro-5-thiazolylmethyl)-2-iminothiazolidine | 1,2 | 1.82 (2H, m), 2.14 (2H, m), 2.54 (2H, t), 3.13 (2H, t), 3.59 (2H, t), 4.84 (2H, s), 4.97 (1H, m), 5.05 (1H, m), 5.85 (1H, m), 7.45 (1H, s) | m/z = 330 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$ ) or MS |
|---|---|---|---|---|---|
| 320 | HOOC-CH2CH2CH=CH2 | 3-(2-chloro-5-pyridinylmethyl)-2-iminothiazolidine | 1,2 | 2.43 (2H, m), 2.59 (2H, m), 3.12 (2H, t), 3.54 (2H, t), 4.81 (2H, s), 4.97 (1H, m), 5.06 (1H, m), 5.89 (1H, m), 7.40 (1H, d), 7.65 (1H, dd), 8.34 (1H, d) | m/z = 310 (M+H) |
| 321 | HOOC-CH2CH2CH=CH2 | 3-(2-chloro-5-thiazolylmethyl)-2-iminothiazolidine | 1,2 | 2.47 (2H, m), 2.63 (2H, m), 3.14 (2H, t), 3.60 (2H, t), 4.84 (2H, s), 4.99 (1H, m), 5.07 (1H, m), 5.92 (1H, m), 7.45 (1H, s) | m/z = 316 (M+H) |
| 322 | HO-CHCH3CF3 | 3-(2-chloro-5-thiazolylmethyl)-2-iminothiazolidine | 3,4 | 1.48 (3H, d), 3.21 (2H, t), 3.68 (2H, t), 4.88 (2H, s), 5.32 (1H, m), 7.48 (1H, s) | m/z = 374 (M+H) |
| 323 | HOOC-CH=CH- (3-thienyl) | 3-(2-chloro-5-pyridinylmethyl)-2-iminothiazolidine | 1,2 | 3.14 (2H, t), 3.57 (2H, t), 4.89 (2H, s), 6.58 (1H, d), 7.32 (3H, m), 7.46 (1H, d), 7.70 (1H, dd), 7.77 (1H, d), 8.37 (1H, d) | m/z = 364 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$ ) or MS |
|---|---|---|---|---|---|
| 324 | HOOC-CH=CH-(4-(2-thienyl)-2-thienyl) | 3-(2-chloro-5-pyridinylmethyl)-2-iminothiazolidine | 1,2 | 3.15 (2H, t), 3.57 (2H, t), 4.89 (2H, s), 6.53 (1H, m), 7.10 (1H, dd), 7.13 (1H, dd), 7.22 (1H, d), 7.27 (1H, d), 7.35 (1H, d), 7.69 (1H, dd), 7.81 (1H, d), 8.37 (1H, d) | m/z = 446 (M+H) |
| 325 | HO-CH2CH=CH-(3-thienyl) | 3-(2-chloro-5-pyridinylmethyl)-2-iminothiazolidine | 3,4 | 3.17 (2H, t), 3.57 (2H, t), 4.82 (2H, dd), 4.84 (2H, s), 6.23 (1H, dt), 6.67 (1H, d), 7.22 (1H, dd), 7.26 (1H, m), 7.32 (1H, d), 7.64 (1H, dd), 8.31(1H, d) | |

[Table 17]

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 326 | HOOC-CH2-(2-tetrahydrofuranyl) | 3-(2-chloro-5-pyridinylmethyl)-2-iminothiazolidine | 1,2 | 1.57 (1H, m), 1.89 (2H, m), 2.08 (1H, m), 2.62 (1H, dd), 2.80 (1H, dd), 3.11 (2H, t), 3.53 (2H, t), 3.73 (1H, m), 3.88 (1H, m), 4.36 (1H, m), 4.81 (2H, s), 7.31 (1H, d), 7.67 (1H, dd), 8.33 (1H, d) | m/z = 340 (M+H) |
| 327 | HOOC-CH=CH-(2-furanyl) | 3-(2-chloro-5-pyridinylmethyl)-2-iminothiazolidine | 1,2 | 3.14 (2H, t), 3.57 (2H, t), 4.89 (2H, s), 6.46 (1H, dd), 6.58 (1h, d), 6.63 (1H, d), 7.32(1H, d), 7.47 (1H, d), 7.69 (1H, dd), 8.37 (1H, d) | m/z = 347 (M+H) |
| 328 | HOOC-CH2CH2-(2-thienyl) | 3-(2-chloro-5-pyridinylmethyl)-2-iminothiazolidine | 1,2 | 2.88 (2H, t), 3.12 (2H, t), 3.24 (2H, t), 3.54 (2H, t), 4.80 (2H, s), 6.82 (1H, d), 6.90 (1H, dd), 7.10 (1H, dd), 7.30 (1H, d), 7.60 (1H, dd), 8.32 (1h, d) | m/z = 366 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 329 | HO-CH2CH2-(2-tetrahydrofuranyl) | 3-(2-chloro-5-pyridinylmethyl)-2-iminothiazolidine | 3,4 | 1.50 (1H, m), 1.82 (7H, m), 3.15 (2H, t), 3.56 (2H, t), 3.72 (1H, m), 3.86 (1H, dd), 3.95 (1H, dd), 4.26 (2H, m), 4.81 (2H, d×2), 7.31 (1H, d), 7.64 (1H, d), 8.31 (1H, d) | m/z = 370 (M+H) |
| 330 | HO-CH2CH2CH2-(2-thienyl) | 3-(2-chloro-5-pyridinylmethyl)-2-iminothiazolidine | 3,4 | 2.10 (2H, m), 2.96 (2H, t), 3.16 (2H, t), 3.57 (2H, t), 4.23 (2H, t), 4.82 (2H, s), 6.81 (1H, d), 6.91 (1H, dd), 7.11 (1H, dd), 7.32 (1H, d), 7.65 (1H, dd), 8.32 (1H, d) | m/z = 396 (M+H) |
| 331 | HOOC-CH=CH-(2-thienyl) | 3-(2-chloro-5-pyridinylmethyl)-2-iminothiazolidine | 1,2 | 3.15 (2H, t), 3.57 (2H, t), 4.90 (2H, s), 6.54 (1H, d), 7.04 (1H, dd), 7.23 (1H, d), 7.34 (1H, dd), 7.70 (1H, dd), 7.88 (1H, d), 8.37 (1H, d) | m/z = 364 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 332 | HOOC-CH2CH2-(2-furanyl) | 3-(2-chloro-5-pyridinylmethyl)-2-iminothiazolidine | 1,2 | 2.84 (2H, t), 3.01 (2H, t), 3.12 (2H, t), 3.54 (2H, t), 4.80 (2H, s), 6.00 (1H, d), 6.20 (1H, dd), 7.29 (1H, d), 7.31 (1H, d), 7.60 (1H, dd), 8.31 (1H, d) | m/z = 350 (M+H) |
| 333 | HOOC-CH2CH2CF3 | 3-(2-chloro-5-pyridinylmethyl)-2-iminothiazolidine | 1,2 | 2.52 (2H, m), 2.75 (2H, m), 3.14 (2H, t), 3.57 (2H, t), 4.81 (2H, s), 7.33 (1H, d), 7.62 (1H, dd), 8.33 (1H, d) | m/z = 352 (M+H) |
| 334 | HOOC-CH2CH2CH2OCH3 | 3-(2-chloro-5-pyridinylmethyl)-2-iminothiazolidine | 1,2 | 1.95 (2H, m), 2.56 (2H, t), 3.12 (2H, t), 3.3 (3H, s), 3.43 (2H, t), 3.52 (2H, t), 4.80 (2H, s), 7.31 (1H, d), 7.64 (1H, dd), 8.33 (1H, d) | m/z = 328 (M+H) |

(continued)

| Compound No. | Chemical Precursor 1 | Chemical Precursor 2 | Synthesis Example | NMR ($CDCl_3$, δ, ppm) | IR (KBr, v, $cm^{-1}$) or MS |
|---|---|---|---|---|---|
| 335 | HOOC-CH2-(3-tetrahydrofuranyl) | 3-(2-chloro-5-pyridinylmethyl)-2-iminothiazolidine | 1,2 | 1.58 (1H, m), 2.13 (1H, m), 2.60 (2H, m), 2.71 (1H, m), 3.13 (2H, t), 3.45 (1H, m), 3.52 (2H, t), 3.77 (1H, q), 3.85 (1H, m), 3.99 (1H, m), 4.80 (2H, s), 7.32 (1H, d), 7.61 (1H, dd), 8.33 (1H, d) | m/z = 340 (M+H) |
| 336 | HOOC-CH2CH2-(2-tetrahydrofuranyl) | 3-(2-chloro-5-pyridinylmethyl)-2-iminothiazolidine | 1,2 | 1.49 (1H, m), 1.80-2.05 (5H, m), 2.61 (2H, m), 3.11 (2H, t), 3.53 (2H, t), 3.71 (1H, m), 3.84 (2H, m), 4.84 (2H, s), 7.33 (1H, d), 7.65 (1H, dd), 8.33 (1H, d) | m/z = 354 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 337 | HO-CH2CH2CH2-(2-furanyl) | 3-(2-chloro-5-pyridinylmethyl)-2-iminothiazolidine | 3,4 | 2.06 (2H, m), 2.76 (2H, t), 3.16 (2H, t), 3.56 (2H, t), 4.21 (2H, t), 4.81 (2H, s), 6.01 (1H, dd), 6.27 (1H, dd), 7.30 (1H, d), 7.33 (1H, d), 7.64 (1H, dd), 8.31 (1H, d) | m/z = 380 (M+H) |
| 338 | HO-CH2CH2-(3-tetrahydrofuranyl) | 3-(2-chloro-5-pyridinylmethyl)-2-iminothiazolidine | 3,4 | 1.56 (1H, m), 1.83 (2H, m), 2.08 (1H, m), 2.32 (1H, m), 3.17 (2H, t), 3.40 (1H, dd), 3.58 (2H, t), 3.76 (1H, m), 3.86 (1H, m), 3.94 (1H, m), 4.20 (2H, m), 4.81 (2H, s), 7.31 (1H, d), 7.64 (1H, dd), 8.31 (1H, d) | m/z = 370 (M+H) |
| 339 | HOOC-CH=CH-(3-furanyl) | 3-(2-chloro-5-pyridinylmethyl)-2-iminothiazolidine | 1,2 | 3.14 (2H, t), 3.56 (2H, t), 4.89 (2H, s), 6.46 (1H, d), 6.64 (1H, s), 7.32 (1H, d), 7.42 (1H, s), 7.63 (1H, s), 7.69 (2H, m), 8.32 (1H, d) | m/z = 348 (M+H) |

(continued)

| Compound No. | Chemical Precursor 1 | Chemical Precursor 2 | Synthesis Example | NMR ($CDCl_3$, $\delta$, ppm) | IR (KBr, v, $cm^{-1}$) or MS |
|---|---|---|---|---|---|
| 340 | HO--CH2CH2CH2-(2-tetrahydrofuranyl) | 3-(2-chloro-5-pyridinylmethyl)-2-iminothiazolidine | 3,4 | 1.40-2.01 (8H, m), 3.15 (2H, t), 3.56 (2H, t), 3.71 (1H, td), 3.84 (2H, t), 4.20 (2H, m), 4.81 (2H, s), 7.33 (1H, d), 7.64 (1H, dd), 8.30 (1H, d) | m/z = 384 (M+H) |
| 341 | HOOC-CH2CH2-(3-furanyl) | 3-(2-chloro-5-pyridinylmethyl)-2-iminothiazolidine | 1,2 | 2.75 (2H, m), 2.82 (2H, m), 3.11 (2H, t), 3.55 (2H, t), 4.80 (2H, s), 6.29 (1H, d), 7.24 (1H, d), 7.32 (1H, d), 7.34 (1H, d), 7.60 (1H, dd), 8.32 (1H, d) | m/z = 350 (M+H) |
| 342 | HO-CH2CH2CH2CF3 | 3-(2-chloro-5-pyridinylmethyl)-2-iminothiazolidine | 3,4 | 2.00 (2H, m), 2.24 (2H, m), 3.17 (2H, td), 3.58 (2h, t), 4.23 (2H, t), 4.82 (2H, s), 7.34 (1H, d), 7.65 (1H, dd), 8.32 (1H, d) | m/z = 370 (M+H) |

(continued)

| Compound No. | Chemical Precursor 1 | Chemical Precursor 2 | Synthesis Example | NMR ($CDCl_3$, δ, ppm) | IR (KBr, ν, $cm^{-1}$) or MS |
|---|---|---|---|---|---|
| 343 | HOOC-CH2CH2-(4-methoxyphenyl) | 3-(2-chloro-5-pyridinylmethyl)-2-iminothiazolidine | 1,2 | 2.78 (2H, t), 2.96 (2H, t), 3.11 (2H, t), 3.52 (2H, t), 3.78 (3H, s), 4.78 (2H, s), 6.81 (2H, d), 7.14 (2H, d), 7.29 (1H, d), 7.56 (1H, dd), 8.31 (1H, d) | |
| 344 | HO-CH2CH2CH2OCH3 | 3-(2-chloro-5-pyridinylmethyl)-2-iminothiazolidine | 3,4 | 1.99 (2H, m), 3.15 (2H, t), 3.34 (3H, s), 3.49 (2H, t), 3.56 (2H, t), 4.26 (2H, t), 4.82 (2H, s), 7.33 (1H, d), 7.64 (1H, dd), 8.32 (1H, d) | m/z = 344 (M+H) |
| 345 | HOOC-CH2CH2-(3,5-dimethoxyphenyl) | 3-(2-chloro-5-pyridinylmethyl)-2-iminothiazolidine | 1,2 | 2.81 (2H, t), 2.98 (2H, t), 3.14 (2H, t), 3.51 (2H, t), 3.75 (6H, s), 4.79 (2H, s), 6.29 (1H, d), 6.39 (2H, d), 7.30 (1H, d), 7.60 (1H, dd), 8.32 (1H, d) | |

[Table 18]

| Compound No. | Chemical Precursor 1 | Chemical Precursor 2 | Synthesis Example | NMR ($CDCl_3$, $\delta$, ppm) | IR (KBr,v,$cm^{-1}$) or MS |
|---|---|---|---|---|---|
| 346 | HO-CH2CH2CH2CH2OCH3 | 3-(2-chloro-5-pyridinylmethyl)-2-iminothiazolidine | 3,4 | 1.68 (2H, m), 1.78 (2H, m), 3.16 (2H, t), 3.33 (3H, s), 3.41 (2H, t), 3.56 (2H, t), 4.20 (2H, t), 4.80 (2H, s), 7.34 (1H, d), 7.65 (1H, dd), 8.34 (1H, d) | m/z = 358 (M+H) |
| 347 | HO-CH2-(3-methoxyphenyl) | 3-(2-chloro-5-pyridinylmethyl)-2-iminothiazolidine | 3,4 | 3.16 (2H, t), 3.56 (2H, t), 3.81 (3H, s), 4.80 (2H, s), 5.19 (2H, s), 6.84 (1H, m), 7.00 (2H, m), 7.24 (1H, m), 7.32 (1H, d), 7.64 (1H, dd), 8.31 (1H, d) | m/z = 392 (M+H) |
| 348 | HO-CH2CH2-(3-methoxyphenyl) | 3-(2-chloro-5-pyridinylmethyl)-2-iminothiazolidine | 3,4 | 3.02 (2H, t), 3.17 (2H, t), 3.57 (2H, t), 3.80 (3H, s), 4.37 (2H, t), 4.80 (2H, t), 6.80 (3H, m), 7.22 (1H, m), 7.32 (1H, d), 7.65 (1H, dd), 8.32 (1H, d) | m/z = 406 (M+H) |

(continued)

| Compound No. | Chemical Precursor 1 | Chemical Precursor 2 | Synthesis Example | NMR ($CDCl_3$, $\delta$, ppm) | IR (KBr,v,$cm^{-1}$) or MS |
|---|---|---|---|---|---|
| 349 | HO-CH2CH2CH2-(3-furanyl) | 3-(2-chloro-5-pyridinylmethyl)-2-iminothiazolidine | 3,4 | 1.98 (2H, m), 2.54(2H, t), 3.16 (2H, t), 3.56 (2H, t), 4.21 (2H, t), 4.81 (2H, s), 6.28 (1H, s), 7.23 (1H, d), 7.33 (1H, d), 7.34 (1H, d), 7.64 (1H, dd), 8.31 (1H, d) | m/z = 380 (M+H) |
| 350 | HOOC-CH2CH2-(3-tetrahydrofuranyl) | 3-(2-chloro-5-pyridinylmethyl)-2-iminothiazolidine | 1,2 | 1.35 (1H, m), 1.78 (3H, m), 2.04 (1H, m), 2.23 (1H, m), 2.50 (2H, m), 3.12 (2H, t), 3.36 (1H, t), 3.54 (2H, t), 3.75 (1H, q), 3.85 (2H, m), 4.80 (2H, s), 7.31 (1H, d), 8.62 (1H, dd), 8.34 (1H, d) | m/z = 354 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 351 | HO-CH2CH2CH2-(3-tetrahydrofuranyl) | 3-(2-chloro-5-pyridinylmethyl)-2-iminothiazolidine | 3,4 | 1.48-1.74 (5H, m), 2.03 (1H, m), 2.20 (1H, m), 3.16 (2H, t), 3.34 (1H, m), 3.57 (2H, t), 3.75-3.92 (3H, m), 4.17 (2H ,t), 4.81 (2H, s), 7.33 (1H, d), 7.64 (1H, dd), 8.32 (1H, d) | m/z = 384 (M+H) |
| 352 | HO-CH2CH2-(2-thienyl) | 3-(2-chloro-5-pyridinylmethyl)-2-iminothiazolidine | 3,4 | 3.17 (2H, t), 3.25 (2H, t), 3.57 (2H, t), 4.39 (2H, t), 4.82 (2H, s), 6.89 (1H, dd), 6.94 (1H, dd), 7.16 (1H, dd), 7.33 (1H, d), 7.67 (1H, dd), 8.32 (1H, d) | m/z = 382 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 353 | HOOC-CH2CH2CH2-(2,3-dihydrobenzo[b][1,4]dioxin-6-yl) | 3-(2-chloro-5-pyridinylmethyl)-2-iminothiazolidine | 1,2 | 1.96 (2H, m), 2.49 (2H, t), 2.58 (2H, t), 3.12 (2H, t), 3.52 (2H, t), 4.21 (4H, s), 4.76 (2H, s), 6.66 (1H, dd), 6.70 (1H, d), 6.77 (1H, d), 7.31 (1H, d), 7.64 (1H, dd), 8.33 (1h, d) | |
| 354 | HO-CH2CH2-(3-thienyl) | 3-(2-chloro-5-pyridinylmethyl)-2-iminothiazolidine | 3,4 | 3.17 (2H, t), 3.17 (2H, t), 3.57 (2H, t), 4.37 (2H, t), 4.80 (2H, s), 7.00 (1H, dd), 7.06 (1H, dd), 7.26 (1H, dd), 7.32 (1H, d), 7.65 (1H, dd), 8.32 (1H, d) | m/z = 382 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 355 | HOOC-CH2CH2-(benzo[ d][ 1,3] dioxol-5-yl) | 3-(2-chloro-5-pyridinylmethyl)-2-iminothiazolidine | 1,2 | 2.77 (2H, t), 2.94 (2H, t), 3.12 (2H, t), 3.53 (2H, t), 4.79 (2H, s), 5.91 (2H, s), 6.68 (1H, dd), 6.71 (1H, d), 6.73 (1H, d), 7.30 (1H, d), 7.58 (1H, dd), 8.32 (1H, d) | |
| 356 | HO-CH2CH2CF3 | 3-(2-chloro-5-pyridinylmethyl)-2-iminothiazolidine | 3,4 | 2.57 (2H, m), 3.18 (2H, t), 3.60 (2H, t), 4.38 (2H, t), 4.81 (2H, t), 7.34 (1H, d), 7.66 (1H, dd), 8.33 (1H, d) | m/z = 368 (M+H) |
| 357 | HOOC-CH2CH2-(2,3-dihydrobenzo[b][1,4]dioxin-6-yl) | 3-(2-chloro-5-pyridinylmethyl)-2-iminothiazolidine | 1,2 | 2.77 (2H, t), 2.92 (2H, t), 3.12 (2H, t), 3.53 (2H, t), 4.22 (4H, s), 4.79 (2H, s), 6.69 (1H, dd), 6.73 (1H, d), 6.76 (1H, d), 7.32 (1H, d), 7.62 (1H, dd), 8.32 (1H, d) | m/z = 418 (M+H) |

(continued)

| Compound No. | Chemical Precursor 1 | Chemical Precursor 2 | Synthesis Example | NMR ($CDCl_3$, $\delta$, ppm) | IR (KBr,v,$cm^{-1}$) or MS |
|---|---|---|---|---|---|
| 358 | HOOC-CH2CH2-(3-methoxyphenyl) | 3-(2-chloro-5-pyridinylmethyl)-2-iminothiazolidine | 1,2 | 2.84 (2H, t), 3.00 (2H, t), 3.53 (2H, t), 3.78 (3H, s), 4.78 (2H, s), 6.73 (1H, d), 6.78 (1H, d), 6.83 (1H, d), 7.18 (1H, t), 7.30 (1H, d), 7.58 (1H, dd), 8.32 (1H, d) | m/z = 390 (M+H) |
| 359 | HO-CH2CH2-(2-furanyl) | 3-(2-chloro-5-pyridinylmethyl)-2-iminothiazolidine | 3,4 | 3.07 (2H, t), 3.16 (2H, t), 3.57 (2H, t), 4.11 (2H, t), 4.80 (2H, s), 6.10 (1H, d), 6.29 (1H, d), 7.30 (1H, d), 7.33 (1H, d), 7.63 (1H, dd), 8.30 (1H, d) | m/z = 366 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 360 | HO-CH2CH2-(3-furanyl) | 3-(2-chloro-5-pyridinylmethyl)-2-iminothiazolidine | 3,4 | 2.85 (2H, t), 3.16 (2H, t), 3.57 (2H, t), 4.31 (2H, t), 4.81 (2H, s), 6.33 (1H, s), 7.31 (1H, d), 7.33 (1H, d), 7.36 (1H, d), 7.64 (1H, dd), 8.31 (1H, d) | m/z = 366 (M+H) |
| 366 | HO-CH2-(2-thienyl) | 3-(2-chloro-5-pyridinylmethyl)-2-iminothiazolidine | 3,4 | 3.14 (2H, t), 3.53 (2H, t), 4.77 (2H, s), 5.34 (2H, s), 6.95 (1H, dd), 7.13 (1H, dd), 7.30 (1H, d), 7.62 (1H, dd), 8.28 (1H, d) | |
| 367 | HO-CH2-(3-thienyl) | 3-(2-chloro-5-pyridinylmethyl)-2-iminothiazolidine | 3,4 | 3.15 (2H, t), 3.55 (2H, t), 4.78 (2H, s), 5.20 (2H, s), 7.16 (1H, d), 7.27-7.35 (3H, m), 7.64 (1H, dd), 8.30 (1H, d) | 1650, 1546, 1460, 1440, 1415 |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 372 | HOOC-CH=CHCF3 | 3-(2-chloro-5-pyridinylmethyl)-2-iminothiazolidine | 1,2 | 3.19 (2H, t), 3.63 (2H, t), 4.88 (2H, s), 6.72 (1H, d), 6.80 (1H, m), 7.34 (1H, d), 7.64 (1H, dd), 8.34 (1H, d) | 1714, 1637, 1616, 1540 |
| 373 | HO-CH2CH2CN | 3-(2-chloro-5-pyridinylmethyl)-2-iminothiazolidine | 3,4 | 2.79 (2H, t), 3.20 (2H, t), 3.61 (2H, t), 4.37 (2H, t), 4.81 (2H, s), 7.33 (1H, d), 7.67 (1H, dd), 8.32 (1H, d) | 1671, 1549, 1457, 1431 |
| 374 | BrCOCH2Br, HOOC-CH2OCH2C≡CH | 3-(2-chloro-5-pyridinylmethyl)-2-iminothiazolidine | 7 | 2.44 (1H, t), 3.16 (2H, t), 3.58 (2H, t), 4.33 (2H, s), 4.36 (2H, d), 4.81 (2H, s), 7.32 (1H, d), 7.65 (1H, dd), 8.31 (1H, d) | |

[Table 19]

| Compound No. | Chemical Precursor 1 | Chemical Precursor 2 | Synthesis Example | NMR ($CDCl_3$, $\delta$, ppm) | IR (KBr,v,$cm^{-1}$) or MS |
|---|---|---|---|---|---|
| 429 | BrCOCH2Br, HO-CH2CH2CH2CF3 | 3-(2-chloro-5-pyridinylmethyl)-2-iminothiazolidine | 7 | 1.85 (2H, m), 2.25 (2H, m), 3.18 (2H, s), 3.54 (2H, t), 3.60 (2H, t), 4.18 (2H, s), 4.77 (2H, s), 7.29 (1H, d), 7.60 (1H, dd), 8.30 (1H, d) | 1653, 1534, 1461, 1414 |
| 430 | BrCOCH2Br, HO-CH2-(2-furanyl) | 3-(2-chloro-5-pyridinylmethyl)-2-iminothiazolidine | 7 | 3.15 (2H, t), 3.57 (2H, t), 4.21 (2H, s), 4.65 (2H, s), 4.80 (2H, s), 6.34 (2H, m), 7.31 (1H, d), 7.39 (1H, d), 7.64 (1H, dd), 8.32 (1H, d) | |
| 431 | BrCOCH2Br, HO-CH2-(3-furanyl) | 3-(2-chloro-5-pyridinylmethyl)-2-iminothiazolidine | 7 | 3.16 (2H, t), 3.66 (2H, t), 4.22 (2H, s), 4.56 (2H, s), 4.82 (2H, s), 6.45 (1H, d), 7.31 (1H, d), 7.39 (1H, d), 7.43 (1H, s), 7.65 (1H, dd), 8.35 (1H, d) | |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 432 | HOOC-(4-ethynylphenyl) | 3-(2-chloro-5-pyridinylmethyl)-2-iminothiazolidine | 1,2 | 2.25 (1H, s), 3.21 (2H, t), 3.74 (2H, t), 5.00 (2H, s), 7.46 (1H, d), 7.52 (2H, d), 7.84 (1H, dd), 8.12 (2H, d), 8.46 (1H, d) | 1617, 1527, 1462, 1419 |
| 427 | BrCH2COBr, HO-CH2CH2CF3 | 3-(2-chloro-5-pyridinylmethyl)-2-iminothiazolidine | 7 | 2.48 (2H, m), 3.16 (2H, t), 3.57 (2H, t), 3.82 (2H, t), 4.23 (2H, s), 4.79 (2H, s), 7.32 (1H, d), 7.64 (1H, dd), 8.33 (1H, d) | m/z = 382 (M+H) |
| 428 | HOOC-CH2OCH2CH2C≡CH | 3-(2-chloro-5-pyridinylmethyl)-2-iminothiazolidine | 1,2 | 1.97 (1H, t), 2.55 (2H, td), 3.15 (2H, t), 3/57 (2H, t), 3.73 (2H, t), 4.21 (2H, s), 4.80 (2H, s), 7.32 (1H ,d), 7.63 (1H, dd), 8.33 (1H, d) | m/z = 338 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 392 | HOOC-CH2CH2CH2CF3 | 3-(2-chloro-5-pyridinylmethyl)-2-iminothiazolidine | 1,2 | 1.93 (2H, m), 2.18 (2H, m), 2.57 (2H, t), 3.13 (2H, t), 3.56 (2H, t), 4.80 (2H, s), 7.31 (1H, d), 7.63 (1H, dd), 8.33 (1H, d) | m/z = 365 (M+H) |
| 433 | HOOC-CH2OCH2CH2CH=CH2 | 3-(2-chloro-5-pyridinylmethyl)-2-iminothiazolidine | 1,2 | 2.42 (2H, q), 3.14 (2H, t), 3.56 (2H, t), 3.64 (2H, t), 4.22 (2H, s), 4.80 (2H, s), 5.01 (1H, dd), 5.13 (1H, dd), 5.86 (1H, m), 7.31 (1H, d), 7.63 (1H, dd), 8.33 (1H, d) | m/z = 340 (M+H) |
| 434 | HOOC-CH2OCH2CH=CH2 | 3-(2-chloro-5-pyridinylmethyl)-2-iminothiazolidine | 1,2 | 3.13 (2H, t), 3.55 (2H, t), 4.14 (2H, d), 4.21 (2H, t), 4.79 (2H, d), 5.18 (1H, dd), 5.31 (1H, dd), 5.94 (1H, m), 7.32 (1H, d), 7.63 (1h, dd), 8.31 (1H, d) | m/z = 326 (M+H) |

(continued)

| Compound No. | Chemical Precursor 1 | Chemical Precursor 2 | Synthesis Example | NMR ($CDCl_3$, δ, ppm) | IR (KBr,v,$cm^{-1}$) or MS |
|---|---|---|---|---|---|
| 435 | HO-CH2CH2CH2-(2-methoxyphenyl) | 3-(2-chloro-5-pyridinylmethyl)-2-iminothiazolidine | 3,4 | 2.03(2H, m), 2.72(2H, t), 3.15 (2H, t), 3.56(2H, t), 3.81 (3H, s), 4.20(2H, t), 4.82 (2H, s), 6.84(1H, d), 6.87 (1H, dt), 7.14(1H, dd), 7.18 (1H, dd), 7.32(1H, dd), 7.66 (1H, dd), 8.31(1H, d) | m/z = 420 (M+H) |

[Formulation Examples]

Formulation Example 1 [Pellets]

| | |
|---|---|
| Compound 6 | 5% by weight |
| Bentonite | 40% by weight |
| Talc | 10% by weight |
| Clay | 43% by weight |
| Calcium lignin sulfonate | 2% by weight |

The above described ingredients were ground and mixed with each other uniformly. Then, water was added thereto, and the mixture was kneaded thoroughly, followed by pelletization and drying. Thus, pellets were obtained.

Formulation Example 2 [Wettable Powder]

| | |
|---|---|
| Compound 6 | 30% by weight |
| Clay | 50% by weight |
| White carbon | 2% by weight |
| Diatomite | 13% by weight |
| Calcium lignin sulfonate | 4% by weight |
| Sodium lauryl sulfate | 1% by weight |

The above described ingredients were mixed with each other uniformly and ground. Thus, a wettable powder

was obtained.

Formulation Example 3 [Water Dispersible Granules]

| | |
|---|---|
| Compound 6 | 30% by weight |
| Clay | 60% by weight |
| Dextrin | 5% by weight |
| Alkylmaleic acid copolymer | 4% by weight |
| Sodium lauryl sulfate | 1% by weight |

The above described ingredients were ground and mixed with each other uniformly. Then, water was added thereto, and the mixture was kneaded thoroughly, followed by granulation and drying. Thus, water dispersible granules were obtained.

Formulation Example 4 [Emulsion]

| | |
|---|---|
| Compound 51 | 15% by weight |
| N,N-dimethylformamide | 20% by weight |
| Solvesso 150 | 55% by weight |

(ExxonMobil Yugen Kaisha)

Polyoxyethylene alkyl aryl ether10% by weight

The above described ingredients were mixed with each other uniformly, and dissolved in each other. Thus, an emulsion was obtained.

Formulation Example 5 [Powder]

| | |
|---|---|
| Compound 51 | 2% by weight |
| Clay | 60% by weight |
| Talc | 37% by weight |
| Calcium stearate | 1% by weight |

The above described ingredients were mixed with each other uniformly. Thus, a powder was obtained.

Formulation Example 6 [Liquefied Drops]

| | |
|---|---|
| Compound 6 | 24% by weight |
| Ethanol | 76% by weight |

The above described ingredients were mixed with each other uniformly. Thus, liquefied drops were obtained.

Formulation Example 7 [Liquefied Drops]

| | |
|---|---|
| Compound 6 | 48% by weight |
| Ethanol | 52% by weight |

The above described ingredients were mixed with each other uniformly. Thus, liquefied drops were obtained.

[Test Examples]

Test Example 1 (Test on Control of *Haemaphysalis longicornis*)

Into a 4-mL glass vial, 30 μl of an acetone solution containing a compound at 200 ppm, 10 ppm, or 1.7 ppm was introduced. This vial was placed on a shaker, and a dry film of the compound was formed on the inner wall of the vial. The vial was dried for 24 hours or more. Ten larvae of *Haemaphysalis longicornis* were introduced in the vial, and then the vial was capped. The vial was allowed to stand still in a thermostatic chamber under conditions of 25 °C, a humidity of 85%, and total darkness. One day after the introduction, the larvae were observed for mortality, and the mortality ratio was calculated in accordance with the following formula. The test was duplicated.

$$\text{Mortality ratio (\%)} = [\text{Number of dead larvae}/(\text{Number of survived larvae} + \text{Number of dead larvae})] \times 100$$

As a result, each of the following compounds exhibited a mortality ratio on the dry film formed from the 200-ppm drug solution of 60% or higher.
2, 4, 5, 6, 7, 8, 9, 10, 11, 12, 13, 14, 15, 16, 18, 21, 28, 29, 42, 43, 44, 45, 46, 47, 48, 51, 52, 53, 57, 58, 59, 61, 62, 64, 67, 70, 72, 73, 75, 76, 77, 78, 81, 83, 88, 89, 90, 93, 100, 104, 106, 107, 113, 114, 115, 117, 119, 120, 121, 122, 123, 124, 125, 126, 127, 128, 129, 131, 132, 133, 134, 135, 139, 140, 141, 142, 143, 144, 145, 147, 150, 151, 153, 154, 155, 156, 163, 167, 171, 173, 175, 176, 177, 179, 180, 182, 184, 185, 189, 190, 197, 198, 199, 200, 201, 202, 203, 204, 205, 206, 207, 208, 209, 210, 211, 213, 214, 215, 216, 217, 218, 219, 220, 221, 222, 226, 227, 228, 229, 230, 231, 232, 233, 234, 235, 239, 240, 241, 242, 243, 244, 247, 248, 249, 251, 252, 254, 255, 256, 258, 259, 260, 263, 264, 265, 267, 268, 270, 271, 273, 274, 277, 278, 279, 283, 284, 287, 289, 290, 291, 292, 299, 300, 301, 302, 303, 304, 305, 306, 307, 308, 309, 310, 311, 312, 313, 314, 315, 316, 317, 318, 319, 320, 321, 322, 326, 327, 328, 329, 330, 332, 333, 334, 335, 336, 337, 338, 339, 340, 341, 342, 343, 344, 345, 346, 347, 348, 349, 350, 353, 354, 357, 358, 359, 360, 361, 362, 366, 367, 372, 373, 374, 427, 428, 429, 430, 431

Each of the following compounds exhibited a mortality ratio on the dry film formed from the 10-ppm drug solution of 60% higher.
2, 5, 6, 7, 8, 9, 10, 12, 18, 42, 43, 44, 45, 47, 51, 53, 57, 58, 59, 61, 70, 72, 75, 76, 81, 83, 88, 106, 107, 1 13, 114, 115, 122, 123, 124, 125, 126, 128, 129, 132, 1 34, 135, 139, 141, 142, 144, 145, 149, 155, 167, 171, 1 73, 184, 185, 189, 190, 197, 198, 199, 201, 203, 210, 2 11, 213, 214, 215, 216, 219, 220, 226, 227, 228, 229, 2 30, 233, 240, 241, 244, 246, 248, 249, 251, 254, 255, 2 56, 258, 259, 260, 267, 268, 283, 284, 292, 299, 300, 3 01, 302, 303, 304, 305, 306, 307, 308, 309, 310 311, 31 2, 313, 314, 315, 316, 318, 321, 322, 326, 328, 331, 33 3, 334, 335, 336, 337, 338, 339, 340, 341, 342, 343, 34 4, 345, 346, 348, 349, 350, 354, 355, 356, 357, 358, 35 9, 361, 362, 366, 367, 373, 374, 427, 429, 430, 431

Each of the following compounds exhibited a mortality ratio on the dry film formed from the 1.7-ppm drug solution of 60% or higher.
6, 42, 43, 44, 45, 57, 61, 72, 76, 81, 83, 106, 107, 113, 114, 115, 122, 123, 124, 125, 128, 132, 134, 135, 139, 141, 144, 145, 155, 167, 171, 198, 211, 213, 216, 226, 227, 228, 230, 233, 240, 241, 244, 251, 255, 267, 268, 283, 292, 299, 302, 303, 308, 309, 313, 326, 328, 329, 336, 337, 338, 341, 343, 345, 349, 351, 352, 354, 355, 358, 359, 366, 374, 430, 431

Test Example 2 (Test on Control of *Haemaphysalis longicornis* on Body Surface of Mouse)

Hair on the back of a mouse (ICR, male, 5-weeks old) was shaved in a region having a diameter of approximately 2 cm, and a 15-mL polystyrene conical tube cut to have a height of approximately 1.5 cm was bonded to the region with an instant adhesive.

Then, 20 μl of a 1000-fold diluted liquid of a pest control agent prepared in the same manner as in Preparation Example 7 was added dropwise onto the body surface of the mouse within the bonded tube. After sufficient drying, 10 or more larvae of *Haemaphysalis longicornis* were introduced, and the tube was capped. Three days after the introduction,

the larvae of *Haemaphysalis longicornis* were observed for mortality, and the mortality rate was calculated in accordance with the following formula.

$$\text{Mortality ratio (\%)} = [\text{Number of dead larvae}/(\text{Number of survived larvae} + \text{Number of dead larvae})] \times 100$$

As a result, each of the following compounds exhibited a mortality ratio of 60% or higher.
81, 173, 283

Test Example 3 (Test on Control of *Tyrophagus putrescentiae*)

Into a 4-mL glass vial, 30 μl of an acetone solution containing a compound at 50 ppm was introduced. This vial was placed on a shaker, and a dry film of the compound was formed on the inner wall of the vial. After the vial was dried for 24 hours or more, five adults of *Tyrophagus putrescentiae* were introduced into the vial, and the vial was capped with absorbent cotton. The vial was allowed to stand in a thermostatic chamber under conditions of 25 °C, a humidity of 85%, a 16-hour light period, and an 8-hour dark period. One day after the introduction, observation was made for mortality, and the mortality ratio was calculated in accordance with the following formula. The test was duplicated.

$$\text{Mortality ratio (\%)} = [\text{Number of dead mites}/(\text{Number of survived mites} + \text{Number of dead mites})] \times 100$$

As a result, each of the following compounds exhibited a mortality ratio on the dry film formed from the 50 ppm drug solution of 90% or higher.
2, 42

Test Example 4 (Effects on Microfilariae of *Dirofilaria immitis;* in vitro test)

Activities of compounds were evaluated on the basis of change in motility of microfilariae of *D. immitis.* Each compound of the present invention was dissolved in an RPMI 1640 liquid culture medium at a concentration of 12.5 ppm, 6.25 ppm, and 3.13 ppm. Then, approximately 20 microfilariae of *D. immitis* were introduced per culture liquid, and cultured at 37°C. Forty eight hours after the start of the culturing, the microfilariae of *D. immitis* were observed for motility. The compound was determined to be effective, when some influences were observed in 90% or more of the microfilariae, or when 1/3 or more of the microfilariae were dead.
As a result, the following compounds were effective at 12.5 ppm.
6, 28, 36, 48, 49, 52, 53, 57, 73, 75, 83, 111, 120, 154, 177, 196, 197, 198, 199, 201, 202, 203, 205, 226, 253, 254, 261, 262, 263, 267, 271, 299, 308, 309
The following compounds were effective at 6.25 ppm.
28, 48, 52, 53, 73, 75, 83, 84, 111, 120, 196, 198, 199, 202, 203, 205, 221, 226, 253, 261, 262, 263, 267, 271, 299, 308, 309, 357, 372
The following compounds were effective at 3.13 ppm. 52, 53, 73, 75, 83, 84, 111, 203, 226, 261, 262, 263, 267, 271, 299, 308, 309, 372

Test Example 5 Effects on Adults of *Ascaridia galli* (in vitro test)

Activities of compounds were evaluated on the basis of change in motility of adults of *A. galli.* Each compound of the present invention was dissolved in dimethyl sulfoxide, and then added to a culture liquid of *A. galli* dissolved in Ringer' s solution, at a concentration of the compound of 50 ppm. Six adults of *A. galli* were introduced per culture liquid, and cultured at 41°C. Twenty four hours after the start of the culturing, the activity of the compound was evaluated by observing the motility of the adults of *A. galli.*
A compound was evaluated to be effective when 4 or more of the adults of *A. galli* stopped their active movement in the observation of the motility of adults of *A. galli* conducted 24 hours after the start of the culturing. As a result, the following compound was effective.
142

Test Example 6 (Test on Control of *Musca domestica*)

A drug solution was prepared by dissolving a compound in a 5% sucrose solution at 7.5 ppm. Five female adults of *Musca domestica* 24 hours after eclosion and absorbent cotton impregnated with 4 mL of the 5% sucrose solution containing the compound were placed in a 200-mL plastic cup in which a filter paper was laid. Then, the plastic cup was allowed to stand still under conditions of 25°C, a 16-hour light period, and an 8-hour dark period. One day after the placement, observation was made for mortality, and the mortality ratio was calculated in accordance with the following formula. The test was duplicated.

Mortality ratio (%) = [Number of dead insects/(Number of survived insects + Number of dead insects)] × 100

As a result, the following compound exhibited a mortality ratio of 90% or higher.
42

Test Example 7 (Test on Control of *Musca domestica*)

One microliter of an acetone solution of a compound was locally administered to the back surfaces of thoraxes of female adults of *Musca domestica* within 24 hours after eclosion. The female adults of *Musca domestica,* and absorbent cotton impregnated with 4 mL of a 5% sucrose solution were placed in a 200-mL plastic cup in which a filter paper was laid. Then, the plastic cup was allowed to stand still under conditions of 25°C, a 16-hour light period, and an 8-hour dark period. One day after the placement, observation was made for mortality, and the mortality ratio was calculated in accordance with the following formula. The test was duplicated.

Mortality ratio (%) = [Number of dead insects/(Number of survived insects + Number of dead insects)] × 100

As a result, the following compound exhibited a mortality ratio of 70% or higher, when administered at 0.1 μg/insect.
240

Test Example 8 (Test on Control of *Culex pipiens pallens*)

One milliliter of a 0.1% acetone solution containing a compound was prepared. To this solution, a small amount of a mixture of a dry yeast and a powdered solid feed for mice was added as a feed. Into this solution, 10 hatched larvae of *Culex pipiens pallens* were introduced. The solution was allowed to stand still under conditions of 25°C, a 16-hour light period, and an 8-hour dark period. One day after the placement, observation was made for mortality, and the mortality ratio was calculated in accordance with the following formula. The test was duplicated.

Mortality ratio (%) = [Number of dead larvae/(Number of survived larvae + Number of dead larvae)] × 100

As a result, the following compound exhibited a mortality ratio of 60% or higher, when used at 1 ppm. 240

In Test Examples 1 to 8, the compounds of the present invention demonstrated excellent controlling effects against animal-parasitic pests and public health pests. The controlling effects were superior to those reported so far against pests in the agricultural field.

[Industrial Applicability]

As described above, the present invention is capable of making a great contribution especially in the fields of controlling animal-parasitic pests and public health pests.

## Claims

1. A pest control agent comprising:

one or more imino derivatives represented by the following chemical formula (I):

[Chem. 1]

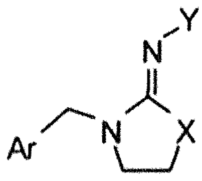

Formula (I)

(where Ar represents a heterocyclic group which may have a substituent on a ring thereof, X represents a sulfur atom, $CH_2$, or NR, Y represents $COR_1$, $CONR_3R_4$, $CONHCOR_5$, or $CO_2R_9$, and R represents a hydrogen atom or an alkyl group,
when Y is $COR_1$, $R_1$ represents a hydrogen atom, a C1-C18 alkyl group, a C1-C18 halogenated alkyl group, a C2-C18 alkenyl group, a C2-C18 halogenated alkenyl group, a C2-C18 alkynyl group, a C2-C18 halogenated alkynyl group, a substituted or unsubstituted C6-C10 aryl group, a substituted or unsubstituted (C6-C10)aryl(C1-C8)alkyl group, a substituted or unsubstituted (C6-C10)aryl(C2-C8)alkenyl group, a substituted or unsubstituted (C6-C10) aryl(C2-C8)alkynyl group, a substituted or unsubstituted (C1-C4)alkoxy(C1-C5)alkyl group, a substituted or unsubstituted (C1-C4)alkoxy(C2-C5)alkenyl group, a substituted or unsubstituted (C1-C4)alkoxy(C2-C5)alkynyl group, a C1-C3 alkoxycarbonyl group, a (C1-C3)alkylsulfonyl(C1-C3)alkyl group, a (C1-C4)alkylthio(C1-C5)alkyl group, a C3-C12 substituted or unsubstituted cycloalkyl group, a 3-membered to 7-membered substituted or unsubstituted heterocycloalkyl group, a 3-membered to 7-membered substituted or unsubstituted heterocycloalkyl(Cl-C8)alkyl group, a 3-membered to 7-membered substituted or unsubstituted heterocycloalkyl(C2-C8)alkenyl group, a 3-membered to 7-membered substituted or unsubstituted heterocycloalkyl(C2-C8)alkynyl group, a cyano(C1-C3)alkyl group, a substituted or unsubstituted phenoxy(C1-C8)alkylgroup, a substituted or unsubstituted phenoxy(C2-C8) alkenyl group, a substituted or unsubstituted phenoxy(C2-C8)alkynyl group, a substituted or unsubstituted heterocyclyl(C1-C8)alkyl group, a substituted or unsubstituted heterocyclyl(C2-C8)alkenyl group, a substituted or unsubstituted heterocyclyl(C2-C8)alkynyl group, a substituted or unsubstituted heterocyclyloxy(C1-C8)alkyl group, a substituted or unsubstituted heterocyclyloxy(C2-C8)alkenyl group, a substituted or unsubstituted heterocyclyloxy(C2-C8)alkynyl group, a furanyl group, a morpholino group, a norbornenyl group, an adamantyl group, an isothiocyanatomethyl group, a rhodanine group, a substituted or unsubstituted heterocyclic or aromatic ring, a (C1-C5) alkylcarbonylamino(C1-C3)alkyl group, a (C1-C5) alkyloxycarbonylaminooxymethyl group, a (C1-C5)alkyloxycarbonylaminomethyl group, or a (C1-C5)alkylcarbonyloxymethyl group,
when Y is $CONR_3R_4$, $R_3$ and $R_4$ each represent a hydrogen atom, a C1-C5 alkyl group, a C1-C5 halogenated alkyl group, a C2-C5 alkenyl group, a C2-C5 halogenated alkenyl group, a C2-C5 alkynyl group, a C2-C5 halogenated alkynyl group, a C1-C3 alkoxy group, an alkenyloxy group, a substituted or unsubstituted C6-C10 aryl group, a substituted or unsubstituted (C6-C10)aryl(C1-C8)alkyl group, a substituted or unsubstituted (C6-C10)aryl(C2-C8) alkenyl group, a substituted or unsubstituted (C6-C10)aryl(C2-C8)alkynyl group, a (C1-C4)alkoxy(C1-C5)alkyl group, a (C1-C4)alkoxy(C2-C5)alkenyl group, a (C1-C4)alkoxy(C2-C5)alkynyl group, a C1-C3 alkoxycarbonylmethyl group, a (C1-C4)alkylthio(C1-C5)alkyl group, a C3-C12 substituted or unsubstituted cycloalkyl group, a 3-membered to 7-membered substituted or unsubstituted heterocycloalkyl group, a substituted or unsubstituted pyridylmethyl group, a substituted or unsubstituted benzenesulfonyl group, a (C1-C5)alkylamino group, a substituted or unsubstituted phenylamino group, a (C1-C5)alkylcarbonylamino group, or a substituted or unsubstituted benzoylamino group, and $NR_3R_4$ may form a ring,
when Y represents $CONHCOR_5$, $R_5$ represents a hydrogen atom, a C1-C5 alkyl group, a C1-C5 halogenated alkyl group, a C2-C5 alkenyl group, a C2-C5 halogenated alkenyl group, a substituted or unsubstituted C6-C10 aryl group,

or a substituted or unsubstituted (C6-C10)aryl(C1-C5)alkyl group,
when Y represents $CO_2R_9$, $R_9$ represents a hydrogen atom, a C1-C18 alkyl group, a C1-C18 halogenated alkyl group, a C2-C18 alkenyl group, a C2-C18 halogenated alkenyl group, a C2-C18 alkynyl group, a C2-C18 halogenated alkynyl group, a substituted or unsubstituted C6-C10 aryl group, a substituted or unsubstituted (C6-C10)aryl(C1-C8)alkyl group, a substituted or unsubstituted (C6-C10)aryl(C2-C8)alkenyl group, a substituted or unsubstituted (C6-C10)aryl(C2-C8)alkynyl group, a substituted or unsubstituted (C1-C4)alkoxy(C1-C5)alkyl group, a substituted or unsubstituted (C1-C4)alkoxy(C2-C5)alkenyl group, a substituted or unsubstituted (C1-C4)alkoxy(C2-C5)alkynyl group, a (C1-C4)alkylthio(C1-C5)alkyl group, a tri(C1-C3 alkyl)silyl(C1-C3)alkyl group, a C3-C12 substituted or unsubstituted cycloalkyl group, a 3-membered to 7-membered substituted or unsubstituted heterocycloalkyl group, a 3-membered to 7-membered substituted or unsubstituted heterocycloalkyl(C1-C8)alkyl group, a 3-membered to 7-membered substituted or unsubstituted heterocycloalkyl(C2-C8)alkenyl group, a 3-membered to 7-membered substituted or unsubstituted heterocycloalkyl(C2-C8)alkynyl group, an a (C1-C3)alkylsulfonyl(C1-C3)alkyl group, a substituted or unsubstituted phenoxy(C1-C8)alkyl group, a substituted or unsubstituted phenoxy(C2-C8)alkenyl group, a substituted or unsubstituted phenoxy(C2-C8)alkynyl group, a substituted or unsubstituted heterocyclyl(C1-C8)alkyl group, a substituted or unsubstituted heterocyclyl(C2-C8)alkenyl group, a substituted or unsubstituted heterocyclyl(C2-C8)alkynyl group, a substituted or unsubstituted heterocyclyloxy(C1-C8)alkyl group, a substituted or unsubstituted heterocyclyloxy(C2-C8)alkenyl group, a substituted or unsubstituted heterocyclyloxy(C2-C8)alkynyl group, a succinimide group, or a 18-crown-6-methyl group, and
carbon chains thereof may be substituted with halogens).

**2.** A pest control agent according to claim 1, wherein
Ar in the chemical formula (I) is represented by the following chemical formula (II) or (III):

[Chem. 2]

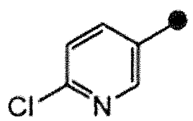

Formula (II)

,

or

[Chem. 3]

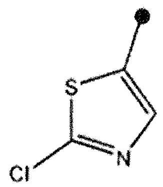

Formula (III)

**3.** The pest control agent according to claim 1 or 2, wherein
,in the chemical formula (I), X represents a sulfur atom, and Y represents $COR_1$ or $CO_2R_9$,
when Y is $COR_1$, $R_1$ represents a C1-C4 halogenated alkyl group, a C2-C6 alkenyl group, a C3-C6 alkynyl group, a substituted or unsubstituted phenyl(C1-C4)alkyl group, a substituted or unsubstituted phenyl(C2-C3)alkenyl group, a substituted or unsubstituted (C1-C4)alkoxy(C1-C3)alkyl group, a 3-membered to 7-membered substituted or unsubstituted heterocycloalkyl(C1-C3)alkyl group, a 3-membered to 7-membered substituted or unsubstituted heterocycloalkyl(C1-C3)alkenyl group, a substituted or unsubstituted heterocyclyl(C1-C3)alkyl group, or a substituted or unsubstituted heterocyclyl(C2-C3)alkenyl group, and
when Y is $CO_2R_9$, $R_9$ represents a C1-C6 halogenated alkyl group, a C3-C6 alkenyl group, a C3-C6 alkynyl group, a substituted or unsubstituted phenyl(C1-C4)alkyl group or a substituted or unsubstituted phenyl(C2-C4)alkenyl group, a methoxy(C3-C4)alkyl group; a 3-membered to 7-membered substituted or unsubstituted heterocycloalkyl (C1-C3)alkyl group, or a substituted or unsubstituted heterocyclyl(C2-C3)alkyl group.

**4.** The pest control agent according to claim 1 or 2, wherein ,in the chemical formula (I), X represents a sulfur atom, Y represents $COR_1$ or $CO_2R_9$, when Y is $COR_1$, $R_1$ represents a C1-C3 halogenated alkyl group, a C4-C6 alkenyl group, a C4-C6 alkynyl group, a substituted phenyl(C2-C4)alkyl group, a substituted or unsubstituted phenyl(C2-C3)alkenyl group, a substituted or unsubstituted (C1-C4)alkoxymethyl group, a 3-membered to 7-membered substituted or unsubstituted heterocycloalkyl(C2-C3)alkyl group, a 3-membered to 7-membered substituted or unsubstituted heterocycloalkyl(C2-C3) alkenyl group, a substituted or unsubstituted heterocyclyl(C2-C3)alkyl group, or a substituted or unsubstituted heterocyclyl(C2-C3)alkenyl group, and when Y is $CO_2R_9$, $R_9$ represents a C3-C4 halogenated alkyl group, a C5-C6 alkenyl group, a C3-C4 alkynyl group, a substituted or unsubstituted phenyl(C2-C4)alkyl group, a substituted or unsubstituted phenyl(C2-C4)alkenyl group, a methoxy(C3-C4)alkyl group, a 3-membered to 7-membered substituted or unsubstituted heterocycloalkyl(C2-C3) alkyl group, or a substituted or unsubstituted heterocyclyl(C2-C3)alkyl group.

**5.** The pest control agent according to claim 1, wherein the imino derivative represented by the chemical formula (I) is any one of the compounds shown in the following Table 1.

[Table 1]

| Compound No. | Y | X | Ar |
|---|---|---|---|
| 6 | CO-phenyl | S | ( I I ) |
| 42 | COOMe | S | ( I I ) |
| 43 | COOC3H7-n | S | ( I I ) |
| 44 | COOC3H7-i | S | ( I I ) |
| 45 | COOCH2CH2Cl | S | ( I I ) |
| 57 | COPh | S | ( I I I ) |
| 61 | CHO | S | ( I I I ) |
| 72 | COMe | S | ( I I ) |
| 76 | COCHF2 | S | ( I I ) |
| 81 | COCH2CH2OMe | S | ( I I ) |
| 83 | COCH=CH2 | S | ( I I ) |
| 06 | CONHOMe | S | ( I I ) |
| 107 | CONHOEt | S | ( I I ) |
| 113 | COOEt | S | ( I I ) |
| 114 | COOCH2CH2F | S | ( I I ) |
| 115 | COOCH2CHF2 | S | ( I I ) |
| 122 | COOCH2CH2CH2F | S | ( I I ) |
| 123 | COOCH(CH2F)2 | S | ( I I ) |
| 124 | COOCH(Me)CF3 | S | ( I I ) |
| 125 | COOCH(CF3)2 | S | ( I I ) |
| 128 | COO-n-butyl | S | ( I I ) |
| 132 | COOCH2CH2CH2CH2F | S | ( I I ) |
| 134 | COOCH2CF2CF2CF3 | S | ( I I ) |
| 135 | COOCH2CH=CMe2 | S | ( I I ) |
| 139 | COO-CH2-cyclopropyl | S | ( I I ) |
| 140 | COOCH2-2-oxiranyl | S | ( I I ) |

(continued)

| Compound No. | Y | X | Ar |
|---|---|---|---|
| 141 | COO-cyclobutyl | S | ( I I ) |
| 44 | COOCH2-3-tetrahydrofuranyl | S | ( I I ) |
| 145 | COOCH2-(2,2-dimethyl-1,3-dioxolan-4-yl) | S | ( I I ) |
| 155 | COCH2-3-thienyl | S | S |
| 167 | COO-iso-propyl | S | ( I I ) |
| 171 | COOCH2C≡CH | S | ( I I ) |
| 198 | CO-4-t-butylphenyl | S | ( I I I ) |
| 211 | COO-n-pentyl | S | ( I I I ) |
| 213 | CO-2,2-difluorocyclopropyl | S | ( I I ) |
| 216 | COCH2CH2CF3 | S | ( I I ) |
| 226 | COO-n-hexyl | S | ( I I ) |
| 227 | COOCH2t-Bu | S | ( I I ) |
| 228 | COO-CH2-Crownether(18-C-6) | S | ( I I ) |
| 230 | COCH2OCOMe | S | ( I I ) |
| 233 | COCH2ONHCOOEt | S | ( I I ) |
| 240 | COCF3 | S | ( I I ) |
| 241 | COCF3 | S | ( I I I ) |
| 244 | COO-3-methoxyphenyl | S | ( I I ) |
| 251 | COOCH2CH2CH2NO2 | S | ( I I ) |
| 255 | COOCH2CH2morpholinyl | S | ( I I ) |
| 267 | CO-3-methylphenyl | S | ( I I ) |
| 268 | CO-2-fluorophenyl | S | ( I I ) |
| 292 | CO-2-fluorophenyl | S | ( I I I ) |
| 299 | CO-phenyl | S | 4,5-dichloro-3-pyridyl |
| 302 | CO-phenyl | S | 4-bromo-3-pyridyl |
| 303 | COOCH2CH2C≡CH | S | ( I I ) |
| 308 | COCH2CH2C≡CH | S | ( I I ) |
| 309 | COCH2CH2CH2C≡CH | S | ( I I ) |
| 313 | COCH2CH2CH2CH2C≡CH | S | ( I I I ) |
| 326 | COCH2-(2-tetrahydrofuranyl) | S | ( I I ) |
| 328 | COCH2CH2-(2-thienyl) | S | ( I I ) |
| 329 | COOCH2CH2-(2-tetrahydrofuranyl) | S | ( I I ) |
| 336 | COCH2CH2-(2-tetrahydrofuranyl) | S | ( I I ) |
| 337 | COOCH2CH2CH2-(2-furanyl) | S | ( I I ) |
| 338 | COOCH2CH2-(3-tetrahydrofuranyl) | S | ( I I ) |
| 341 | CO-CH2CH2-(3-furanyl) | S | ( I I ) |
| 343 | COCH2CH2-(4-methoxyphenyl) | S | ( I I ) |

(continued)

| | | | |
|---|---|---|---|
| 155 | COCH2-3-thienyl | S | S |
| 345 | COCH2CH2-(3,5-dimethoxyphenyl) | S | ( I I ) |
| 349 | COOCH2CH2CH2-(3-furanyl) | S | ( I I ) |
| 351 | COO-CH2CH2CH2-(3-tetrahydrofuranyl) | S | ( I I ) |
| 352 | COOCH2CH2-(2-thienyl) | S | ( I I ) |
| 354 | COOCH2CH2-(3-thienyl) | S | ( I I ) |
| 355 | COCH2CH2-(benzo[d][1,3]dioxol-5-yl) | S | ( I I ) |
| 358 | COCH2CH2-(3-methoxyphenyl) | S | ( I I ) |
| 359 | COOCH2CH2-(2-furanyl) | S | ( I I ) |
| 366 | COOCH2-(2-thienyl) | S | ( I I ) |
| 374 | COCH2OCH2C≡CH | S | ( I I ) |
| 430 | COCH2OCH2-(2-furanyl) | S | ( I I ) |
| 431 | COCH2OCH2-(3-furanyl) | S | ( I I ) |

**6.** A compound represented by the following chemical formula (Ia):

[ Chem. 4]

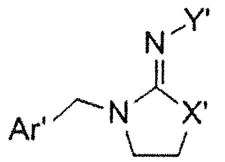

Formula (Ia)

(where Ar' represents a heterocyclic group which may have a substituent on a ring thereof, X' represents a sulfur atom, and Y' represents $COR_1$' or $CO_2R_9$',
when Y is $COR_1$', $R_1$' represents a substituted phenyl(C2-C4)alkyl group, a 3-membered to 7-membered substituted or unsubstituted heterocycloalkyl(C2-C3)alkyl group, a substituted or unsubstituted heterocyclyl(C2-C3)alkyl group, a substituted or unsubstituted (C1-C4)alkoxymethyl group, a 3-membered to 7-membered heterocycloalkyl group or a substituted or unsubstituted heterocycle and
when Y' is $CO_2R_9$', $R_9$' represents a substituted or unsubstituted phenyl(C2-C4)alkyl group, a 3-membered to 7-membered substituted or unsubstituted heterocycloalkyl(C2-C3)alkyl group, or a substituted or unsubstituted heterocyclyl(C2-C3)alkyl group).

**7.** The compound according to claim 6, wherein
Ar' in the chemical formula (Ia) is represented by the following chemical formula (II) or (III):

[Chem. 5]

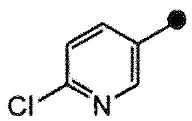

Formula (II)

,

or

[Chem. 6]

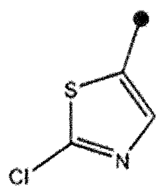

Formula (III)

**8.** The pest control agent according to any one of claims 1 to 5, wherein the pest is an animal-parasitic pest.

**9.** The pest control agent according to any one of claims 1 to 5, wherein the pest is an animal-parasitic tick or mite.

**10.** A method for controlling a pest, comprising:

treating the pest with an effective amount of the pest control agent according to any one of claims 1 to 5 and 9.

## INTERNATIONAL SEARCH REPORT

International application No.
PCT/JP2011/065285

A. CLASSIFICATION OF SUBJECT MATTER
*A01N43/78*(2006.01)i, *A01N47/18*(2006.01)i, *A01N47/36*(2006.01)i, *A01P5/00*(2006.01)i, *A01P7/02*(2006.01)i, *A01P7/04*(2006.01)i, *C07D277/20*(2006.01)i, *C07D277/32*(2006.01)i, *C07D401/04*(2006.01)i, *C07D401/14*(2006.01)i,
According to International Patent Classification (IPC) or to both national classification and IPC

B. FIELDS SEARCHED
Minimum documentation searched (classification system followed by classification symbols)
A01N43/78, A01N47/18, A01N47/36, A01P5/00, A01P7/02, A01P7/04, C07D277/20, C07D277/32, C07D401/04, C07D401/14, C07D417/06, C07D417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2010/001922 A1 (Kureha Corp.),<br>07 January 2010 (07.01.2010),<br>claims 1, 7; paragraph [0072]; examples<br>& AU 2009267308 A & CA 2729191 A | 1-10<br>8,9 |
| X<br>Y<br>A | Ikuya OHNO et al., Journal of Agricultural and Food Chemistry, 2010.04.06, 58, p.4999-5003 | 1-5,10<br>8,9<br>6,7 |
| X<br>Y<br>A | Eiki WATANABE et al., Journal of Agricultural and Food Chemistry, 2004, 52, p.2756-2762 | 1,2,10<br>8,9<br>3-7 |
| X<br>Y<br>A | Ikuya OHNO et al., Chemical Research in Toxicology, 2009, 22, p.476-482 | 1,2,10<br>8,9<br>3-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
14 July, 2011 (14.07.11)

Date of mailing of the international search report
26 July, 2011 (26.07.11)

Name and mailing address of the ISA/
Japanese Patent Office

Authorized officer

Facsimile No.

Telephone No.

Form PCT/ISA/210 (second sheet) (July 2009)

**INTERNATIONAL SEARCH REPORT**

International application No.
PCT/JP2011/065285

C (Continuation). DOCUMENTS CONSIDERED TO BE RELEVANT

| Category* | Citation of document, with indication, where appropriate, of the relevant passages | Relevant to claim No. |
|---|---|---|
| P,X<br>A | Ikuya OHNO et al., Bioorganic & Medical Chemistry Letters, 2010.07.16, 20, p.5933-5935 | 1,2,8-10<br>3-7 |

Form PCT/ISA/210 (continuation of second sheet) (July 2009)

**INTERNATIONAL SEARCH REPORT**

International application No.
PCT/JP2011/065285

Continuation of A. CLASSIFICATION OF SUBJECT MATTER
(International Patent Classification (IPC))

*C07D417/06*(2006.01)i, *C07D417/14*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP SHO63150275 B
- JP 2007506674 A
- WO 2010001922 A


### Non-patent literature cited in the description


- *Chemical Research in Toxicology,* 2009, vol. 22 (3), 476-482
- *Journal of Medicinal Chemistry,* 1999, vol. 42 (12), 2227